# EUROPÄISCHE PATENTANMELDUNG

(11) **EP 4 008 176 A1**
(43) Veröffentlichungstag der Anmeldung: **08.06.2022**
(21) Anmeldenummer: 21205168.4
(22) Anmeldetag: 21.12.2016
(51) Int. Cl.: A01H 5/10, C12N 15/82, C12Q 1/68

(54) **RESTORER-PFLANZE**

(30) Priorität: 21.12.2015 DE 102015016445
(62) Teilanmeldung aus: 16828943.7
(71) Anmelder: KWS SAAT SE & Co. KGaA, 37574 Einbeck (DE)
(72) Erfinder: Wilde, Peer, 29230 Hermannsburg (DE); Korzun, Viktor, 37574 Einbeck (DE); Menzel, Jutta, 29303 Bergen (DE); Zhou, Ruonan, 06466 Stadt Seeland (DE); Stein, Nils, 06484 Quedlinburg (DE); Hackauf, Bernd, 18190 Sanitz (DE)

(57) **Zusammenfassung**

Bereitgestellt werden Hybridgetreide, die durch Restauration der Pollenfertilität für die Pampa cytoplasmatische männliche Sterilität (P CMS) erhalten werden, und sich durch einen verminderten Linkage Drag auszeichnen. Es werden Pflanzen zur Verfügung gestellt, insbesondere Roggen, die als männlicher Pollenelter in der Lage sind, die Pollenfertilität für die P CMS zu restaurieren. Des Weiteren werden das Nukleinsäuremolekül, das die notwendigen Informationen zur Restauration der P CMS trägt, DNA und Vektoren, die ein solches Nukleinsäuremolekül enthalten, entsprechende Wirtszellen sowie ein durch das Nukleinsäuremolekül kodierbares Protein und dagegen gerichtete Antikörper beschrieben. Ferner werden Verfahren zur Herstellung von entsprechenden Hybridpflanzen und transgenen Pflanzen bereitgestellt.

SEQUENCE LISTING

<110>  KWS SAAT SE & Co. KGaA

<120>  Restorer-Pflanze

<130>  KWS0224PCT/EP/D1

<150>  DE 10 2015 016 445.7
<151>  2015-12-21

<160>  33

<170>  PatentIn version 3.5

<210>  1
<211>  1146
<212>  DNA
<213>  Secale cereale

<400>  1
atgctcctcc tcctccggca gcgcgtcctc tccgctgcgc catctccttc cacctcccca      60

ctcctctctc tccaccgcct cctctgcgcc gccgcgcccg tcaaccctag cttcgccgtc     120

gacgactacc tcgtcggcac ctgcggcctc agccgtgccc aggcactcaa ggcatccgcc     180

aagctgtccc acctcaagtc ccccgccaac cccgacgccg tcctcgcctt cctcgccgga     240

ctcggcctct ccggcgccga tgtggcggcc gtcgtcgcca aggatcccaa gttcctctgc     300

gccggcgtgg agacaaccct ggcccccgtc gtcgctgggc tcaccggcct gggtctgtca     360

aatgctgaga ctgcgcgcct cgtctcgctt gcccccgaca aattccgcca gagatccatc     420

gtctccaagc tagactacta cctgccgctc ttcggctcca tcgacaactt ggtccggtcg     480

ctcaaacacg gcgccggcat cctcggctcc gacctcgaga gggtggtcaa gcccaatgtt     540

agtctcctag cagagtgcgg gctaggtgct tgtgatattg ccaagctgtt cgtccaaata     600

ccgaggatgc tgtgtgctaa accagagcgt gtcctggaga tggttgcgtg tgccgaaagt     660

ataggtgtgc cccgtggctc tggaatgttc aggcacgcgc tgcacgctgt ctcatacttc     720

agcgacgaca agctcaccgc taaagtggac tacttgaaga agacatttag gtggtcggat     780

gccgaggttg ccattgctgt gtccaagggt ccatttctgc tgaggaggtc aaaggatatt     840

ctgaagcaca gctccgagtt ccttatcact gaggtagggt tgcagccggc ctacattgct     900

catcggccgg ctatgctcac ttacagcctg gagggccggc tcaggccccg ctactatgtt     960

gtgagatttc tcaaggaaaa tggattgcta gagcacgggc ggagctacta tacaacactg    1020

attagtactg agaaggtttt catggaaaag ttcatacgcc ctcacaagga agccgcacca    1080

cacctcgctg aagactacgc ggctgcttac aaaggacaag tgccggctag attcagattt    1140

acatga                                                               1146


<210>  2

<211>  381
<212>  PRT
<213>  Secale cereale

<400>  2

Met Leu Leu Leu Leu Arg Gln Arg Val Leu Ser Ala Ala Pro Ser Pro
1               5                   10                  15

Ser Thr Ser Pro Leu Leu Ser Leu His Arg Leu Leu Cys Ala Ala Ala
            20                  25                  30

Pro Val Asn Pro Ser Phe Ala Val Asp Asp Tyr Leu Val Gly Thr Cys
        35                  40                  45

Gly Leu Ser Arg Ala Gln Ala Leu Lys Ala Ser Ala Lys Leu Ser His
    50                  55                  60

Leu Lys Ser Pro Ala Asn Pro Asp Ala Val Leu Ala Phe Leu Ala Gly
65                  70                  75                  80

Leu Gly Leu Ser Gly Ala Asp Val Ala Ala Val Val Ala Lys Asp Pro
                85                  90                  95

Lys Phe Leu Cys Ala Gly Val Glu Thr Thr Leu Ala Pro Val Val Ala
            100                 105                 110

Gly Leu Thr Gly Leu Gly Leu Ser Asn Ala Glu Thr Ala Arg Leu Val
        115                 120                 125

Ser Leu Ala Pro Asp Lys Phe Arg Gln Arg Ser Ile Val Ser Lys Leu
    130                 135                 140

Asp Tyr Tyr Leu Pro Leu Phe Gly Ser Ile Asp Asn Leu Val Arg Ser
145                 150                 155                 160

Leu Lys His Gly Ala Gly Ile Leu Gly Ser Asp Leu Glu Arg Val Val
                165                 170                 175

Lys Pro Asn Val Ser Leu Leu Ala Glu Cys Gly Leu Gly Ala Cys Asp
            180                 185                 190

Ile Ala Lys Leu Phe Val Gln Ile Pro Arg Met Leu Cys Ala Lys Pro
        195                 200                 205

Glu Arg Val Leu Glu Met Val Ala Cys Ala Glu Ser Ile Gly Val Pro
    210                 215                 220

Arg Gly Ser Gly Met Phe Arg His Ala Leu His Ala Val Ser Tyr Phe

225                 230                 235                 240

Ser Asp Asp Lys Leu Thr Ala Lys Val Asp Tyr Leu Lys Lys Thr Phe
                245                 250                 255

Arg Trp Ser Asp Ala Glu Val Ala Ile Ala Val Ser Lys Gly Pro Phe
            260                 265                 270

Leu Leu Arg Arg Ser Lys Asp Ile Leu Lys His Ser Ser Glu Phe Leu
        275                 280                 285

Ile Thr Glu Val Gly Leu Gln Pro Ala Tyr Ile Ala His Arg Pro Ala
    290                 295                 300

Met Leu Thr Tyr Ser Leu Glu Gly Arg Leu Arg Pro Arg Tyr Tyr Val
305                 310                 315                 320

Val Arg Phe Leu Lys Glu Asn Gly Leu Leu Glu His Gly Arg Ser Tyr
                325                 330                 335

Tyr Thr Thr Leu Ile Ser Thr Glu Lys Val Phe Met Glu Lys Phe Ile
            340                 345                 350

Arg Pro His Lys Glu Ala Ala Pro His Leu Ala Glu Asp Tyr Ala Ala
        355                 360                 365

Ala Tyr Lys Gly Gln Val Pro Ala Arg Phe Arg Phe Thr
    370                 375                 380

<210>  3
<211>  3870
<212>  DNA
<213>  Secale cereale

<400>  3
aggagatcga ccaagtatgg tggcttttct gtactacacg tgattctaaa ggctctgtta      60

gttgatgatg cgctgatact tgctcctatt gtcctagaga ttgtatgtaa actcctaaat     120

atgttacccc tggtgcatgt attgttctgt ggttaaaggt aattaaacaa cccaatgtgc     180

tcgtgtaatt gctattcctt tcgagtcatc attagcaaat gttccaacta cacttgatat     240

atattataat tgtactagaa atgatgaata atatacccag ttcattctgg gaatggcaaa     300

tccaaaataa gcagtgagtg gacgtacaca actatcttat ttgcagccaa aatcaggggc     360

atcaataaat gttgtttaac atgccagatc cagtcaaata cagtgaaaat tcggatatct     420

gcttaagaaa aagggctaaa tggtgttagt ttctgttgca ggtatcaaca ctgaacctga     480

tacttgcatg ataatttact tccacactag aacagctttt tctatgcact gactctggaa     540

tagaaactca tgctctgaac ttttcattta tacactgaga aacttctggt gttataaata 600

ttctttattt ttactctacc tttgtgagca ctgttttgta atgggtcatc gcttcaccaa 660

caaagctgct tcttacttga cagttggatt tagcagcagc taatgcatgt aatttcttat 720

tatttttata gaaaagccgc aaatgctcga ggagctatgt gtaacatgtg aattatgtgt 780

tttcatattc atttgtacaa tgagtcaacc atgcacgcca tcaattcttt tctggagttg 840

ataatcgaat gtaaaaaaca tattatctcc tttccattca tattaatggc atggaagcaa 900

ttagatgcag aatttacagc ggctagataa ttgattgtaa tttactccct ccgtccttta 960

aaaagtgtaa ttccaacttt gttggagggt caaactatct caatgtttga ccgagtttgt 1020

gcaaaaatat atcagcggct gcggttgcgg ttgtgggctt atcgttggag caaccgagca 1080

gtcctttctg attgttttgc tcctcgtggg tggtttttca ggcttccttt ttgggtaaat 1140

ttttaccttt ttaggttttc aggcttcttg caaagcaatt tcgtttgaaa aaaaaaagaa 1200

gagagcctgc tttgagatct ggcgatgcaa atgccaggga ccaaagccac cgctgctgtc 1260

aggctgcaga cgcaaggtta catggtcgct ggaggagaga cattggtttc agccatggcg 1320

gcgcgagctt gctcacgcac tctgaagatg gcaggggaag atcagcgtta tacgtacaat 1380

cgagcgtcgc tatttggtgc agttggccgc tgtttggggg gtgaggaagg ttagatgaat 1440

gtgtaatggc tgggcttttt cgaaccctgt ctgaactctg aagagggccc gaaactatgt 1500

ttaaaccctg ctgtcgtgcg cggtatgctt catttctgtt ctaggacggt gctgattcat 1560

ggtgctggga gggtatgcga gattgatatg tttttatata tagcccggtc gctgctgatt 1620

gatttgaaaa ttgttggccg gtcaaaatcg taaaccatat ccaaaataga atacctcatt 1680

tggataaaga gcttcaaaat tagggaacat agtgaattaa aacaattgaa tgggagagct 1740

tgacaaattg ttgatccggt caaaattgaa tggcccaatt ctaaattgaa tactttaatt 1800

aatcgatcca aataagccat ggtgttatcg aagatgaaaa ggcttatggg tgagccgacc 1860

tcaaagtttt ttgtacctca tggtgatttg agttttttga aacagccgac cccaaagttg 1920

ataggtactc ctattagtga aaaaaattgg ttttatgcat ttcacatttc agttcagttt 1980

tatgcatttg gataaattat tatttgtatt aatgttgttc cgatgttcta gaaatgttgg 2040

actactcatt cggtgataaa gccggtcctg gagaaggcaa gctaatggat caccgtcctc 2100

tgaagttgaa taaagatgac tatgagcgcg ttaagcgaat accatttgag aaggtaggtg 2160

gctggatttt tccaaatgtt tcttgcgtga ttactctttt ttccctatgt gaaaattttg 2220

aattctcgct tagggagcca acttccgtga cctggaaggc gtgagagtaa ggccaaacaa 2280

catcgctgag tttgaccccg agattccacg agtttacctt gagtctggca atccattggt 2340

acgtgtcgta ccgcccgccc aatatcgtca gggcttttct agtcacaatt tctgaagatt 2400

gcatgtctgt atggccaggt ccccgaatat gcaataaagt tcaggagtgg caagtctctc 2460

aggttagtag tgccttatgt tccctgtctt cttttctgaa actatatagg ccgtttggac 2520

ggctgtggtg ggacgagacg gttcctacag tggtaaccag cgcaaggccg catagccagg 2580

tcagctttct cacaagctgc ctcagttttc tgatctgacc atttaggaag cacacaacca 2640

tttgttcagt ttcgacagtg tcaggtccag aatcctctaa acacgtttcg tgttgctgcc 2700

tgcaggccat actacacccg agccaggcct tttttataag ggttgctgcc tgaaggagtt 2760

ggacattttt ataagggttc ctctttcaga tgctattgga gcttgacaag tggctcctcc 2820

tccgctgccc accttgcgtc aggcgcgcac actcgtgagc actgtgaata gtcgacacta 2880

caccttcacc ctaatccacg attctataca taagtggttt tgttgctagg ggtgggccgc 2940

ccaaatctcc tcttgtggtt tctcagcctt ttgcggcgac aatgacggtg ttgagcgatg 3000

tgtcgtgttt gtctatcgaa gttggagttt cctttctttt ttgtatgcct cattttagcg 3060

gcgatttggt gccactgtta cgaggttctt tgcatttcgt tgaggaccaa atacgatgat 3120

attgtggtag tgtcaatgct ccccacttct tgtagtgtgt tttcaaaagc aggtttccga 3180

gtgccagcgt ggctttgacg atggtttccg ttcattttct ttattaaccc gacaaccgcc 3240

ttcttctaat accaccgcag aacttccgcc ctatactttt cggttttgtg tctcccaaaa 3300

aattgtcatg tataacaaat gttgaacaaa ttatgaatgg gaaacgcttc ttatcatacg 3360

tctgatacga agcaaacttc aaacctgtcg ccgccgccct accgattttc tttattaact 3420

actaggcaac tcccttcttc gcaaaaacaa aacaaacaaa acaaaacaag gcaactccct 3480

tcttataata ctacaacgga gccggagcgg cagaaatctc gccccggttg atgattttta 3540

tcgaagaaaa cagtttttct tatggatata gaatttttct tgaccggtat gaataataga 3600

tgtttttcta gggtggggtg tgcataatag atgtttttgc gtgctagtta tatttgggtt 3660

ctgatagact cttggcaacg cgtggatgga aatgcgccgt tggatgtcat ttggatggca 3720

gggattaata atacacttca actcagccaa gttcacttca tttggatgac aagtccgtcc 3780

accacaccac cggagacaca ctcaacgcca ctcgaattcc ccaccgcccg ccgccgcccg 3840

ccgccgccgc tgttccgccg tcgcggcgcc 3870

<210> 4
<211> 20
<212> DNA
<213> Artificial Sequence

<220>
<223> ctg2 Forward Primer

<400> 4
cagcctctgg ttgttgaggt 20

<210> 5

<211>  20
<212>  DNA
<213>  Artificial Sequence

<220>
<223>  ctg2 Reverse Primer

<400>  5
catcgccact gcaaagttta                                                    20


<210>  6
<211>  19
<212>  DNA
<213>  Artificial Sequence

<220>
<223>  P20 Forward Primer

<400>  6
tgtcgaaact gaacaaatg                                                     19


<210>  7
<211>  20
<212>  DNA
<213>  Artificial Sequence

<220>
<223>  P20 Reverse Primer

<400>  7
ggagccaact tccgtgacct                                                    20


<210>  8
<211>  21
<212>  DNA
<213>  Artificial Sequence

<220>
<223>  72F13_c2_mTERF Forward Primer

<400>  8
tcgtcgccaa ggatcccaag t                                                  21


<210>  9
<211>  22
<212>  DNA
<213>  Artificial Sequence

<220>
<223>  72F13_c2_mTERF Reverse Primer

<400>  9
actttagcgg tgagcttgtc gt                                                 22


<210>  10
<211>  22
<212>  DNA
<213>  Artificial Sequence

<220>
<223>  ctg16b Forward Primer

<400>  10
ctccaagaac tctttctcgg tc                                                22


<210>  11
<211>  22
<212>  DNA
<213>  Artificial Sequence

<220>
<223>  ctg16b Reverse Primer

<400>  11
cccaatatga agctcctagc ag                                                22


<210>  12
<211>  23
<212>  DNA
<213>  Artificial Sequence

<220>
<223>  7_01_H_1441 Forward Primer

<400>  12
ggtcatatga agacaccatg tca                                               23


<210>  13
<211>  22
<212>  DNA
<213>  Artificial Sequence

<220>
<223>  7_01_H_1441 Reverse Primer

<400>  13
tttcgccatt ttcgaagtag tc                                                22


<210>  14
<211>  20
<212>  DNA
<213>  Artificial Sequence

<220>
<223>  ctg24met2a5 Forward Primer

<400>  14
aaagagtaca acggtcacag                                                   20


<210>  15
<211>  24
<212>  DNA
<213>  Artificial Sequence

<220>
<223>  ctg24met2a5 Reverse Primer

<400>  15
gaagaatcct cgctatcttt agac                                              24


<210>  16
<211>  20
<212>  DNA
<213>  Artificial Sequence

<220>
<223>  ctg32 Forward Primer

<400>  16
ccgaggaaag aagccaagag                                                   20


<210>  17
<211>  20
<212>  DNA
<213>  Artificial Sequence

<220>
<223>  ctg32 Reverse Primer

<400>  17
ccttgagaat ccgatccacc                                                   20


<210>  18
<211>  20
<212>  DNA
<213>  Artificial Sequence

<220>
<223>  c40745_1 Forward Primer

<400>  18
gtcgctgctg attgatttga                                                   20


<210>  19
<211>  20
<212>  DNA
<213>  Artificial Sequence

<220>
<223>  c40745_1 Reverse Primer

<400>  19
cgttgtttgg ccctactctc                                                   20


<210>  20
<211>  18425
<212>  DNA
<213>  Secale cereale - 175O19-N3_c9

<400>  20
gatttttttt caaaactgtg cctaaattta tacaaaattt gaaaactgtt agtctgttag      60

agaaaaaatc gaatctatga ccccgtcggc cacgactggg ccgaccagcc agccgtcggc     120

ctcggctggg ccgaccaggg gcccgtcggc ctcgccctgg ccgaccagtg gcccgtcggc 180

ctcgctctgg ccgaccagtg cccgtcggcc tggcagcggc cgctcggctc gtcctgtggc 240

cgaacacgag ctaacaacag ccggatcaat ttaacaaaga caagaaaagc tgctgctggt 300

gctgcatcgg tttggttgta ctagttggtg ttgtcacgct gagccggagg tgcaattccg 360

tccacaaaca gagttaggaa aagcgaccac cgtcagccgg tctgccggtg cacgcacgta 420

cgcatgcgcc gaccaggaga attaaaggag aatctgaggc ggagaaaggc tgcgatgcaa 480

cgtgaggacg gtttcttgga tcagaccaga gcccatggtt tctccagttc tcatgcagtt 540

cccgccaatg gagcagtttg attgaggtgc atgcatgaac gtgatggctg actcggattc 600

ttgtgcgctt cctgttggcc tttgattgct tgtcacttct tatggatcgc tccacacgta 660

cgctgcatcg gagtggctct tgttggcatc caaactttga gctggatcca acagatactc 720

aaacgtactt atccagccgc cgctcggccg ctgccaggcc gacgggcact ggtcggccag 780

ggcgaggccg acgggccact ggtcggccag ggcgaggccg acgggcccct ggtcggccca 840

gccgaggccg acggctggct ggtcggccca gtcgtggccg agaggccgcc tttcggccca 900

gttaaggccg acggggtcat agattcgatt ttttctctaa cagactaaca gttttcaaat 960

tttgtataaa tttaggcaca gttttgaaaa aaaatcgaaa tatgtgtatt agtattcgag 1020

agatttgttt catatgcata cttgaggaaa gagtgtagga cgacataagg ggccatggcg 1080

atggcctgca cctgcacgcc cgccatggtg ctcatccttc catttctatt gtgatggctt 1140

gcctgcagaa accaatccaa ccacaggggt catccaaaaa aagaagaaga agaagaagaa 1200

gaagaagaag aggggagggg aggaggatgg agggcgtgcg tgtgtacctt tgaagtctag 1260

gtgctgcagc ccgtcgcggg cgtcgaggca tccatggcct tcctcgagga ccacgtggat 1320

gatggtgcac cggtgccggt gccgctcctc gatctggggc ctcccctccc ctccccttcc 1380

cttcccttgc tttggagagg aaggaaggtg tcacccatgg ctcgccgtgc cgagttgaat 1440

gcccgccgtc cgtctgccaa gaggagagaa ggagatggcg accgagagga gggaggaagt 1500

gaaggcggcg gcggccttca cttcctcctc tcggtcgaga gagaggaggg acgccaatcc 1560

tgttcgctgt agtccaccca atcccacgct caatgcaatc cacctttctc accgaggcac 1620

acccgagaca cacactacag aattgggccc gccgggaaag agccccaccc gtcatcggct 1680

gtagcgcgaa atagtaacgt ccagagaaac ccgtcgtaaa aaactgccag cgaggtagat 1740

cgctcgatcc agactctttg cgcacgcagt tgccccagga tggcgggtaa cacagcatgg 1800

tttatatgtt caattaatgc agagtatatc tgcatgctct acatcttgat gttctcagtg 1860

gcaattgcag ctttcaatgc aatactgagt cagtctatct ttccatgaaa aacggttctc 1920

aatatggcca tgaagatgat atgaaatccc aaattcgttg aactattttt gcgggaacaa 1980

ttctggaggg gttgatggcc acaacgtgtt gatcactgct gagtgtgcag ttttgatata 2040

gtctggtggt tgcttcctct agttcttctt atttcttcgc tgaaagtatt cattcttcct 2100

gcagtagctt tgtttataag atattaacag ttgtgaaagc aggtcatcgt tttctagtac 2160

tttgtatctg tcttggatgg cccactggct gatacttttt ctgggagcat ctgatctcaa 2220

aactgaatag ttggccagta gcatttttgt tgacggccta cccatatagc attacttaat 2280

ggatatatgg aaggccctgg tatttgtact ggagaagcaa attgatgttt gtatggtatg 2340

gtgctgaaaa gcagaggcca tttcattcaa tacaagaata tgttaaggta atattaaaaa 2400

gggttatgac attttattct ttattagcac tatctataga tgctcatgac atttttatcc 2460

attggcatat ttgttttggg attgtaaggt gggtcttatg taccattctc tgttttgtac 2520

tttgcacttt tagatgctct tctatatttg ctttagttgt gctggttaca atcacttctg 2580

aagacacgta tgctgcaatt ggagaaaaga gaatgaagat gtcgaagact gttaccttag 2640

cgaagagatg gtcccaggga tgatatctta accaagcttg gtcttaacat tcaaggtatt 2700

gcagtaattt tttttctcag tttataccga atgtcatctc ttcgtacact cttttcagtt 2760

aaatgtcagt ctaggcatgc aagtactatc tgtcagatat atcatagtta cagaatcttg 2820

cagaatttca aacacagaca ttgtagactt gctggtggtc ctcaaattaa gaatcaagtc 2880

gtgtgggggg aggatggtgc acatttgttt cctgaggtca ggctatagaa atcggaagaa 2940

atgagatatc agctgtgtgc tcccagtaag acaacggaga tgggcacatg ctattattgg 3000

caaagagctc gtatttggaa tgaagatcaa ttgtgtgtct cttctctcgt gcctagttta 3060

gcttttggga attatcgaaa caagattaat gcaatgcagg cgaggggaag agtcgattga 3120

tgaacgaagc gacttgccag gtgaatatat gcattttttt tttcgaaaaa attactttcc 3180

tcaagtaaga attggctggg aaattaagag gagcatactg attgatttac tgaatgttat 3240

agatcaatgg tgctttcttt ctacctagac aagaagtgat tctttcttct ttctaggaga 3300

tcgaccaagt atggtggctt ttctgtacta cacgtgattc taaaggctct gttagttgat 3360

gatgcgctga tacttgctcc tattgtccta gagattgtat gtaaactcct aaatatgtta 3420

cccctggtgc atgtattgtt ctgtggttaa aggtaattaa acaacccaat gtgctcgtgt 3480

aattgctatt cctttcgagt catcattagc aaatgttcca actacacttg atatatatta 3540

taattgtact agaaatgatg aataatatac ccagttcatt ctgggaatgg caaatccaaa 3600

ataagcagtg agtggacgta cacaactatc ttatttgcag ccaaaatcag gggcatcaat 3660

aaatgttgtt taacatgcca gatccagtca aatacagtga aaattcggat atctgcttaa 3720

gaaaaagggc taaatggtgt tagtttctgt tgcaggtatc aacactgaac ctgatacttg 3780

catgataatt tacttccaca ctagaacagc tttttctatg cactgactct ggaatagaaa 3840

ctcatgctct gaacttttca tttatacact gagaaacttc tggtgttata aatattcttt 3900

atttttactc tacctttgtg agcactgttt tgtaatgggt catcgcttca ccaacaaagc 3960

tgcttcttac ttgacagttg gatttagcag cagctaatgc atgtaatttc ttattatttt 4020

tatagaaaag ccgcaaatgc tcgaggagct atgtgtaaca tgtgaattat gtgttttcat 4080

attcatttgt acaatgagtc aaccatgcac gccatcaatt cttttctgga gttgataatc 4140

gaatgtaaaa aacatattat ctcctttcca ttcatattaa tggcatggaa gcaattagat 4200

gcagaattta cagcggctag ataattgatt gtaatttact ccctccgtcc tttaaaaagt 4260

gtaattccaa ctttgttgga gggtcaaact atctcaatgt ttgaccgagt ttgtgcaaaa 4320

atatatcagc ggctgcggtt gcggttgtgg gcttatcgtt ggagcaaccg agcagtcctt 4380

tctgattgtt ttgctcctcg tgggtggttt ttcaggcttc ctttttgggt aaatttttac 4440

ctttttaggt tttcaggctt cttgcaaagc aatttcgttt gaaaaaaaaa agaagagagc 4500

ctgctttgag atctggcgat gcaaatgcca gggaccaaag ccaccgctgc tgtcaggctg 4560

cagacgcaag gttacatggt cgctggagga gagacattgg tttcagccat ggcggcgcga 4620

gcttgctcac gcactctgaa gatggcaggg gaagatcagc gttatacgta caatcgagcg 4680

tcgctatttg gtgcagttgg ccgctgtttg gggggtgagg aaggttagat gaatgtgtaa 4740

tggctgggct ttttcgaacc ctgtctgaac tctgaagagg gcccgaaact atgtttaaac 4800

cctgctgtcg tgcgcggtat gcttcatttc tgttctagga cggtgctgat tcatggtgct 4860

gggagggtat gcgagattga tatgttttta tatatagccc ggtcgctgct gattgatttg 4920

aaaattgttg gccggtcaaa atcgtaaacc atatccaaaa tagaatacct catttggata 4980

aagagcttca aaattaggga acatagtgaa ttaaaacaat tgaatgggag agcttgacaa 5040

attgttgatc cggtcaaaat tgaatggccc aattctaaat tgaatacttt aattaatcga 5100

tccaaataag ccatggtgtt atcgaagatg aaaaggctta tgggtgagcc gacctcaaag 5160

ttttttgtac ctcatggtga tttgagtttt ttgaaacagc cgaccccaaa gttgataggt 5220

actcctatta gtgaaaaaaa ttggttttat gcatttcaca tttcagttca gttttatgca 5280

tttggataaa ttattatttg tattaatgtt gttccgatgt tctagaaatg ttggactact 5340

cattcggtga taaagccggt cctggagaag gcaagctaat ggatcaccgt cctctgaagt 5400

tgaataaaga tgactatgag cgcgttaagc gaataccatt tgagaaggta ggtggctgga 5460

tttttccaaa tgtttcttgc gtgattactc ttttttccct atgtgaaaat tttgaattct 5520

cgcttaggga gccaacttcc gtgacctgga aggcgtgaga gtaaggccaa acaacatcgc 5580

tgagtttgac cccgagattc cacgagttta ccttgagtct ggcaatccat tggtacgtgt 5640

cgtaccgccc gcccaatatc gtcagggctt ttctagtcac aatttctgaa gattgcatgt 5700

ctgtatggcc aggtccccga atatgcaata aagttcagga gtggcaagtc tctcaggtta 5760

gtagtgcctt atgttccctg tcttcttttc tgaaactata taggccgttt ggacggctgt 5820

ggtgggacga gacggttcct acagtggtaa ccagcgcaag gccgcatagc caggtcagct 5880

ttctcacaag ctgcctcagt tttctgatct gaccatttag gaagcacaca accatttgtt 5940

cagtttcgac agtgtcaggt ccagaatcct ctaaacacgt ttcgtgttgc tgcctgcagg 6000

ccatactaca cccgagccag gcctttttta taagggttgc tgcctgaagg agttggacat 6060

ttttataagg gttcctcttt cagatgctat tggagcttga caagtggctc ctcctccgct 6120

gcccaccttg cgtcaggcgc gcacactcgt gagcactgtg aatagtcgac actacacctt 6180

caccctaatc cacgattcta tacataagtg gttttgttgc taggggtggg ccgcccaaat 6240

ctcctcttgt ggtttctcag ccttttgcgg cgacaatgac ggtgttgagc gatgtgtcgt 6300

gtttgtctat cgaagttgga gtttcctttc ttttttgtat gcctcatttt agcggcgatt 6360

tggtgccact gttacgaggt tctttgcatt tcgttgagga ccaaatacga tgatattgtg 6420

gtagtgtcaa tgctccccac ttcttgtagt gtgttttcaa aagcaggttt ccgagtgcca 6480

gcgtggcttt gacgatggtt tccgttcatt ttctttatta acccgacaac cgccttcttc 6540

taataccacc gcagaacttc cgccctatac ttttcggttt tgtgtctccc aaaaaattgt 6600

catgtataac aaatgttgaa caaattatga atgggaaacg cttcttatca tacgtctgat 6660

acgaagcaaa cttcaaacct gtcgccgccg ccctaccgat tttctttatt aactactagg 6720

caactccctt cttcgcaaaa acaaaacaaa caaaacaaaa caaggcaact cccttcttat 6780

aatactacaa cggagccgga gcggcagaaa tctcgccccg gttgatgatt tttatcgaag 6840

aaaacagttt ttcttatgga tatagaattt ttcttgaccg gtatgaataa tagatgtttt 6900

tctagggtgg ggtgtgcata atagatgttt ttgcgtgcta gttatatttg ggttctgata 6960

gactcttggc aacgcgtgga tggaaatgcg ccgttggatg tcatttggat ggcagggatt 7020

aataatacac ttcaactcag ccaagttcac ttcatttgga tgacaagtcc gtccaccaca 7080

ccaccggaga cacactcaac gccactcgaa ttccccaccg cccgccgccg cccgccgccg 7140

ccgctgttcc gccgtcgcgg cgccatgctc ctcctcctcc ggcagcgcgt cctctccgct 7200

gcgccatctc cttccacctc cccactcctc tctctccacc gcctcctctg cgccgccgcg 7260

cccgtcaacc ctagcttcgc cgtcgacgac tacctcgtcg gcacctgcgg cctcagccgt 7320

gcccaggcac tcaaggcatc cgccaagctg tcccacctca agtcccccgc caaccccgac 7380

gccgtcctcg ccttcctcgc cggactcggc ctctccggcg ccgatgtggc ggccgtcgtc 7440

gccaaggatc ccaagttcct ctgcgccggc gtggagacaa ccctggcccc cgtcgtcgct 7500

gggctcaccg gcctgggtct gtcaaatgct gagactgcgc gcctcgtctc gcttgccccc 7560

gacaaattcc gccagagatc catcgtctcc aagctagact actacctgcc gctcttcggc 7620

tccatcgaca acttggtccg gtcgctcaaa cacggcgccg gcatcctcgg ctccgacctc 7680

gagagggtgg tcaagcccaa tgttagtctc ctagcagagt gcgggctagg tgcttgtgat 7740

attgccaagc tgttcgtcca aataccgagg atgctgtgtg ctaaaccaga gcgtgtcctg 7800

gagatggttg cgtgtgccga aagtataggt gtgccccgtg gctctggaat gttcaggcac 7860

gcgctgcacg ctgtctcata cttcagcgac gacaagctca ccgctaaagt ggactacttg 7920

aagaagacat ttaggtggtc ggatgccgag gttgccattg ctgtgtccaa gggtccattt 7980

ctgctgagga ggtcaaagga tattctgaag cacagctccg agttccttat cactgaggta 8040

gggttgcagc cggcctacat tgctcatcgg ccggctatgc tcacttacag cctggagggc 8100

cggctcaggc cccgctacta tgttgtgaga tttctcaagg aaaatggatt gctagagcac 8160

gggcggagct actatacaac actgattagt actgagaagg ttttcatgga aaagttcata 8220

cgccctcaca aggaagccgc accacacctc gctgaagact acgcggctgc ttacaaagga 8280

caagtgccgg ctagattcag atttacatga accaagaatg ggctatgaga attggtaaac 8340

tgtgtacggt gcaacaaagt tttcattttg ttgaaagctg gtaattcctg tttcagtgta 8400

ctttgcctaa ctggatgttt gttgtccgct ctgttagatg ctagtatggt ctttgccaac 8460

gcttgatgaa actgatagtt tgaatcctga tatgttagtg ttatcatttg gtagagcaga 8520

aacagtgact tggttatgaa cttgtccatc ctcatgccat gttatttaac acttggaact 8580

aagtaaacaa gaacaggcca ttgtacattc cactaaaatc tctattttaa aaatttaatg 8640

tattctgggt gatgtttgct tgcaccgatg ggcataggtt tgctccgtga ctctgggata 8700

ttcaggcatg ccctgcgcat tgtcgcattc cttggcagga aaaatatagc tgccaaaatg 8760

gaatacctga agaagacatt caggttgact gatgccgagg gtaagtattc ccgtgtcttt 8820

aaggttctga ttttgttgag gacatccaag gaattggttc agagaaagtt tgagttcctc 8880

atctctgagt tggggttgga acagtcttac gttgcttatc gtccagcaat gttctattgt 8940

gaccaatgtt ctgctgtaaa gacaattaac aaatttaaat gcttactgca cctcaaaggt 9000

tttacttttt ggcaccttaa gctggcaagc tttgttttag tgttcttccc tgacaagatg 9060

tttgttatct ggtatggtat gtctgtcgcg tgctagttag gtcattgcta gcacttgatg 9120

aagctagtca ttacatccac gagtcctcat atttcattat aatttttttc tttctgaatg 9180

tcaattctgg accctctttt tgtgaacagt tttggactaa ttgatggtca cagtacgttt 9240

accaatccga ggattctgtt ttgatccagt ttgttagttg cttgttctat aaattgatgg 9300

tcacagtgac ttttttggaa gtggttcggc cagtgcgtat atcgcttgga gaatggctga 9360

agaagagtgg tttggctctc tgcctgctat ctatcagtga gcttgtttgg atttcgttca 9420

gtccgagagg aggatgcgga cttgatccag tctgttggtt gctgtgctct agttcgcgtg 9480

acctggaaat cttcggtata tctgccaaac ataaattaag tttaatatcc aagttcagtc 9540

tggaagacat tcaggttgac tgatgccgag ggtatgtatt cctgtgtctt taaggttctg 9600

attttgttga ggacatccaa ggaatcagtt tagcaaaagt ttgagttcct catctctgag 9660

ttggggttgg aataggcttg gagggtaaag tttcttaaga caattaacaa atttaaatgc 9720

ttactgtacc acagaggttt tacttggcac cttaagctgg caacctttgt ttcagtgttc 9780

ttgcctgaca agagttggtt gtcaggtctg gtatgtcttt cacatgctag gttggtcatt 9840

gctaacactt gatgaagcta gtaattggat ccatgagttc tcatatgtcc atatcatcat 9900

atatttgtct ctgaatgtta attctggact ctctttttcg gaacaatttt ggactaattg 9960

atggtcacaa tacatcgatc aatccgagga ttcagttttg atccagtttg tttgttgctt 10020

gttctaattc ctgtgagctg aaaatcttcg ttctttctgc agtatatata tatatatttt 10080

gaaatgatta aatgttgtga aagtaattca tgctttacta gtctgtatca gatttttcca 10140

tattttttct ccagtagtat ttcttaatgg cctatttatt ggctgataca ttcagttcta 10200

gggacaagtt atctcaaaac tgaacaatag agttgtacac tttctattta cggcctacca 10260

tctagtccaa ttaaatgcta tggctggtta aaacaaagcc tcatcaggtt ttcaccaatc 10320

aggcttacat gtgtctgaag ctgtgttgtt atatcatgat cgatgctagc gaatgattga 10380

aataatttat ttctgccttg aatgacctca ggtgttaacc tcgctgttct gtatcatttg 10440

gaaaatgaga aattgtactg gttttcatag gtggctggtg gcaagtgatg aaaactggcg 10500

tcctgcgctt ggagaatggc tgaagaatag tggtttggca ctctgtctgc tatctatcag 10560

tgagcttgtt gggatgtcgg tcagtacata aatcttttgg catttctgcc caacataaat 10620

tttgttggaa ccgtgtgcta tataggtttg ttcggtagta actccccatt tgtgctgctg 10680

cgcatatgtt tgccgtttac atttgcacct tatggtttaa taagacctcc tgctccagca 10740

gcttgtcaaa attcagcacc tatacatgac aatgctgcat ttgtttgttg ttggctgagt 10800

tacataggga gtatgttgtg atagtgattt acagttaacc cttttttgac cccttgctac 10860

catagctttt tctttcaagt cagtagcact catgccatgc gcgattatat aatgtacaat 10920

atctctcaat ttttttggaa gtggttcagt cagtgctgta acaggagggc tcaccttgga 10980

agggcggcga gtattgcttg gggccgtaga gttgtctgtt gcacaactcc cgtgtggtcc 11040

agccgacctc cgggtagcca acccaaccct gcagagaaaa cgaccacctc cttctccatg 11100

gccggcaaga ctctacatcc ctatgcttga cggagagcgc cgcctggatg ccggagaagg 11160

tgttcacggt caggagctgg tgctcgtggt cgtcgaagat acagccacca tcatgggagg 11220

caatatattt taacaaagag gtcaataaaa agtactccct ccgtcccata atgtaagaca 11280

ttttttgaat ctagtgtagt gtcataaaac atcttacatt atgggacgga ggggtagtta 11340

gcttataact cgttgttaga acatagatca agaatgatgt agtccaagtg tcttgataga 11400

atgcatcaaa attttctatt tataaatttc aacgcaatac aaaatggcct attctcaatt 11460

acatagttcc aggtgttaaa tagcatggca tgatggtctc cccagatgat cactctcttg 11520

gccgggtctt ccgggtggac taggaatgtt ttgtctcctt tgtcggctgg aaagccagat 11580

ctttccttgg cttgattagt actagcattt ttgcacccgg attggcgaac ctcttgatag 11640

catcgagctt cactgacgct tgtgcgacct caacgagcgt cacgttcgcg acttctgctt 11700

tgagccctct ctcagtgctg ccatgaattg ttatcatggt gctcagagct agcatgatga 11760

gctagaagca cggataggat aggaggatag cgtcatgaaa ttgacgtagg cgccaagaat 11820

gaggcgactt tgaaccagat tatttccgag ctgaagttct cctcgtccca agggataacc 11880

tgccgaccga actatccgag ggggtgcacc tgcaaaacgg tacgagactg tggaagccag 11940

tggtcaaggg gaggaaatga gaccgggaga tccccatctt ggacagagtg taggggaaga 12000

tgatgtttac gccactttca ccgtctgcca gaactcgggg aaagcagaag ccctcgatga 12060

tcggatctac aaccaaggca aatttcccag atgggaagca taggttgaat gattggttca 12120

ggcaaaggca accgctcgag tctaatcact gcaggtcctc tgttggtaat ggtgcaatca 12180

cttaaaccat gggtcaagtg aatttctgag cccggcgcat gtcagcggct tgaatgatca 12240

tgttgacgcc atggtatcga gtaggatagt gggttgctga aggccctgcg gttggttgtg 12300

accagtgcta ccccacgcga taagggtggt ggaggggtgc agaggcacgt gatcttgcag 12360

cgacgatcaa agaggaactt gagcctccta aagtatattg catcgatgta gggtggcgtg 12420

acgtcacaga gtagcccatg gttttcgacg gtgagctcga aagagccgaa agtgaagatt 12480

tggaatgaca tgtagtcatt tggcatgaac cgagatccta tctgggttca ttctttgact 12540

agtgcagccc ttgccgggtg caccaactaa tagtacgtgg aaatgccaat acccgtagta 12600

tcgtggcgaa cgcagccata agtaccagaa gggattatac acaacaaggt tacccaggtt 12660

cggaccatct gggataggta atatcctatg ccatgcttct aatatgtatt catggtcgag 12720

tacctcatgc aagtgataac aatgaagatg atgtataagc taccgacatt atatttctat 12780

gagagcatat gtgcatacct actcctagac atgtctcata tcgatggcga gggctagggt 12840

ttacagagat caatacgatc tgaggacgcc gccatcttgg cttgtatgcc aagatgatct 12900

cccaatcctt atccaggttc ctgggccgac ccctagggcc gggtcttcct taggcttcct 12960

gggccctggg gccagcttgc tgcctggttc cctccaaaag gccaccctgt tgtgttggac 13020

cgtcaacctc taaagtctaa ggacatgccc tgttactaca tagccaaagc gaccatagta 13080

aggaagacgc tcacatccct gttatcgtac taaacttatt aggtattctg tcaagccagt 13140

gataatataa attggcaaca cttttgggcg aatacaaaat agatgccatg ttcatgacta 13200

accaaacaca caaacttatg ttgaaataaa aggtgtttga ttgtctagtt gtgagcacga 13260

aaactacacc tattgcttcc ataccatcta tggcggccga ggtcgtcctt agtcagtaca 13320

atcactcgac aaagatatca catgaaaata ttgactttta gtggtatctt caatttccaa 13380

atttgatcat taatgccatg tggaaacttt gtgtgtgaaa gcgatgggta cataaattca 13440

atggtgaagg actcattagt agttagattc cagcaaaaca tgatatgtcc ttgtgttacg 13500

ctaaccgact acaaacggga tagtagattt ttttatgata caagagggtc aggggcggag 13560

ccaggattta gacatagggg ggcgagaccg gcggtaaggc caaatttttt tccctcccaa 13620

actagctgct acgttgcacg gatacttgga tacgtatcgg atatgcgata cggggatacg 13680

cccgatacgt cagttttcta aaaacatgga tacggggata cgttcatata tagatgttac 13740

atatattaat tattacaaat atgaagaaaa aattaaggag cttcttggtc aaagcatgcc 13800

tcaatacgat tactcctttc ttttcttgca cttagtccac ttccattaat tggcagtgag 13860

atttgatcag gtttgctgtt caaatcaaat taattattac aaatatgaag aaaaaattaa 13920

ggagcttcta ggtcaaagca tgtctcaata cgattactcc tttcttttct tgcacttagt 13980

ccacttccat taattgacag tgagatttga tcaggtttgc tattcaaatc aatgcatcga 14040

ctctctctta ctcacctttc aattgaataa aaaacattga catacatgtt ttcacatgaa 14100

ctcacgccca tgttgggaat cttcaaaggt gcgtctttat tattgagggg gttttacgtc 14160

gaacgaggga tttcaggtat ccaaaacgga atggctttac agacgacaca gactgcacga 14220

tctgtgaacg ataacactgt acatgcatta gcaaacagaa aaaacaattg cctcctgcat 14280

gtacgcgcgt tgctggcgct gccacatgcg atcgtgcacg catcggctgt aatttcgtcg 14340

tgtgtatagc agctctctat ccaaaacgta tcgcagaagt atcctaggag tatcaaacat 14400

tattttcttt tttttaatat caaaatttcg taggatactt acaagatatg tatcgggaag 14460

tatcggggca gtatccggct ggatatggac actttctgaa gtatccgtgc aacgtagact 14520

agctgaagcc aactagtttt acatccgagt acattcatcc ttaactttgc atagacacac 14580

acaacatttt gcatctgaag ccgactagac aaagttgata atcaaactac cctgaaatat 14640

gacatgaaat gtgctgctac aaaacaatat catatgtaca aataagaaaa atcattgtat 14700

aaccgaatga ttggctttta gcttgcattt aaataggttt aaatggcaaa tctgttatgc 14760

aactttgttg ttgtactaaa aaaagatcaa tgttgtcaga atacatacat agagaatcaa 14820

atccagcccc ctactcgtcc cctctaccgc taggtcgacg tagaccggct ggtcgtggtt 14880

ggaaggtgca tgatttcgaa cggttgcttg tgcctgagcg cgacgcgtca caaatcacaa 14940

ccaaaagtta aaatctgatg tataacgaat taatgataca agatgagaat tgtgagagat 15000

tagtagatca ctggttgcag caggaataat tggttgcagc acgaataatt ggcgagtaaa 15060

tcactgaacc agcttgcgtt tacgaatcag atcatgagaa gagatagcag aggcgggcgg 15120

cggcgagggc gctggagggc aacagcgcag cgctaataaa ctcacgggct aatcacagtg 15180

gataatgccc aacgctcagg agacatttaa cggaggccaa tcaacaaata tggccttgtc 15240

atgggccttt aaacgatcgc tggtgggctt ttacattggc ccttttcctt tcttcataaa 15300

tcatgttgag ttaggggggg cctattactt ctgtacctga atcatatcgc taatatctat 15360

ggcctactcg atgtttgctc gatcgatagg gggggccagg cccctgctcg accccccctt 15420

gtctccgcca ctgaagaggg tcaaccaaat cccgtacttc tgaaggatat atcagatgga 15480

gataagctga gaacttgagc aagcgtatca ttcttatggg tacaatatgt tacaaggcga 15540

gatactgttc ttgaagagtg gtgttgtcca accacttgtc cttccagaaa cggatctcag 15600

agtcatcctt tattttggta gatttgtact gggaaagatg tttacttgct gccatgaggc 15660

ctgaccataa atgtgaatca ccagcagttc ttttagtgat gcatcatgca tggcaaccat 15720

ggagcaaaag agaacccact gaagatggtc atgttctgcc atggcctttt tatctcatgc 15780

atgttggagc gagcttgcta ggaatgagag gcaagtgaaa gcgaaggcgc acttcaatta 15840

cctccaggtt ggtgactctg tatcttgaaa catctaaatt taaattctct tgctttaatc 15900

acctccaatc acacaaacta tagacatttg agaaagtatt ggtaggtgtg cattaaggaa 15960

acgtgggaaa tattgcagct ctataaatta cgaaggatgg agttgctttt agaaaactac 16020

caaagagcaa ctatatattt cttagagtga tgcatcagga atcacaagta ttggactgaa 16080

catggtgacc ttgaacatag caagcttatt gatcactgtc atggcctttt ggtctcttca 16140

agcttgttgt agctaactag ctaggattag gattgagagg caactggaag caaatgcgca 16200

ctttgataac ataggtgaca tcatagactg gttggtcaat aagtaagatg acacaaagag 16260

gttctttcag ctagcggatt tgtccacatc cctgtcattc ctttgcaaga accaatgctt 16320

ctcacgctga ccaacacact gtgtgtttcc atcatccaag tgattggcag cctcgtctag 16380

gtgatcaaca actagctcat cgccagccaa cttaacatgg actctggatc catgttatct 16440

gcaatgagct aagaaccact caagaaaaat gtggcacggg ataatcacat gatcatctcc 16500

tccttcacta ggtgcccaag agaaaactgt ttgatacatg aacacaacat ttctgtttgt 16560

gccttttgtg gagcaatttc cccgtgtgtc agaggtcaga ccatttcgaa cgtgccaaat 16620

aaaccaaaga gtcatgagca gcttcttgcg ctccatctct tgcgaattca taacatcgtg 16680

acaccgctga tcattttaat gaaaaatgat gagactgcga gcttttttga tctggaaaag 16740

cattgtcatt cagataatgc agagacaata tgataccctg ctcctagtgc ttgatgttgc 16800

tctgtacaga aaacttcttc atggaatgga atttaatttt ttaatagatt cttcttatca 16860

tgactttctt acgtgttagt atggggatga tactagttgc ttatagtact ccctccggtc 16920

atttttactc tgcgtattag atttgtatca agtcaaactt tccaaagttt taccaaattt 16980

atattaaaaa tatcaacata tataatatca aatatatgta ctgtgaagct aagtttcata 17040

acgaatctaa cagtattgat ttggcattgt agatgttgat aatttttttt ataagtgtag 17100

tcaaagtaga gatactttga ctttagacaa aatgtatatg cagaataaaa agaaccgaag 17160

agagcacata ctttctagga attcagacca aacataccca aacattgtat gtaaagagat 17220

gcatgcaaca attacacaca aacatgtgga gctctgcacg catgtggcgc taatatacta 17280

ctactagtac tccctccgtc cgggtgcata gggcgcctaa gagaattcta aaattccaaa 17340

aatgttagac gtaattcaat taaatgctcc tcgtttattt aaatcgtctc gtttttctcc 17400

gttcgcttca ccaggtcgca ctccttctct taatctccca ccggtccacc tccaagtaat 17460

ctcgtgtgtg aaagcaaata gcatgcatgg ccatacgtga gagaagtaaa gagccaatgc 17520

atgcaccgta gcccggagct gattagacga gcaaattgtg cgggtgggct gtgcgacgtg 17580

ggacttttcc tttgcatgag ttagcccaaa cggccagcac taatgatagg attaatttcc 17640

aaattcagtt tcgattttga ggaaaacctg cgcacgcttt catccaatct caacccacct 17700

aaatcgcaga gctttttctt agacgcccta tgcacctgga cggagggagt acctcctttc 17760

cggtttatag ggctcaattc aaaaatctca tcaaccaaga tagatggtga gtggtggaac 17820

aatttttgta gtttgcatag gcacacaatt aatgcactcg ttttcctcac aattttatgt 17880

ttaccaatgc attaattgca atgcatgcat acataaagta aatgcattga tcaattttct 17940

cttaatcttt gcatgcaatg atttaatgca ccttgaaatc tgaacatgtg atggggaaca 18000

atcaaattga gcattataaa acggaaaatc taaatttttg ggataagccc tataaaccgg 18060

aaaggaggga gtagctgcat tgcatgtagt tgcagcggac tcaagaaacc actactactg 18120

tatgtgttgc agtagtaaag aagcttgatt ggtcccaact accactacta ctacaattac 18180

acacaaaccg acatgtgttg tactccctcc attttcttat acaaggccat tatgaaaaat 18240

atattttgca tctatataag gccactaaca gtaattgagg caaaaattaa tgaagttttc 18300

ttgtattagc aactttttta atatttgaga gtttttaaga tgacattgta ccttcgtggt 18360

gattaataca aggaacatct caatctaacc cttagactag ccataatggg tagtaacata 18420

gagta 18425

<210> 21
<211> 21
<212> DNA
<213> Artificial Sequence

<220>
<223> tc256739 Forward Primer

<400> 21
cccacctcaa gtctccctcc a 21

<210> 22
<211> 21
<212> DNA
<213> Artificial Sequence

<220>
<223>  tc256739 Reverse Primer

<400>  22
acctcggaga tgaggaactc g                                                 21


<210>  23
<211>  20
<212>  DNA
<213>  Artificial Sequence

<220>
<223>  tc300731 Forward Primer

<400>  23
gctgcaacag cagaaaagag                                                   20


<210>  24
<211>  20
<212>  DNA
<213>  Artificial Sequence

<220>
<223>  tc300731 Reverse Primer

<400>  24
gctgatcgag aattcccttg                                                   20


<210>  25
<211>  23
<212>  DNA
<213>  Artificial Sequence

<220>
<223>  RB1R Primer

<400>  25
ctgaatggcg aatgctagag cag                                               23


<210>  26
<211>  20
<212>  DNA
<213>  Artificial Sequence

<220>
<223>  UBIF Primer

<400>  26
ctgcagtgca gcgtgacccg                                                   20


<210>  27
<211>  23
<212>  DNA
<213>  Artificial Sequence

<220>
<223>  RB2R Primer

<400>  27
cgtttcccgc cttcagttta aac                                              23


<210>  28
<211>  1158
<212>  DNA
<213>  Secale cereale

<400>  28
atgctccgcc tccggagttg cctcgtcgcc cacctcctat cctctcccac cccctcccca      60

ctcccctctc tccaccgcct cctctccgcc gccgccgcgc ccgccgtctc ccccagctcc     120

ggcttccaag tggaggacta cctcgtctcc acctgcggcc tcacccgagc gcaggccctc     180

aagaccgccc ccaagctctc ccacctcaag tcccccgcca accccgacgc cgtccgctcc     240

ttcctcgccg gcctcggcct ctccggcgcc gacgtcgcgg ccctcgtcgc cagggacccg     300

ctcttcctct gcgccggcgt ggagggaaac ctgggccccg ccgtcgccgg gctcaccgac     360

ctcggcctct cgcgctccga ggtcgcgcgc ctcgtctcgc tctccccgga ccgattccgc     420

cgcaagagcg tcgtccccaa ggtgcggtac tacctgcctc tcttcggctc ccccgcggac     480

ctcctctcgg gggtcaagac cggcctgttc cttctctccg tcgacctcga ccgggtcgtc     540

aagcccaacg tcgccgtcct gcgcaagtgc gggctagatg tttgtgatat tgccaagctg     600

ctcatccaaa tgccgaggat cgtcaccgcc agcccggggc gcaccctcgc gatggtcgcg     660

tgcgccgagc gcttgggtgt gccccgtggc tccgggatgt ttaggcaggc gctgcaggcc     720

gtcgcatccc tcagcgagga caagattgcc gccaaagtgg agcagttgaa gaagacactg     780

aggtggtcgg atgccgatgt cggcattgct gtctgcaagt ggccggctgt gctgaggtgg     840

tcaagggaca tgctgcagcg caagtccgag ttcctcttct ctgaggtggg cttggaaccg     900

gcgtacattg ctcaccgtcc ggcaatgctc ggtcttagct tggagcgccg cctcaagccc     960

cggtactatg ttatgaggtt tcttaaggaa aatggattgc tcagtcatgc cagagactac    1020

tattgtatgg tcttggtcag cgagaaggta tttgtggagc ggttcatacg cccccacaag    1080

caagctgcac cacacattgc tgaagactat gcagccgctt gcatagggga ggtgcctgct    1140

agattcagat ttacatga                                                  1158


<210>  29
<211>  385
<212>  PRT
<213>  Secale cereale

<400>  29

Met Leu Arg Leu Arg Ser Cys Leu Val Ala His Leu Leu Ser Ser Pro
1               5                   10                  15


Thr Pro Ser Pro Leu Pro Ser Leu His Arg Leu Leu Ser Ala Ala Ala

20                  25                  30

Ala Pro Ala Val Ser Pro Ser Ser Gly Phe Gln Val Glu Asp Tyr Leu
        35                  40                  45

Val Ser Thr Cys Gly Leu Thr Arg Ala Gln Ala Leu Lys Thr Ala Pro
    50                  55                  60

Lys Leu Ser His Leu Lys Ser Pro Ala Asn Pro Asp Ala Val Arg Ser
65                  70                  75                  80

Phe Leu Ala Gly Leu Gly Leu Ser Gly Ala Asp Val Ala Ala Leu Val
                85                  90                  95

Ala Arg Asp Pro Leu Phe Leu Cys Ala Gly Val Glu Gly Asn Leu Gly
            100                 105                 110

Pro Ala Val Ala Gly Leu Thr Asp Leu Gly Leu Ser Arg Ser Glu Val
        115                 120                 125

Ala Arg Leu Val Ser Leu Ser Pro Asp Arg Phe Arg Arg Lys Ser Val
    130                 135                 140

Val Pro Lys Val Arg Tyr Tyr Leu Pro Leu Phe Gly Ser Pro Ala Asp
145                 150                 155                 160

Leu Leu Ser Gly Val Lys Thr Gly Leu Phe Leu Leu Ser Val Asp Leu
                165                 170                 175

Asp Arg Val Val Lys Pro Asn Val Ala Val Leu Arg Lys Cys Gly Leu
            180                 185                 190

Asp Val Cys Asp Ile Ala Lys Leu Leu Ile Gln Met Pro Arg Ile Val
        195                 200                 205

Thr Ala Ser Pro Gly Arg Thr Leu Ala Met Val Ala Cys Ala Glu Arg
    210                 215                 220

Leu Gly Val Pro Arg Gly Ser Gly Met Phe Arg Gln Ala Leu Gln Ala
225                 230                 235                 240

Val Ala Ser Leu Ser Glu Asp Lys Ile Ala Ala Lys Val Glu Gln Leu
                245                 250                 255

Lys Lys Thr Leu Arg Trp Ser Asp Ala Asp Val Gly Ile Ala Val Cys
            260                 265                 270

Lys Trp Pro Ala Val Leu Arg Trp Ser Arg Asp Met Leu Gln Arg Lys
        275                 280                 285

Ser Glu Phe Leu Phe Ser Glu Val Gly Leu Glu Pro Ala Tyr Ile Ala
    290                 295                 300

His Arg Pro Ala Met Leu Gly Leu Ser Leu Glu Arg Arg Leu Lys Pro
305                 310                 315                 320

Arg Tyr Tyr Val Met Arg Phe Leu Lys Glu Asn Gly Leu Leu Ser His
                325                 330                 335

Ala Arg Asp Tyr Tyr Cys Met Val Leu Val Ser Glu Lys Val Phe Val
            340                 345                 350

Glu Arg Phe Ile Arg Pro His Lys Gln Ala Ala Pro His Ile Ala Glu
        355                 360                 365

Asp Tyr Ala Ala Ala Cys Ile Gly Glu Val Pro Ala Arg Phe Arg Phe
    370                 375                 380

Thr
385

<210>  30
<211>  1146
<212>  DNA
<213>  Secale cereale

<400>  30
atgctcctcc tcctccggca gcgcgtcctc tccgccgcgc catctccatc cacctcccct      60

ctccaccgcc tcctctccgc ggccgcgccc gccgtttccc ggaaccctag cttcgccgtg     120

gaggagtacc tcgtctccac ctgcggcctc acccgtgccc aggcactcaa ggcctccgcc     180

aagctctccc acctcaagtc ccccgccaag cccgacgccg tcctcgcctt cctcgccgga     240

ctcggcctct ccggcgccga catcgctgcc ctcgtcgcca aggacgcgcg gttcctctgc     300

gccggcgtgg agagaaccct gtcccccatc gtcgctgggc tcaccggcct tggcctgtca     360

aatgctgaga ctgcgcgcct cgtctcgctt gcccccgaca aattccgcca gagatccatc     420

gtctccaagc tagagtacta cctgccgctc gtcggctcca tcgacaactt ggtccggtcg     480

ctcaaacacg gcgccggcat cctcggctcc gacctcgaga gggtggtcaa gcccaatgtt     540

agtctcctag cagagtgcgg gctaggtgct tgtgatattg ccaagctgtt cgtccaaata     600

ccgaggatgc tgtgtgctaa accagagcgt gtcctggaga tggttgcgtg tgccgaaagt     660

ataggtgtgt cccgtggctc tggaatgttc tggcaagcgc tgcacaccgt cgcatacgtc     720

agcgtggaca atatcgctgc cagagtggac tacttgaaga agacgtttag gtggtcagat     780

attgaggttg gcattgctgt gtccaagggt ccatttctgc ttaggaggtc aaaggatatg 840

ctgaaacaca ggtcggagtt ccttatcact gagctagggt tgcagccggc ctacattgct 900

catcggccgg ctatgctcac ttacagcctg gagggccggc tcaggccccg ctactatgtt 960

gtgagatttc tcaaggaaaa tggattgcta gagcacgggc ggagctacta tacaacactg 1020

attagtactg agaaggtttt catggaaaag ttcatacgcc ctcacaagga agccgcacca 1080

cacctcgctg aagactacgc ggctgcttgc aaaggacaag tgccggctag attcagattt 1140

acatga 1146

<210> 31
<211> 381
<212> PRT
<213> Secale cereale

<400> 31

Met Leu Leu Leu Leu Arg Gln Arg Val Leu Ser Ala Ala Pro Ser Pro
1               5                   10                  15

Ser Thr Ser Pro Leu His Arg Leu Leu Ser Ala Ala Ala Pro Ala Val
            20                  25                  30

Ser Arg Asn Pro Ser Phe Ala Val Glu Glu Tyr Leu Val Ser Thr Cys
        35                  40                  45

Gly Leu Thr Arg Ala Gln Ala Leu Lys Ala Ser Ala Lys Leu Ser His
    50                  55                  60

Leu Lys Ser Pro Ala Lys Pro Asp Ala Val Leu Ala Phe Leu Ala Gly
65                  70                  75                  80

Leu Gly Leu Ser Gly Ala Asp Ile Ala Ala Leu Val Ala Lys Asp Ala
                85                  90                  95

Arg Phe Leu Cys Ala Gly Val Glu Arg Thr Leu Ser Pro Ile Val Ala
            100                 105                 110

Gly Leu Thr Gly Leu Gly Leu Ser Asn Ala Glu Thr Ala Arg Leu Val
        115                 120                 125

Ser Leu Ala Pro Asp Lys Phe Arg Gln Arg Ser Ile Val Ser Lys Leu
    130                 135                 140

Glu Tyr Tyr Leu Pro Leu Val Gly Ser Ile Asp Asn Leu Val Arg Ser
145                 150                 155                 160

Leu Lys His Gly Ala Gly Ile Leu Gly Ser Asp Leu Glu Arg Val Val
                165                 170                 175

Lys Pro Asn Val Ser Leu Leu Ala Glu Cys Gly Leu Gly Ala Cys Asp
            180                 185                 190

Ile Ala Lys Leu Phe Val Gln Ile Pro Arg Met Leu Cys Ala Lys Pro
        195                 200                 205

Glu Arg Val Leu Glu Met Val Ala Cys Ala Glu Ser Ile Gly Val Ser
    210                 215                 220

Arg Gly Ser Gly Met Phe Trp Gln Ala Leu His Thr Val Ala Tyr Val
225                 230                 235                 240

Ser Val Asp Asn Ile Ala Ala Arg Val Asp Tyr Leu Lys Lys Thr Phe
                245                 250                 255

Arg Trp Ser Asp Ile Glu Val Gly Ile Ala Val Ser Lys Gly Pro Phe
            260                 265                 270

Leu Leu Arg Arg Ser Lys Asp Met Leu Lys His Arg Ser Glu Phe Leu
        275                 280                 285

Ile Thr Glu Leu Gly Leu Gln Pro Ala Tyr Ile Ala His Arg Pro Ala
    290                 295                 300

Met Leu Thr Tyr Ser Leu Glu Gly Arg Leu Arg Pro Arg Tyr Tyr Val
305                 310                 315                 320

Val Arg Phe Leu Lys Glu Asn Gly Leu Leu Glu His Gly Arg Ser Tyr
                325                 330                 335

Tyr Thr Thr Leu Ile Ser Thr Glu Lys Val Phe Met Glu Lys Phe Ile
            340                 345                 350

Arg Pro His Lys Glu Ala Ala Pro His Leu Ala Glu Asp Tyr Ala Ala
        355                 360                 365

Ala Cys Lys Gly Gln Val Pro Ala Arg Phe Arg Phe Thr
    370                 375                 380

<210>  32
<211>  1158
<212>  DNA
<213>  Secale cereale

<400>  32
atgctccgcc tccggagttg cctcgtcacc caccttctat cctctcccac cacctcccca        60

ctcccctctc tccaccgcct cctctccgcc gccgccgcgc ccgccgtctc ccccagctcc 120

ggcttcgacg tcgacgacta tctcgtctcc acctgcgggc tgacccgagc gcaggccctc 180

aaggccaccc ccaagctctc ccacctcaag tcccccgcca accccgacgc cgtccgctcc 240

ttcctcgccg gcctcggcct ctccggcgcc gacgtcgcgg ccctcgtcgc cagggacccg 300

ctcttcctct gcgccggcgt ggacggaaac ctgggccccg ccgtcgccgg gctcaccgac 360

ctcggcctct cgcgggccga ggtcgcgcgc ctcgtctcgc tctccccgga ccgattccgc 420

cgcaagagcg tcgtccccaa ggtgcggtac tacctgcctc tcttcggctc ccccgcggac 480

ctcctctcgg gggtcaagac cggcctattc cttctctccg tcgacctcga ccgggtcgtc 540

aagcccaatg tcgccgtcct gcgcaagtgc gggctaggtg tttgtgatat tgccaagctg 600

ctcatccaaa tgccgaggat cgtcaccgcc agccccgagc gcaccctcgc gatggtcgcg 660

tgcgccgagc gcttgggtgt gccccgtggc tccgggatgt ttaggcaggc gctgcaggcc 720

gtcgcatgcc tcagcgagga caagattgcc gccaaagtgg agcagttgaa gaagacactg 780

aggtggtcgg atgccgatgt cggcattgct gtccgcaagt ggccgactgt gctgaggtgg 840

tcaaggacat gctgcagcg caagtccgag ttcctcttct ctgaggtggg cttggaaccg 900

gcgtacgttg ctcaccgtcc ggcaatgctc ggtcttagct tggagcgccg gctcaagccc 960

aggtactatg ttatgaggtt tcttaaggaa aatggattgc tcagtcatgc cagagactac 1020

tattgtatgg tcttggtcag cgagaaggta tttgtggagc ggttcatacg cccccacaag 1080

caagctgtgc cacgcattgc tgaagactat gcagccgctt gcatagggga ggtgcctgct 1140

agattcagat ttacatga 1158

<210> 33
<211> 385
<212> PRT
<213> Secale cereale

<400> 33

Met Leu Arg Leu Arg Ser Cys Leu Val Thr His Leu Leu Ser Ser Pro
1               5                   10                  15

Thr Thr Ser Pro Leu Pro Ser Leu His Arg Leu Leu Ser Ala Ala Ala
            20                  25                  30

Ala Pro Ala Val Ser Pro Ser Ser Gly Phe Asp Val Asp Asp Tyr Leu
        35                  40                  45

Val Ser Thr Cys Gly Leu Thr Arg Ala Gln Ala Leu Lys Ala Thr Pro
    50                  55                  60

Lys Leu Ser His Leu Lys Ser Pro Ala Asn Pro Asp Ala Val Arg Ser
65                  70                  75                  80

Phe Leu Ala Gly Leu Gly Leu Ser Gly Ala Asp Val Ala Ala Leu Val
                85                  90                  95

Ala Arg Asp Pro Leu Phe Leu Cys Ala Gly Val Asp Gly Asn Leu Gly
            100                 105                 110

Pro Ala Val Ala Gly Leu Thr Asp Leu Gly Leu Ser Arg Ala Glu Val
        115                 120                 125

Ala Arg Leu Val Ser Leu Ser Pro Asp Arg Phe Arg Arg Lys Ser Val
    130                 135                 140

Val Pro Lys Val Arg Tyr Tyr Leu Pro Leu Phe Gly Ser Pro Ala Asp
145                 150                 155                 160

Leu Leu Ser Gly Val Lys Thr Gly Leu Phe Leu Leu Ser Val Asp Leu
                165                 170                 175

Asp Arg Val Val Lys Pro Asn Val Ala Val Leu Arg Lys Cys Gly Leu
            180                 185                 190

Gly Val Cys Asp Ile Ala Lys Leu Leu Ile Gln Met Pro Arg Ile Val
        195                 200                 205

Thr Ala Ser Pro Glu Arg Thr Leu Ala Met Val Ala Cys Ala Glu Arg
    210                 215                 220

Leu Gly Val Pro Arg Gly Ser Gly Met Phe Arg Gln Ala Leu Gln Ala
225                 230                 235                 240

Val Ala Cys Leu Ser Glu Asp Lys Ile Ala Ala Lys Val Glu Gln Leu
                245                 250                 255

Lys Lys Thr Leu Arg Trp Ser Asp Ala Asp Val Gly Ile Ala Val Arg
            260                 265                 270

Lys Trp Pro Thr Val Leu Arg Trp Ser Arg Asp Met Leu Gln Arg Lys
        275                 280                 285

Ser Glu Phe Leu Phe Ser Glu Val Gly Leu Glu Pro Ala Tyr Val Ala
    290                 295                 300

His Arg Pro Ala Met Leu Gly Leu Ser Leu Glu Arg Arg Leu Lys Pro
305                 310                 315                 320

Arg Tyr Tyr Val Met Arg Phe Leu Lys Glu Asn Gly Leu Leu Ser His
                325                 330                 335

Ala Arg Asp Tyr Tyr Cys Met Val Leu Val Ser Glu Lys Val Phe Val
            340                 345                 350

Glu Arg Phe Ile Arg Pro His Lys Gln Ala Val Pro Arg Ile Ala Glu
        355                 360                 365

Asp Tyr Ala Ala Ala Cys Ile Gly Glu Val Pro Ala Arg Phe Arg Phe
    370                 375                 380

Thr
385

## Beschreibung

### GEBIET DER ERFINDUNG

Die vorliegende Erfindung betrifft das technische Gebiet der Pflanzenzüchtung und der grünen Biotechnologie, insbesondere das Gebiet der Herstellung von Hybridpflanzen mittels molekularbiologischer Methoden, Markertechnologie und der Gentechnik. Insbesondere werden Hybridgetreide zur Verfügung gestellt, die durch Restauration der Pollenfertilität für die cytoplasmatische männliche Sterilität (P-CMS), die durch das Pampa Cytoplasma erzeugt wird, erhalten werden und/oder die eine vollständigeRestauration der Pollenfertilität für die cytoplasmatische männliche Sterilität (P-CMS), die durch das Pampa Cytoplasma erzeugt wird, aufweisen. Sie zeichnen sich dadurch aus, dass negative, meist ertragsmindernde Effekte unterbleiben, die ansonsten mit der Introgression von chromosomalen Segmenten, die den für die Restauration verantwortlichen Locus enthalten, in Kultivare verbunden sind. Diesbezüglich stellt die vorliegende Erfindung Pflanzen zur Verfügung, insbesondere Roggenpflanzen, die als männlicher Pollenelter in der Lage sind, die Pollenfertilität für die P-CMS zu restaurieren, wobei in Hybridpflanzen aus Kreuzung dieser Pollenelter mit einem weiblichen CMS Elter ein ansonsten mit der Restaurationseigenschaft gekoppelter Linkage Drag reduziert ist oder vollständig unterbleibt.

Des Weiteren betrifft die vorliegende Erfindung Nukleinsäuremoleküle, die die notwendigen Informationen zur Restauration der P-CMS trägt, DNA und Vektoren, die ein solches Nukleinsäuremolekül enthalten, entsprechende Wirtszellen sowie ein durch das Nukleinsäuremolekül kodierbares Protein und dagegen gerichtete Antikörper. Die Erfindung betrifft ferner die Verwendung der Nukleinsäuremoleküle, DNA, Vektoren und Antikörper beispielsweise in der Herstellung von Hybridpflanzen.

### HINTERGRUND DER ERFINDUNG

Roggen zeigt dank seiner ausgeprägten Stresstoleranz auf nährstoffarmen, trockenen Standorten sowie in Wassereinzugsgebieten bei eingeschränktem Pestizideinsatz gegenüber der Gerste und dem Weizen erhebliche Ertragsvorteile und entspricht damit spezifischen Aspekten einer nachhaltigen Landwirtschaft. Die Nutzung der cytoplasmatisch-männlichen Sterilität (CMS) hat u.a. im Roggen die Möglichkeit eröffnet, durch Nutzung der Heterosis Hybridsorten mit hohem Ertragspotenzial zu züchten (Geiger, H. H., and T. Miedaner. "Hybrid rye and heterosis." Genetics and Exploitation of Heterosis in Crops. Crop Science Society. America, Madison, Wisconsin, USA (1999): 439-450). Hybriden im Roggen zeigen eine stetig wachsende Bedeutung als landwirtschaftliche Kulturart in Europa. Allein in Deutschland, Dänemark oder Österreich macht Hybridroggen bereits mehr als 70% der Gesamtproduktion an Roggen aus. Auch in anderen Regionen, insbesondere in Osteuropa, wird in den kommenden Jahren mit einer deutlichen Zunahme des Anbaus von Hybridroggen gerechnet. Die Hauptverwendungen von Roggen liegen in der Tierernährung und der Brotherstellung, für die Roggen in der Regel als Mischung mit anderen Getreiden eingesetzt wird. Ferner gewinnt Roggen zunehmend an Bedeutung auch als Substrat zur Bioenergiegewinnung.

Derzeit basieren die meisten Hybridsysteme in Roggen auf der Nutzung des Pampa (P) Zytoplasmas, das gemeinsam mit Nichtrestorergenen im Kerngenom männliche Sterilität (P-CMS), vermittelt. Dieses wurde Ende der 1960iger Jahre in einer argentinischen Landrasse entdeckt (Geiger, H. H., and F. W. Schnell. "Cytoplasmic male sterility in rye (Secale cereale L.)." Crop Science 10.5 (1970): 590-593). Diese CMS zeigt eine exzellente Stabilität gegenüber Umweltbedingungen und ist von in allen europäischen Zuchtungspopulationen vorkommenden Nichtrestorer-Genotypen zuverlässig aufrechtzuerhalten. Auf der Suche nach effizienten Restorern für die männliche Fertilität von P-CMS wurde man in primitiven Roggenakzessionen wie IRAN IX, Pico Gentario oder Altevogt 14160 fündig (Geiger HH, Miedaner T (1996) Genetic basis and phenotypic stability of male-fertility restoration in rye. Vortr Pflanzenzüchtg 35:27-38; Miedaner T, Glass C, Dreyer F, Wilde P, Wortmann H, Geiger HH (2000) Mapping of genes for male fertility restoration in "Pampa" CMS winter rye (Secale cereale L.). Theor Appl Genet 101:1226-1233; Falke KC, Wilde P, Miedaner T (2009) Rye introgression lines as source of alleles for pollen-fertility restoration in Pampa CMS. Plant Breeding 128:528-531). IRAN IX ist eine selbst-inkompatible Roggenpopulation, die in der Elburz-Karaj Region von Kuckuck (1956; Report to the government of Iran on the distribution and variation of cereals in Iran. FAO Report No. 517:1-22) gesammelt und in der Genbank der ehemaligen Bundesforschungsanstalt für Landwirtschaft (FAL) eingelagert wurde. Die Pico Gentario Akzession stammt aus Argentinien und die Altevogt 14160-Population auch aus dem Iran. Beide sind ebenfalls selbst-inkompatibel und können vom Botanischen Garten der Polnischen Akademie der Wissenschaften in Warschau erhalten werden. Verglichen mit Restorergenotypen, die aus zentraleuropäischen Quellen stammen, zeigen die Restorer aus IRAN IX, Pico Gentario oder Altevogt 14160 eine hohe und stabile Restaurationsleistung. Diese manifestiert sich im Gegensatz zu derjenigen von zentraleuropäischen Quellen in Form einer sehr guten Pollenschüttung, einer Eigenschaft also, welche bei der Minimierung von Mutterkorn eine entscheidende Rolle spielt. Der Befall mit Mutterkorn zählt zu den wirtschaftlich bedeutendsten Krankheiten des Roggens (*Claviceps purpurea* [*Fr*.] *Tul.*). Seit 2008 wird demzufolge die Anfälligkeit der Roggensorten für Mutterkorn in der beschreibenden Sortenliste des deutschen Bundessortenamtes offiziell eingestuft und auch von dem polnischen Sortenamt (COBORU) bei der Bewertung von Roggenhybriden berücksichtigt. Die Verbesserung des Pollenschüttungsvermögens in Hybridsorten bei z.B. Winterroggen durch effektive Restorerloci wie *Rfp1* ist gegenwärtig die wirksamste und nachhaltigste Strategie, die Kontamination des Erntegutes mit Mutterkorn in Hybridroggen zu minimieren. Insgesamt stellte somit die Introgression dieser Restorerquellen in Pollenelterlinien einen bedeutenden Fortschritt für die Fertilitätsrestauration von Hybriden dar.

Kartierungsarbeiten zur Lokalisation des Restorerlocus *Rfp1* des Donors IRAN IX, *Rfp2* des Donors Pico Gentario bzw. des Locus aus Altevogt 14160 ergaben jeweils eine Position auf dem langen Arm des Chromosom 4R (Miedaner et al. 2000. Mapping of genes for male fertility restoration in "Pampa" CMS winter rye (Secale cereale L.). Theor Appl Genet 101:1226-1233; Stracke et al. 2003. Development of PCR-based markers linked to dominant genes for male-fertility restoration in Pampa CMS of rye (Secale cereale L.), Theor Appl Genet (2003) 106:1184-1190; Falke et al. 2009. Rye introgression lines as source of alleles for pollen-fertility restoration in Pampa CMS. Plant Breeding 128:528-531, Hackauf et al. 2012. Development of COS Markers for the Restorer Gene Rfp1 in Rye. Molecular Breeding 30: 1507-1518). Studien mit assoziierten Selektionsmarkern belegen, dass in der betreffenden Region auf Chromosom 4 RL möglichweise entweder mehrere Restorergene geclustert sind oder es sich bei den betreffenden Restorergenen um Allele ein und desselben Genortes handelt könnte (Hackauf et al. (2012) "Development of conserved ortholog set markers linked to the restorer gene Rfp1 in rye." Molecular breeding 30.3: 1507-1518).

Auch wenn die Verwendung der vorstehenden Restorerloci einerseits im Hinblick auf die Restaurationsleistung und die Pollenschüttung vorteilhaft ist, so vermindern andererseits die zugehörigen, die Restorerloci enthaltenden Introgressionssegmente die agronomische Leistung heutiger Zuchtpopulationen. Insbesondere der Kornertrag wird deutlich von dem die Restorergene flankierenden Genombereich (Linkage Drag) negativ beeinflusst, sodass der Vorteil des Heterosis-Effekts in den Hybriden drastisch geschmälert oder sogar gänzlich zunichte gemacht wird. Weiterhin wurde auch die Möglichkeit diskutiert, dass der beobachtete Linkage Drag-Effekt oder zumindest ein Teil davon tatsächlich ein pleiotroper Effekt des Restorergens ist. Trotz intensiver Rückkreuzungsarbeiten begleitet von einer ausgedehnten Markerentwicklung über nunmehr mehr als zehn Jahren ist es bis heute lediglich gelungen eine grobe genetische Position festzulegen. Durch die durchgängige Selektion auf mehr oder weniger engekoppelter Foregroundmarker und das Zielgen bleibt die Größe des Introgressionsfragments mit dem Restorerlocus weithin erhalten, und somit auch eine große Anzahl nicht-angepasster Donor-Gene, welche den beobachteten Linkage Drag-Effekt unmittelbar plausibel machen. Hackauf et al., (2012) ("Development of conserved ortholog set markers linked to the restorer gene Rfpl in rye." Molecular breeding 30.3 (2012): 1507-1518) zeigen für *Rfp1* den aktuellsten veröffentlichten Stand der Kartierung der interessierenden Genomregion um den Restorerlocus auf 4R. Zwar gelang es über einen vergleichenden Ansatz der Genkartierung auf Basis vollständig entschlüsselter Gräsergenome das Introgressionssegment samt dem Rfp1-Locus selbst auf ein Intervall von ca. 2,0 cM, flankiert durch die Marker tc135788 und tc176835, bzw. auf ein Intervall von 0,7 cM, flankiert durch die Marker tc256739 und tc300731, zu beschränken. Jedoch konnte weder das Restorergen selbst identifiziert werden, noch gab es bislang fundierte experimentelle Ergebnisse zu Ausmaß und Lokalisation der Minderung der agronomischen Leistung. Auch ist nicht bekannt, dass Rekombinanten hergestellt wurden, bei denen die Veränderung des Introgressionssegmentes und der agronomischen Leistung miteinander in Beziehung gesetzt werden konnten

Die Aufgabe der vorliegenden Erfindung war daher, die den genannten Restorerloci zugrunde liegenden Introgressionssegmente weiterzuentwickeln, sodass diese zwar die gewünschte Restaurationseigenschaft in Getreide beibehalten, jedoch die Leistungsminderungen nicht mehr zeigen bzw. diese deutlich reduzieren oder minimieren. Insbesondere war es die Aufgabe der Erfindung, die Restorergene, die eine wesentliche Grundlage von Hybridzüchtungsprogrammen in Gräsern darstellen, vorzugsweise in Getreide, in eine hochauflösende Feinkartierung der betreffenden Region einzubetten und somit bereitszustellen. Weiterhin sollten im Rahmen der Erfindung Genotypen zur Verfügung gestellt werden, die anhand von eng-gekoppelten Markern um den Restorerlocus herum Haplotypen für die Zielregion beschreiben, denen die agronomische Leistungsänderung quantitativ und qualitativ präzise zugeordnet werden kann. Ferner ist es Aufgabe der vorliegenden Erfindung Marker zu identifizieren, die im Restorergen selbst angesiedelt sind, so dass mit ihnen das Restorergen für züchterische Zwecke bereitgestellt ist.

### BESCHREIBUNG DER ERFINDUNG

Die vorstehende Aufgabe wird durch die Bereitstellung einer Pflanze, insbesondere aus der Ordnung der Grasartigen (*Poales*), die geeignet ist, als männlicher Pollenelter die Pollenfertilität für die Pampa cytoplasmatische männliche Sterilität (P-CMS) zu restaurieren, gelöst. Bevorzugt handelt es sich um eine Pflanze aus der Familie der Süßgräser (*Poaceae*) oder der Gattung *Secale* oder *Hordeum* und besonders bevorzugt um eine Pflanze der Spezies *Secale cereale* oder *Hordeum vulgare.* Die Pflanze ist weiterhin dadurch gekennzeichnet, dass in der Pflanze oder in einer Hybridpflanze erhältlich aus einer Kreuzung der Pflanze mit einem weiblichen CMS Elter derselben Spezies, ein ansonsten mit der Restaurationseigenschaft gekoppelter Linkage Drag-Effekt (siehe Hackauf et al. 2012), vorzugsweise ein ertragsmindernder Effekt, reduziert ist oder vollständig unterbleibt. Mit Hilfe der vorliegenden Erfindung konnte somit eine Pflanze bereitgestellt werden, bei welcher im Restorerlocus negative, unerwünschte agronomische Eigenschaften von den Restorergenen *Rfp1a* und *Rfp1b* entkoppelt werden konnten. Diese Entkopplung erlaubt es, einen hohen Ertrag mit einer effizienten Restaurationsleistung zu verknüpfen.

In einer besonderen Ausführungsform weisen Zellen der Pflanze ein die Pampa cytoplasmatische männliche Sterilität (CMS) vermittelndes Zytoplasma auf. Somit stellt die vorliegende Erfindung auch eine Hybridpflanze mit hohem Ertragspotenzial bereit, welche über eine sehr effiziente Restaurationsleistung verfügt, oder eine Pflanze, die als Pollenelter geeignet ist, die Pollenfertilität für die Pampa cytoplasmatische männliche Sterilität (CMS), vorzugsweise vollständig, zu restaurieren. Gleichzeitig ist der Linkage Drag, der eine deutliche Minderung der agronomischen Leistung, insbesondere eine Ertragsminderung, bewirken kann, reduziert oder vollständig aufgehoben.

In einer bevorzugten Ausführungsform handelt es sich um eine Pflanze, die ein chromosomales Segment umfasst, das mindestens ein Nukleinsäuremolekül aufweist, welches in der Lage ist, die Restaurationseigenschaft für die Pampa cytoplasmatische männliche Sterilität zu vermitteln. Bevorzugt ist das chromosomale Segment ein Intervall zwischen den Markerloci tc256739, ctg32 oder ctg24met2a5 und tc300731 oder 7_01_H_1441 auf dem Chromosom 4R von einem Donor ausgewählt aus der Gruppe bestehend aus IRAN IX, Pico Gentario und Altevogt 14160. Ferner kann das beschriebene chromosomale Segmente auch in anderen verwandten Donoren gefunden werden. Solche Donoren sind insbesondere im mediterranen Regionen beispielsweise der Türkei oder Spaniens in Kenntnis der hier beschriebenen genetischen Struktur des chromosomalen Segment wie auch des mindestens einen Nukleinsäuremolekül auffindbar. Hierbei können beispielsweise erfindungsgemäße molekulare Marker wie weiter unten beschrieben eingesetzt werden. Daher ist die vorliegende Erfindung auch nicht auf die Donoren aus der Gruppe bestehend aus IRAN IX, Pico Gentario und Altevogt 14160 beschränkt, sondern schließt, auch wenn nicht explizit benannt, auch immer verwandte Donoren auf, welche als Quelle des erfindungsgemäßen chromosomalen Segments und des erfindungsgemäßen mindestens einen Nukleinsäuremolekül dienen können.

Ein solches chromomales Segment kann beispielsweise eines der folgenden Intervalle sein: zwischen den Markerloci tc256739 und tc300731, zwischen den Markerloci ctg32 und tc300731, zwischen den Markerloci ctg24met2a5 und tc300731, zwischen den Markerloci ctg2 und tc300731, zwischen den Markerloci ctg16b und tc300731, zwischen den Markerloci c40745_1 und tc300731, zwischen den Markerloci P20 und tc300731, zwischen den Markerloci tc256739 und 7_01_H_1441, zwischen den Markerloci ctg32 und 7_01_H_1441, zwischen den Markerloci ctg24met2a5 und 7_01_H_1441, zwischen den Markerloci ctg2 und 7_01_H_1441, zwischen den Markerloci ctg16b und 7_01_H_1441, zwischen den Markerloci c40745_1 und 7_01_H_1441, zwischen den Markerloci P20 und 7_01_H_1441, zwischen den Markerloci tc256739 und 72F13_c2_mTERF, zwischen den Markerloci tc256739 und P20, zwischen den Markerloci tc256739 und c40745_1, zwischen den Markerloci tc256739 und ctg16b, zwischen den Markerloci ctg32 und 72F13_c2_mTERF, zwischen den Markerloci ctg32 und P20, zwischen den Markerloci ctg32 und c40745_1, zwischen den Markerloci ctg32 und ctg16b, zwischen den Markerloci ctg24met2a5 und 72F13_c2_mTERF, zwischen den Markerloci ctg24met2a5 und P20, zwischen den Markerloci ctg24met2a5 und c40745_1, zwischen den Markerloci ctg24met2a5 und ctg16b, zwischen den Markerloci ctg2 und 72F13_c2_mTERF, zwischen den Markerloci ctg2 und P20, zwischen den Markerloci ctg2 und c40745_1 oder zwischen den Markerloci ctg2 und ctg16b. Ferner ist der Linkage Drag-Effekt vorzugsweise der derjenige Linkage Drag-Effekt, der mit dem chromosomalen Segment, aus dem das restaurierende Nukleinsäuremolekül stammt, ursprünglich gekoppelt war. Weiterhin ist das Nukleinsäuremolekül vorzugsweise ein Nukleinsäuremolekül, das eine Nukleotidsequenz aufweist, die mindestens einen mitochondrialen Transkriptionsterminationsfaktor (mTERF), ein Homolog, ein Analog, ein Ortholog oder ein funktionelles Fragment davon kodiert. Mindestens ein Nukleinsäuremolekül kann ein, zwei, drei, vier oder fünf Nukleinsäuremoleküle bedeuten; vorzugsweise meint mindestens ein Nukleinsäuremolekül ein oder zwei Nukleinsäuremoleküle.

Als Quelle für das chromosomale Segment, das mindestens ein Nukleinsäuremolekül aufweist, welches in der Lage ist, die Restaurationseigenschaft für die Pampa cytoplasmatische männliche Sterilität zu vermitteln, können die primitiven Roggen-Akzessionen IRAN IX, Pico Gentario und Altevogt 14160 dienen (Geiger et al., Vortr Pflanzenzüchtg 35 (1996), 27-38; Miedaner et al., Theor Appl Genet 101(2000), 1226-1233; Falke et al., Plant Breeding 128 (2009), 528-531). IRAN IX ist eine selbstinkompatible Roggenpopulation aus Elburz-Karaj, gesammelt von Kuckuck (FAO Report No. 517 (1956), 1-22) und gehalten in der Genbank der Bundesforschungsanstalt für Landwirtschaft (FAL). Die Pico Gentario Akzession aus Argentinien und die Altevogt 14160 Population aus Iran sind ebenfalls selbstinkompatibel und wurden beide von dem Botanischen Garten der "Polish Academy of Sciences" in Warschau, Polen bereitgestellt.

In einer besonders bevorzugten Ausführungsform weist das mindestens eine Nukleinsäuremolekül eine Nukleotidsequenz auf, welche ausgewählt ist aus der Gruppe bestehend aus einer: (i) Nukleotidsequenz gemäß einer der SEQ ID NO: 1 oder SEQ ID NO: 28 oder einem funktionellen Fragment davon, (ii) Nukleotidsequenz, die eine Aminosäuresequenz gemäß einer der SEQ ID NO: 2 oder SEQ ID NO: 29 oder einem funktionellen Fragment davon kodiert, (iii) Nukleotidsequenz, die komplementär ist zu einer Nukleotidsequenz nach (i) oder (ii), (iv) Nukleotidsequenz, welche an eine Sequenz nach (iii) unter stringenten Bedingungen hybridisiert, (v) Nukleotidsequenz, die eine Identität von mindestens 70%, 75%, 80%, 85% oder 90%, bevorzugt von mindestens 91%, 92%, 93% 94% oder 95%, oder besonders bevorzugt von mindestens 96%, 97%, 98%, 99% oder 99,5% zu der Nukleotidsequenz nach (i) oder (ii) aufweist, (vi) Nukleotidsequenz, die eine Aminosäuresequenz, die eine Identität von mindestens 65%, 70%, 75%, 80%, 85% oder 90%, bevorzugt von mindestens 91%, 92%, 93% 94% oder 95%, oder besonders bevorzugt von mindestens 96%, 97%, 98%, 99% oder 99,5% zu einer der SEQ ID NO: 2 oder SEQ ID NO: 29 oder einem funktionellen Fragment davon aufweist, kodiert, (vii) Nukleotidsequenz, die eine Aminosäuresequenz kodiert, die gegenüber der in SEQ ID NO: 2 oder SEQ ID NO: 29 gezeigten Aminosäuresequenz Abweichungen in Form von Aminosäure-Deletionen, Substitutionen, Additionen und/oder Insertionen in der Aminosäuresequenz aufweist, vorzugsweise nicht mehr als 30%, 25% oder 20%, bevorzugt nicht mehr als 18%, 16%, 14%, 12% oder 10% oder besonders bevorzugt nicht mehr als 9%, 8%, 7%, 6%, 5%, 4%, 3%, 2%, 1% oder 0,5% über die gesamte Aminosäuresequenz. Vorzugsweise kodiert das mindestens eine Nukleinsäuremolekül für einen oder mehrere mitochondriale Transkriptionsterminationsfaktoren (mTERF) oder ein funktionelles Fragment davon. Die mTERF Protein-Familie teilt mehrere wichtige Funktionen mit der sogenannten Pentatricopeptide (PPR)-Familie. Wie die mTERF Protein-Familie ist auch die Pentatricopeptide (PPR)-Familie eine ungewöhnliche Familie von RNA-Bindeproteinen, welche durch degenerierte Helices-Wiederholungen gekennzeichnet ist. Bei der PPR-Familie bestehen diese aus ca. 35 Aminosäuren (Small et al., Trends Biochem. Sci. 25 (2000), 46-47), im Gegensatz zu den mTERF Wiederholungen, welche meist durch ca. 31 Aminosäuren gekennzeichnet sind, die drei anstelle von zwei Helices bilden (Hammani et al., Nucleic Acids Res 42 (2014), 5033-5042). Es kann nicht ausgeschlossen werden, dass das Vorhandensein von einem oder mehreren funktionellen PPR-Genen sich störend auf den positiven Effekt der oben beschriebenen Pflanzen mit verbesserten Eigenschaften auswirkt. Daher weist das Nukleinsäuremolekül der Pflanze in einer Ausführungsform bevorzugt kein funktionelles Pentatricopeptid (PPR)-Gen auf, welches vom Donor stammt.

Wie oben unter (vii) beschrieben kann das mindestens eine Nukleinsäuremolekül eine Aminosäuresequenz kodieren, die gegenüber der Aminosäuresequenz der SEQ ID NO: 2 oder der SEQ ID NO: 29 eine Aminosäuresequenz Abweichungen in Form von Aminosäure-Substitutionen, Deletionen, Insertionen und/oder Additionen enthält. Bevorzugt ist ein solches Nukleinsäuremolekül in der Lage, unter stringenten Bedingungen an eine komplementäre Sequenz zu der SEQ ID NO: 1 oder 2 bzw. zu der SEQ ID NO: 28 oder 29 zu binden.

Wie in den Beispielen weiter unten gezeigt, konnten eng-flankierende Marker der *Rfp1*-Gene identifiziert werden und somit eng-gekoppelte Marker zur hochauflösenden Kartierung der *Rfp1*-Gene in Roggen bereitgestellt werden, die es u.a. ermöglichten, das Restorergen zu flankieren und die *Rfp1-*Zielregion in Getreidegenomen aufzuklären. Damit wird erstmals eine Marker-gestützte Übertragung des Zielgenes in neues Zuchtmaterial möglich, dergestalt dass eine effiziente Selektion gegen den unerwünschten genetischen Hintergrund des Donorgenoms mit eingeschlossen ist.

In einer bevorzugten Ausführungsform weist das chromosomale Segment der Pflanze einen oder mehrere der folgenden Markerloci des Donors auf: ctg2 (Amplifikationsprodukt der Primer mit SEQ ID NOs: 4 und 5), P20 (Amplifikationsprodukt der Primer mit SEQ ID NOs: 6 und 7), 72F13_c2_mTERF (Amplifikationsprodukt der Primer mit SEQ ID NOs: 8 und 9) oder ctg16b (Amplifikationsprodukt der Primer mit SEQ ID NOs: 10 und 11). Die Restaurationseigenschaft der Pflanze kann auch durch Abwesenheit eines oder mehrerer der folgenden Markerloci des Donors im chromosomalen Segment gekennzeichnet sein: 7_01_H_1441 (Amplifikationsprodukt der Primer mit SEQ ID NOs: 12 und 13), ctg24met2a5 (Amplifikationsprodukt der Primer mit SEQ ID Nr.: 14 und 15), oder ctg32 (Amplifikationsprodukt der Primer mit SEQ ID Nr.: 16 und 17).

In einer besonders bevorzugten Ausführungsform weist das chromosomale Segment der Pflanze die Markerloci des Donors ctg32, ctg24met2a5, ctg2, ctg16b und c40745_1 (Amplifikationsprodukt der Primer mit SEQ ID NOs: 18 und 19) auf und die Markerloci des Donors tc256739 (Amplifikationsprodukt der Primer mit SEQ ID NOs: 21 und 22), 72F13_c2_mTERF, P20, 7_01_H_1441 und tc300731 (Amplifikationsprodukt der Primer mit SEQ ID NOs: 23 und 24)sind auf dem chromosomalen Segment abwesend.

In einer weiteren besonders bevorzugten Ausführungsform weist das chromosomale Segment der Pflanze die Markerloci des Donors ctg32, ctg24met2a5, ctg2 und ctg16b auf und die Markerloci des Donors tc256739, c40745_1, 72F13_c2_mTERF, P20, 7_01_H_1441 und tc300731 sind auf dem chromosomalen Segment abwesend.

In einer weiteren besonders bevorzugten Ausführungsform weist das chromosomale Segment der Pflanze die Markerloci des Donors ctg32, ctg24met2a5 und ctg2 auf und die Markerloci des Donors tc256739, ctgl6b, c40745_1, 72F13_c2_mTERF, P20, 7_01_H_1441 und tc300731 sind auf dem chromosomalen Segment abwesend.

In einer weiteren besonders bevorzugten Ausführungsform weist das chromosomale Segment der Pflanze die Markerloci des Donors 72F13_c2_mTERF, P20 und 7_01_H_1441 auf und die Markerloci des Donors tc256739, ctg32, ctg24met2a5, ctg2, ctg16b, c40745_1 und tc300731 sind auf dem chromosomalen Segment abwesend.

In einer weiteren besonders bevorzugten Ausführungsform weist das chromosomale Segment der Pflanze die Markerloci des Donors 72F13_c2_mTERF und P20 auf und die Markerloci des Donors tc256739, ctg32, ctg24met2a5, ctg2, ctg16b, c40745_1, 7_01_H_1441 und tc300731 sind auf dem chromosomalen Segment abwesend.

In einer weiteren besonders bevorzugten Ausführungsform weist das chromosomale Segment der Pflanze die Markerloci des Donors c40745_1, 72F13_c2_mTERF, P20 und 7_01_H_1441 auf und die Markerloci des Donors tc256739, ctg32, ctg24met2a5, ctg2, ctg16b und tc300731 sind auf dem chromosomalen Segment abwesend.

In einer weiteren besonders bevorzugten Ausführungsform weist das chromosomale Segment der Pflanze die Markerloci des Donors c40745_1, 72F13_c2_mTERF und P20 auf und die Markerloci des Donors tc256739, ctg32, ctg24met2a5, ctg2, ctg16b, 7_01_H_1441 und tc300731 sind auf dem chromosomalen Segment abwesend.

In einer weiteren besonders bevorzugten Ausführungsform weist das chromosomale Segment der Pflanze die Markerloci des Donors ctg16b, c40745_1, 72F13_c2_mTERF, P20 und 7_01_H_1441 auf und die Markerloci des Donors tc256739, ctg32, ctg24met2a5, ctg2 und tc300731 sind auf dem chromosomalen Segment abwesend.

In einer weiteren besonders bevorzugten Ausführungsform weist das chromosomale Segment der Pflanze die Markerloci des Donors ctg16b, c40745_1, 72F13_c2_mTERF und P20 auf und die Markerloci des Donors tc256739, ctg32, ctg24met2a5, ctg2, 7_01_H_1441 und tc300731 sind auf dem chromosomalen Segment abwesend.

In einer weiteren besonders bevorzugten Ausführungsform weist das chromosomale Segment der Pflanze die Markerloci des Donors ctg2, ctg16b, c40745_1, 72F13_c2_mTERF, P20 und 7_01_H_1441 auf und die Markerloci des Donors tc256739, ctg32, ctg24met2a5 und tc300731 sind auf dem chromosomalen Segment abwesend.

In einer weiteren besonders bevorzugten Ausführungsform weist das chromosomale Segment der Pflanze die Markerloci des Donors ctg2, ctg16b, c40745_1, 72F13_c2_mTERF und P20 auf und die Markerloci des Donors tc256739, ctg32, ctg24met2a5, 7_01_H_1441 und tc300731 sind auf dem chromosomalen Segment abwesend.

In einer weiteren besonders bevorzugten Ausführungsform weist das chromosomale Segment der Pflanze die Markerloci des Donors ctg24met2a5, ctg2, ctg16b, c40745_1, 72F13_c2_mTERF, P20 und 7_01_H_1441 auf und die Markerloci des Donors tc256739, ctg32 und tc300731 sind auf dem chromosomalen Segment abwesend.

In einer weiteren besonders bevorzugten Ausführungsform weist das chromosomale Segment der Pflanze die Markerloci des Donors ctg24met2a5, ctg2, ctg16b, c40745_1, 72F13_c2_mTERF und P20 auf und die Markerloci des Donors tc256739, ctg32, 7_01_H_1441 und tc300731 sind auf dem chromosomalen Segment abwesend.

In einer bevorzugten Ausführungsform ist das chromosomale Segment nicht größer als 190 kb, nicht größer als 150 kb oder nicht größer als 100 kb, bevorzugt nicht größer als 75 kb oder nicht größer als 50 kb, besonders bevorzugt nicht größer als 40 kb, nicht größer als 30 kb, nicht größer als 25 kb oder nicht größer als 20 kb. In einer besonders bevorzugten Ausführungsform umfasst das chromosomale Segment einen DNA-Abschnitt von 18,425 kb, welcher vorzugsweise eine Nukleotidsequenz gemäß SEQ ID NO: 20 aufweist oder eine Nukleotidsequenz, die eine Identität von mindestens 85% oder 90%, bevorzugt von mindestens 91%, 92%, 93% 94% oder 95%, oder besonders bevorzugt von mindestens 96%, 97%, 98%, 99% oder 99,5% zu der Nukleotidsequenz gemäß SEQ ID NO: 20 aufweist.

In einer weiteren Ausführungsform weist das Nukleinsäuremolekül kein funktionelles Pentatricopeptid (PPR)-Gen auf, welches vom Donor stammt.

Die Pflanze der vorliegenden Erfindung ist vorzugsweise eine Inzuchtpflanze, eine doppelhaploide Pflanze oder eine Hybridpflanze und/oder bevorzugt homozygot oder heterozygot für die Restaurationseigenschaft, für das chromosomale Segment oder das mindestens eine Nukleinsäuremolekül. Die Hybridpflanze kann sein ein 'single-cross' Hybrid, ein 'double-cross' Hybrid, ein 'topcross' Hybrid, ein 'three-way-cross' Hybrid, ein 'triple-cross' Hybrid, ein 'composite'-Hybrid, ein 'blended' Hybrid, ein voll restaurierter Hybrid, ein ,second generation' Hybrid oder ein anderer Hybrid. Vorzugsweise wirkt eine Pflanze der vorliegenden Erfindung als Pollenspender in einer Hybride-erzeugenden Kreuzung und/oder in der Befruchtung von Körnern bzw. Samen auf einer hybriden Pflanze.

Die in dieser Arbeit dargestellte Identifizierung der Restorergene sowie der am Linkage Drag verantwortlichen Faktoren wurde im Allgemeinen in Gräsern durchgeführt. Vorzugsweise wurden die hier dargestellten Ergebnisse jedoch in Getreide gewonnen, wobei die Pflanzen hauptsächlich der Gattungen Roggen (*Secale*), Gerste (*Hordeum*) oder einer aus Roggen (erster Partner) und Weizen (zweiter Partner) gezüchteten Getreideart, der sogenannten *Triticale* angehörten. Der negative Effekt des Linkage Drag eng-gekoppelt an den Restorerlocus *Rfp1* spielt insbesondere in der Hybridzüchtung von Getreide, wie z.B. Roggen, eine entscheidende Rolle und führt im Roggen bekanntermaßen zu erheblicher Ertragsminderung. Vergleichbare Schwierigkeiten sind auch aus der Hybridzüchtung von Gerste bekannt. Aufgrund der genetischen Ähnlichkeiten im chromosomalen Bereich des Restorerlocus zwischen Roggen und Gerste wie auch *Triticale,* ist in einer Ausführungsform der vorliegenden Erfindung die Pflanze eine Pflanze der Gattung *Secale, Hordeum* oder *Triticale,* vorzugsweise eine Pflanze der Art *Secale cereale* oder *Hordeum vulgare.*

Die Erfindung umfasst neben den Pflanzen mit exzellenter Restaurationseigenschaft und ohne Linkage Drag bzw. mit vermindertem Linkage Drag auch Samen oder Nachkommen dieser Pflanzen, wobei diese das definierte chromosomale Segment oder das mindestens eine definierte Nukleinsäuremolekül für die Restaurationseigenschaft umfassen. Nachkommen zeigen ebenfalls die verbesserte Restaurationseigenschaft ohne Linkage Drag bzw. mit vermindertem Linkage Drag. Des Weiteren werden auch Organe, Teile, Gewebe oder Zellen der Pflanze zur Verfügung gestellt, welchen die verbesserte Restaurationseigenschaft innewohnt.

Die Erfindung betrifft auch ein Oligonukleotid, vorzugsweise mit einer Länge von maximal 50 Nukleotiden, das eine der folgenden Nukleotidsequenzen aufweist: (i) SEQ ID NOs: 4, 6, 8, 10, 12, 14, 16, 18 oder einem Komplement davon, oder (ii) SEQ ID NOs: 5, 7, 9, 11, 13, 15, 17, 19 oder einem Komplement davon. Solche Oligonukleotide in der Verwendung als molekulare Marker oder auf solchen Oligonukleotiden basierende molekulare Marker sind ebenfalls von der vorliegenden Erfindung miterfasst. Solche molekulare Marker, welche die Anwesenheit oder Abwesenheit eines Markerlocus des Donors nachweisen, basieren beispielsweise auf einem SNP (Beispiele: KASPar oder TaqMan Marker).

Die vorstehend beschriebenen Verbesserungen des erfindungsgemäßen chromosomalen Segments, das beispielsweise aus dem Donor IRAN IX stammt, war ausschließlich durch die umfangreiche und ausgefeilte Entwicklung und Durchdringung des chromosomalen Segments mit molekularen Marker, die eng-gekoppelt zu dem Restaurationslocus für P-CMS sind (siehe oben beschriebene Marker sowie Tabelle 2) und die flankierenden Regionen, welche mutmaßlich die agronomisch nachteiligen Gene (Linkage Drag) tragen. Zudem war eine grundsätzliche Voraussetzung, dass die Marker für ein Hochdurchsatz-Screening geeignet sind. Im Rahmen der vorliegenden Erfindung ist die Erzeugung, Identifizierung und Bewertung von Rekombinanten erstmals gelungen, obwohl die Rekombinationhäufigkeit aufgrund der großen genetischen Distanz zwischen den zentraleuropäischen Elitepopulationen als Empfänger des chromosomalen Segments und den iranischen bzw. argentinischen Donorpopulationen außerordentlich niedrig ist. Diese Schwierigkeiten sind dem Fachman auch aus der Literatur bekannt (Ruge B, Linz A, Pickering R, Proeseler G, Greif P, Wehling P (2003) Mapping of Rym14Hb, a gene introgressed from Hordeum bulbosum and confering resistance to BaMMV and BaYMV in barley. Theor Appl Genet 107:965-971).

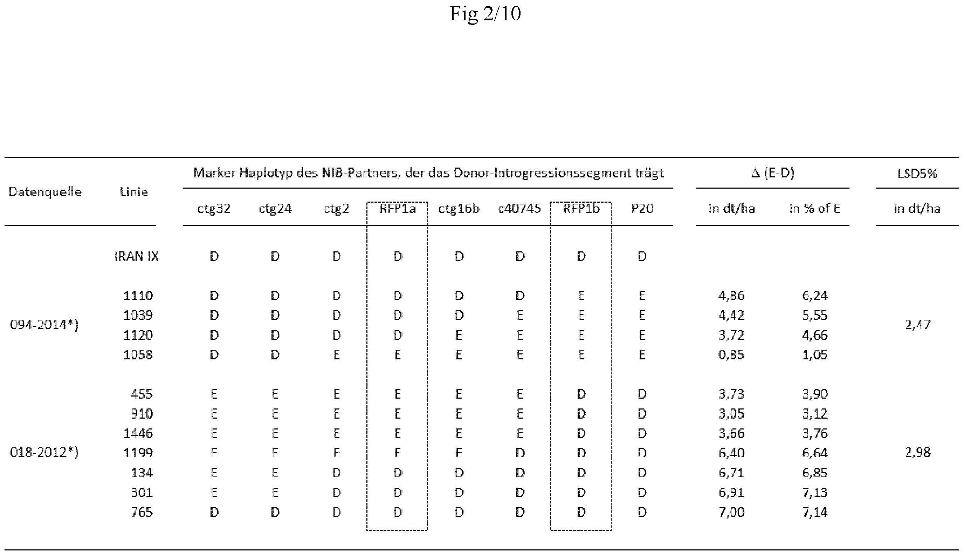

Neben dem außerordentlichen Fortschritt im Bereich des Genotypisierens der Rfp1-Zielregion konnte die vorliegende Erfindung aber auch nur durch die Entwicklung eines neuen hochdiagnostischen Phänotypisierungssystem möglich. Das angewendete "Near Isogenic Bulks (NIB)" Phänotypisierungstests (siehe Beispiel 1 und 2) erlaubten erst die zuverlässige Bestimmung des Linkage Drag-Effekts mit der benötigten Genauigkeit, denn nur so konnte der Linkage Drag-Effekt von Effekten des genetischen Hintergrunds phänotypisch getrennt werden. So können Linkage Drag Effekte als Differenz (Δ_{E-D}) zwischen Testkreuzungsmittelwerten von NIB-Partner, welche das Eliteallel (E), und entsprechenden NIB-Partner, welcher das Donorallel (D) trägt, für alle Marker im chromosomalen Intervall berechnet werden (siehe auch Fig. 2).

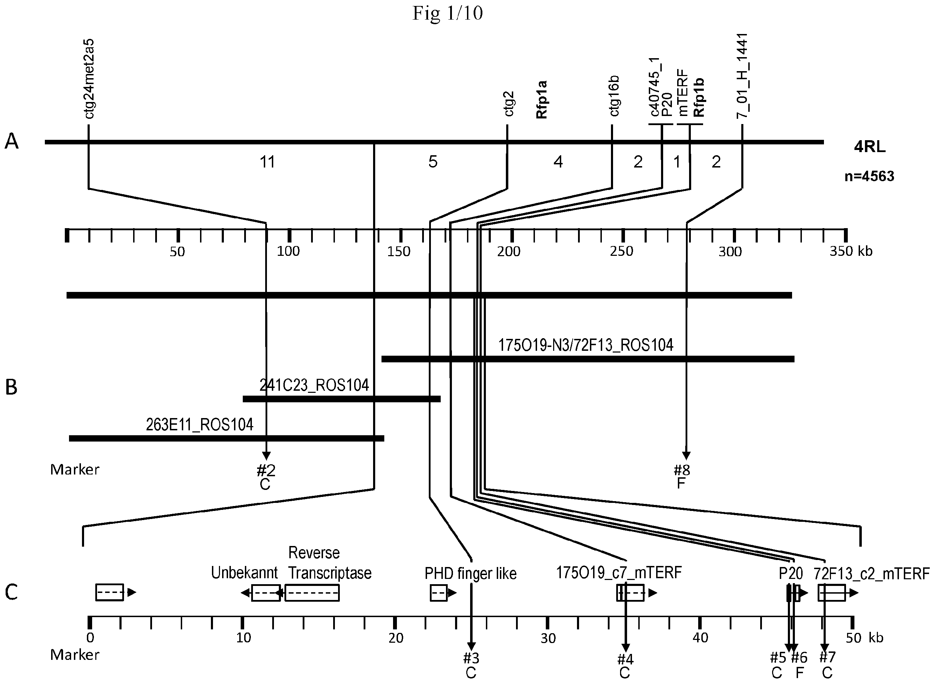

In einem weiteren Aspekt erfasst die vorliegende Erfindung ein Verfahren zur Herstellung einer Pflanze, insbesondere aus der Ordnung der Grasartigen (*Poales*), vorzugsweise der Familie der Süßgräser (Poaceae), die geeignet ist, als männlicher Pollenelter die Pollenfertilität für die P-CMS zu restaurieren, wobei in einer Hybridpflanze aus einer Kreuzung mit einem weiblichen CMS Elter ein ansonsten mit der Restaurationseigenschaft gekoppelter Linkage Drag, vorzugsweise ertragsmindernder Effekt reduziert ist oder vollständig unterbleibt. Ein solches Verfahren umfasst den folgenden Schritt: Entfernen von einem oder mehreren chromosomalen Intervallen, die einen oder mehrere der folgenden Markerloci des Donors enthalten: 7_01_H_1441, ctg24met2a5 oder ctg32, von dem Genom einer Pflanze, vorzugsweise von Chromosom 4R, wobei das erfindungsgemäße chromosomale Segment mit dem *Rfp1a* Gen und/oder *Rfp1b* Gen (siehe auch Figur 1) wie oben beschrieben erhalten bleibt. Beispielsweise kann die Entfernung eines oder mehrerer chromosomaler Intervalle durch genetische Rekombination während eines Kreuzungsprozesses zwischen zwei Pflanzen erreicht werden, wobei eine Pflanze den bekannten *Rfp1* Locus heterozygot trägt. Diese konventionellen Züchtungstechnik zur Erzeugung einer genetischen Rekombination führt zu dem Ergebnis, dass mindestens eines der oben identifizierten Donor-Intervalle mit Linkage Drag durch genomische Sequenzen des rekurrenten Elter, welche bevorzugt frei sind von unerwünschten Genen, ersetzt werden. Somit kann das Entfernen folgende Schritte umfassen: (I) Kreuzung einer ersten Pflanze, umfassend den Restaurationslocus von einem Donor ausgewählt aus der Gruppe bestehend aus IRAN IX, Pico Gentario und Altevogt 14160, mit einer zweiten Pflanze, welche diesen Restaurationslocus nicht aufweist; (II) Selektieren von Nachkommen, welche das erfindungsgemäße chromosomale Segment wie oben beschrieben aufweisen. Bevorzugt erfolgt die Selektion Markerbasiert; geeignete Marker sind durch die vorliegende Offenbarung dem Fachmann zugänglich. Diese Marker-gestützte Selektion der Restorergene kann wesentlich zur Beschleunigung des Zuchtprozesses beitragen, da die gewünschte Information über das Vorhandensein des Restorergens frühzeitig und ohne aufwändige Testkreuzungen erfasst werden kann.

Demnach schließt die vorliegende Erfindung auch ein Verfahren zum Nachweis einer Pflanze, insbesondere aus der Ordnung der Grasartigen (*Poales*), vorzugsweise der Familie der Süßgräser (Poaceae), die geeignet ist, als männlicher Pollenelter die Pollenfertilität für die P-CMS zu restaurieren, wobei in einer Hybridpflanze aus einer Kreuzung mit einem weiblichen CMS Elter ein ansonsten mit der Restaurationseigenschaft gekoppelter Linkage Drag, vorzugsweise ertragsmindernder Effekt reduziert ist oder vollständig unterbleibt, mit ein. Dieses Verfahren umfasst den Nachweis in der Pflanze von Allelen von mindestens zwei Markern, stammend aus einem Donor ausgewählt aus der Gruppe bestehend aus IRAN IX, Pico Gentario und Altevogt 14160, wobei mindestens ein Marker auf bzw. in dem chromosomalen Intervall zwischen tc256739 und ctg2 und mindestens ein Marker auf bzw. in dem chromosomalen Intervall zwischen ctg16b und tc300731 lokalisiert sind, oder wobei mindestens ein Marker auf bzw. in dem chromosomalen Intervall zwischen tc256739 und c40745_1 und mindestens ein Marker auf bzw. in dem chromosomalen Intervall zwischen 7_01_H_1441 und tc300731 lokalisiert sind. Alternativ umfasst das Verfahren den Nachweis in der Pflanze der Anwesenheit oder Abwesenheit von mindestens einem Markerallel, stammend aus einem Donor ausgewählt aus der Gruppe bestehend aus IRAN IX, Pico Gentario und Altevogt 14160, auf bzw. in dem Rfp1-Locus, und Selektieren von Pflanzen, in welchen das mindestens eine Markerallels vorhanden ist. Vorzugsweise meint der Rfp1-Locus einen chromosomalen Abschnitt zwischen den Markerloci tc256739, ctg32 oder ctg24met2a5 und tc300731 oder 7_01_H_1441 auf dem Chromosom 4R von einem Donor ausgewählt aus der Gruppe bestehend aus IRAN IX, Pico Gentario und Altevogt 14160. Der Rfp1-Locus kann beispielsweise eines der folgenden Abschnitte sein: zwischen den Markerloci tc256739 und tc300731, zwischen den Markerloci ctg32 und tc300731, zwischen den Markerloci ctg24met2a5 und tc300731, zwischen den Markerloci ctg2 und tc300731, zwischen den Markerloci ctg16b und tc300731, zwischen den Markerloci c40745_1 und tc300731, zwischen den Markerloci P20 und tc300731, zwischen den Markerloci tc256739 und 7_01_H_1441, zwischen den Markerloci ctg32 und 7_01_H_1441, zwischen den Markerloci ctg24met2a5 und 7_01_H_1441, zwischen den Markerloci ctg2 und 7_01_H_1441, zwischen den Markerloci ctg16b und 7_01_H_1441, zwischen den Markerloci c40745_1 und 7_01_H_1441, zwischen den Markerloci P20 und 7_01_H_1441, zwischen den Markerloci tc256739 und 72F13_c2_mTERF, zwischen den Markerloci tc256739 und P20, zwischen den Markerloci tc256739 und c40745_1, zwischen den Markerloci tc256739 und ctg16b, zwischen den Markerloci ctg32 und 72F13_c2_mTERF, zwischen den Markerloci ctg32 und P20, zwischen den Markerloci ctg32 und c40745_1, zwischen den Markerloci ctg32 und ctg16b, zwischen den Markerloci ctg24met2a5 und 72F13_c2_mTERF, zwischen den Markerloci ctg24met2a5 und P20, zwischen den Markerloci ctg24met2a5 und c40745_1, zwischen den Markerloci ctg24met2a5 und ctg16b, zwischen den Markerloci ctg2 und 72F13_c2_mTERF, zwischen den Markerloci ctg2 und P20, zwischen den Markerloci ctg2 und c40745_1 oder zwischen den Markerloci ctg2 und ctg16b. Beispielsweise können zum Nachweis einer oder mehrere der folgenden Oligonukleotide, die eine der folgenden Nukleotidsequenzen aufweist: (i) SEQ ID Nr.: 4, 6, 8, 10, 12, 14, 16, 18 oder einem Komplement davon, oder (ii) SEQ ID Nr.: 5, 7, 9, 11, 13, 15, 17, 19 oder einem Komplement davon, als Marker eingesetzt werden. Im Rahmen der vorliegenden Erfindung wurden mittels der oben bereits beschriebenen Marker rekombinante Genotypen identifiziert und das jeweils verbleibende Introgressionssegment beschrieben; siehe Beispiel 3. Der Marker P20, wie in Beispiel 3 dargestellt, spielte bei der Identifizierung und Beschreibung die wichtigste Rolle, da mit seiner Hilfe weitere Markersequenzen identifiziert werden konnten und zahlreiche Markerkombinationen entworfen werden konnten, siehe Tabelle 2 und Beispiel 6.

Alternativ stellt die moderne Biotechnologie dem Fachmann diverse weitere Werkzeuge zur Verfügung, welche ein präzises Genom-Engineering ermöglichen: Gentechnische Ansätze, mit deren Hilfe die Eliminierung von Linkage Drag-tragenden Nukleotidsequenzen aus einem pflanzlichen Genom unterstützt wird oder unmittelbar erreicht werden kann, umfassen die Verwendung von TALE-Nukleasen (TALENs) oder Zinkfinger-Nukleasen (ZFNs) sowie CRISPR/Cas Systeme, die u.a. beispielhaft in der deutschen Patentanmeldung DE 10 2013 014 637 zur Eliminierung von Linkage Drag-tragenden Nukleotidsequenzen aus dem Genom von *Helminthosporium turcicum* resistenten (Hybrid)Mais beschrieben sind; siehe die DE 10 2013 014 637 auf Seite 13 und 14 in Paragraphen [0038] bis [0042] und die dort zitierten Referenzen. Diese Techniken, die auch in der internationalen Patentanmeldung WO 2014/104878 beschrieben sind, können equivalent in der Herstellung der vorliegenden erfindungsgemäßen Pflanzen verwendet werden.

Die vorliegende Erfindung schließt weiterhin auch eine Kombination von der konventionellen Züchtungstechnik und der moderne Biotechnologie mit ein. So können beispielsweis mit Hilfe dieser neuartigen Genom-Editing Ansätze Rekombinations-"Hot spots" in einer Pflanze erzeugt werden, die an geeigneten Stellen stattfinden, um die Entfernung des Linkage Drags unmittelbar zu begünstigen. Die vorliegende Erfindung stellt dem Fachmann hierfür die notwendigen Informationen über Lokalisation des Linkage Drags, sowie der Position des Restaurationsgenes / derRestaurationsgene zur Verfügung.

Darüberhinaus ermöglichen die neuartigen Genom-Editing Ansätze auch ein direktes Einbringen des erfindungsgemäßen chromosomalen Segments mit vermindertem oder vollständig aufgehobenen Linkage Drag. Somit ist von dieser Erfindung auch ein weiteres Verfahren zur Herstellung einer erfindungsgemäßen Pflanze, insbesondere aus der Ordnung der Grasartigen (*Poales*), vorzugsweise der Familie der Süßgräser (Poaceae), die geeignet ist, als männlicher Pollenelter die Pollenfertilität für die Pampa cytoplasmatische männliche Sterilität (CMS) zu restaurieren, wobei in einer Hybridpflanze aus einer Kreuzung mit einem weiblichen CMS Elter ein ansonsten mit der Restaurationseigenschaft gekoppelter Linkage Drag, vorzugsweise ertragsmindernder Effekt reduziert ist oder vollständig unterbleibt, miterfasst. Ein solches Verfahren umfasst die folgenden Schritte: (I) Bereitstellen eines Teils einer Pflanze, welche vorzugsweise den Restaurationslocus der vorliegenden Erfindung nicht trägt, als Zielstruktur enthaltend die Nukleinsäure-Zielregion, vorzugsweise eine genomische DNA welche der chromosomalen Positionierung derjenigen des Bereichs des Rfp1-Locus entspricht; (II) Bereitstellen eines oder mehrerer rekombinanter Konstrukte, welche zusammen die Komponenten des Genome Editing Werkzeugs umfassen oder kodieren; (III) Bereitstellen mindestens eines Vektors zur Einbringung des bzw. der rekombinanten Konstrukts/Konstrukte; (IV) Bereitstellen mindestens eines weiteren rekombinanten Konstrukts umfassend das erfindungsgemäß definierte Nukleinsäuremolekül, die rekombinante DNA, die Expressionskassette oder das chromosomale Segment zur gezielten Homologie-gerichteten Reparatur der Nukleinsäure-Zielregion in der pflanzlichen Zielstruktur oder Insertion in die Nukleinsäure-Zielregion in der pflanzlichen Zielstruktur; (V) Einbringen der rekombinanten Konstrukte aus (II) und (IV) in die pflanzliche Zielstruktur; (VI) Kultivieren der pflanzlichen Zielstruktur unter Bedingungen, welche die Aktivierung der Komponenten des Genome Editing Werkzeugs bewirken und dadurch eine gezielte Veränderung der Nukleinsäure-Zielregion in der pflanzlichen Zielstruktur gestatten, um eine pflanzliche Zielstruktur, umfassend mindestens eine Zelle, die die gezielte Veränderung der Nukleinsäure-Zielregion umfasst, zu erhalten; und (VII) Regenerieren einer Pflanze aus der mindestens einen Zelle.

In einem weiteren Aspekt erfasst die vorliegende Erfindung ein Verfahren zur Herstellung einer erfindungsgemäßen Hybridpflanze, bevorzugt aus der Ordnung der Grasartigen (*Poales*), besonders bevorzugt aus der Familie der Süßgräser (*Poaceae*) oder der Gattung *Secale* oder *Hordeum* und ganz besonders bevorzugt der Spezies *Secale cereale* oder *Hordeum vulgare.* Dieses Verfahren umfasst in einen ersten Schritt (1) eines der Verfahren zur Herstellung einer Pflanze, die geeignet ist, als männlicher Pollenelter die Pollenfertilität für die Pampa cytoplasmatische männliche Sterilität (CMS) zu restaurieren, wie definiert in den vorhergehenden Absätzen. In einem weiteren Schritt (2) dieses Verfahrens wird die in die Schritt 1 erzeugte Pflanze oder ein Nachkomme davon, welche das erfindungsgemäße chromosomale Segment oder das erfindungsgemäße Nukleinsäuremolekül weiterhin aufweist, als männlicher Pollenelter mit einem weiblichen CMS-Elter, bevorzugt derselben Spezies, gekreuzt. Hierbei handelt sich sich bei dem männlicher Pollenelter und/oder dem weiblichen CMS-Elter vorzugsweise um eine doppelhaploide Pflanze,eine Inzuchtpflanze, eine CMS-Einfachkreuzung oder einen sogenannten Polleneltersynthetic. In einem Schritt (3) erfolgt das Ernten des hybriden Saatgutes von dem weiblichen CMS-Elter. Ein optionaler Schritt (4) umfasst das Aussäen des hybriden Saatguts zur Erzeugung der Hybridpflanze und weiterere optionale Schritte umfassen (5) das Ernten des Saatgutes von der Hybridpflanze und (6) das Aussäen des Saatgutes von der Hybridpflanze. Die vorliegende Erfindung schließt ferner Saatgut bzw. Samen und Pflanzen bzw. Hybridpflanzen erhalten oder erhältlich durch vorstehendes Verfahren ein.

In einem weiteren Aspekt betrifft die vorliegende Erfindung auch ein Nukleinsäuremolekül, das geeignet ist, die Restaurationseigenschaft mit vermindertem oder vollständig aufgehobenen Linkage Drag zu vermitteln, wobei das Nukleinsäuremolekül eine Nukleotidsequenz aufweist, welche ausgewählt ist aus der Gruppe bestehend aus einer: (i) Nukleotidsequenz gemäß einer der SEQ ID NO: 1 oder SEQ ID NO: 28 oder einem funktionellen Fragment davon, (ii) Nukleotidsequenz, die eine Aminosäuresequenz gemäß einer der SEQ ID NO: 2 oder SEQ ID NO: 29 oder einem funktionellen Fragment davon kodiert, (iii) Nukleotidsequenz, die komplementär ist zu einer Nukleotidsequenz nach (i) oder (ii), (iv) Nukleotidsequenz, welche an eine Sequenz nach (iii) unter stringenten Bedingungen hybridisiert, (v) Nukleotidsequenz, die eine Identität von mindestens 70%, 75%, 80%, 85% oder 90%, bevorzugt von mindestens 91%, 92%, 93% 94% oder 95%, oder besonders bevorzugt von mindestens 96%, 97%, 98%, 99% oder 99,5% zu der Nukleotidsequenz nach (i) oder (ii) aufweist, (vi) Nukleotidsequenz, die eine Aminosäuresequenz, die eine Identität von mindestens 65%, 70%, 75%, 80%, 85% oder 90%, bevorzugt von mindestens 91%, 92%, 93% 94% oder 95%, oder besonders bevorzugt von mindestens 96%, 97%, 98%, 99% oder 99,5% zu der SEQ ID NO: 2 oder einem funktionellen Fragment davon aufweist, kodiert, (vii) Nukleotidsequenz, die eine Aminosäuresequenz kodiert, die gegenüber der in SEQ ID NO: 2 oder SEQ ID NO: 29 gezeigten Aminosäuresequenz Abweichungen in Form von Aminosäure-Deletionen, Substitutionen, Additionen und/oder Insertionen in der Aminosäuresequenz aufweist, vorzugsweise nicht mehr als 30%, 25% oder 20%, bevorzugt nicht mehr als 18%, 16%, 14%, 12% oder 10% oder besonders bevorzugt nicht mehr als 9%, 8%, 7%, 6%, 5%, 4%, 3%, 2%, 1% oder 0,5% über die gesamte Aminosäuresequenz. Durch die genaue Identifizierung und Feinkartierung der Restaurationseigenschaft des Restorerlocus *Rfp1* ist es möglich, das oben definierte Nukleinsäuremolekül auch auf andere Weisen zu nutzen, um die verbesserten Eigenschaften der Pflanze zu erhalten. Aus diesem Grund umfasst die hier vorliegende Erfindung auch eine Expressionskassette, rekombinante DNA oder Vektoren, die jeweils das Nukleinsäuremolekül umfassen.

In einer Ausführungsform wird das Nukleinsäuremolekül von einer rekombinanten DNA umfasst. Dabei wird in der Regel ein Promotor und/oder andere Transkriptions- oder Translationskontrollelemente enthalten sein bzw. mit dieser assoziiert sein. Bei den verwendeten Promotoren wird es sich hauptsächtlich um Promotoren handeln, die die Transkription der DNA nur in vorbestimmten Zellen ermöglichen. Neben den Promotoren gibt es eine Vielzahl weiterer Transkriptionskontrollelemente, wie beispielsweise die Enhancer, Operatoren, Repressoren und Transkriptionsterminationssignale, jedoch nicht auf diese beschränkt, welche mit der DNA funktionell verbunden sind, um eine gerichtete Zell-spezifische Transkription zu ermöglichen. Promotoren und andere Transkriptionsregulationselemente sind allgemein bekannt und dem Fachmann im Stand der Technik zugänglich; siehe beispielsweise WO 00/75359 auf Seite 23, Zeile 5 bis Seite 24, Zeile 17.

Bei dem Vektor kann es sich um ein Plasmid, ein Cosmid, eine Phage oder einen Expressionsvektor, einen Transformationsvektor, Shuttle-Vektor oder Klonierungsvektor handeln, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist das erfindungsgemäße Nukleinsäuremolekül in einem Vektor mit einer oder mehreren regulatorischen Sequenzen, welche die Transkription und optional die Expression in einer prokaryotischen oder eukaryotischen Wirtszelle erlauben, operativ verknüpft; siehe beispielsweise Sambrook et al., Molecular Cloning: A Laboratory Manual 3rd Ed. Cold Spring Harbor Laboratory Press. Cold Spring Harbor, NY, 2001 und die internationale Anmeldung WO 00/75359 auf Seite 21, Zeile 20 bis Seite 22, Zeile 32. Eine regulatorische Sequenz, vorzugsweise DNA, kann homolog oder heterolog zu der erfindungsgemäßen Nukleinsäure sein. Beispielsweise steht die Nukleinsäure unter der Kontrolle eines geeigneten Promotors oder eines Terminators. Geeignete Promotoren können Promotoren sein, welche konstitutiv induziert werden (Bsp.: 35S-Promotor aus dem "Cauliflower mosaic virus" (Odell et al. 1985), welche gewebespezifisch, stressspezifisch oder entwicklungsspezifisch sind (Bsp.: Antherenspezifische Expression). Geeignete Promotoren können auch synthetische bzw. chimäre Promotoren sein, welche in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Chimäre Promotoren können den gewünschten Spezifitäten nach konzipiert werden und werden durch unterschiedliche Faktoren induziert oder reprimiert. Beispiele für solche Promotoren finden sich in Gurr & Rushton (Gurr, SJ; Rushton, PJ. Engineering plants with increased disease resistance: what are we going to express?. TRENDS in Biotechnology, 2005, 23. Jg., Nr. 6, S. 275-282) oder Venter (Synthetic promoters: genetic control through cis engineering. Trends in Plant Science, 2007, 12. Jg., Nr. 3, S. 118-124). Ein geeigneter Terminator ist beispielsweise der nos-Terminator (Depicker, A, Stachel, S, Dhaese, P, Zambryski ,P and Goodman, H (1982) J. Mol. Appl. Genet., 1, 561-575).

Zusätzlich zu den oben beschriebenen Vektoren, stellt die vorliegende Erfindung auch ein Verfahren bereit, das die Einbringung eines beschriebenen Vektors in eine Wirtszelle umfasst. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren ebenso wie Verfahren zur Präparation von beschriebenen Vektoren sind dem Fachmann geläufig (Sambrook et al. 2001, Molecular cloning: a laboratory manual (3-volume set) (Vol. 999). Cold Spring Harbor, New York:: Cold spring harbor laboratory press). Ferner gibt es verschiedene Verfahren im Stand der Technik mit denen sich transgene Pflanzen herstellen lassen und das Restaurationsmerkmal eingebracht werden kann. Diese beinhalten direkte und indirekte Verfahren. Die Verfahren schließen Partikelbeschuss (Weeks et al. Plant Physiol. 102, (1993) 1077-1084; Vasil et al., Bio/Technology 10 (1992), 662-674), Agrobacterium-Transformation (Chan et al., Plant Mol. Biol. 22 (1993), 491-506), Elektroporation von regenerierbarem Gewebe (Shillito et al. 1985 "High efficiency direct gene transfer to plants." Nature Biotechnology 3.12: 1099-1103), Siliziumcarbidfaser-vermittelte Genübertragung (Dalton et al., Plant Sci. 132 (1998), 31-43) und Protoplasten-vermittelte Genübertragung (Shimamoto et al., Nature, 338 (1989), 274-276), biolistischer oder Agrobacterium-verrnittelter Gentransfer (WO 01/73084) ein. Die Einbringung des Restaurationsmerkmals kann auch durch Introgression (Harper et al., Annals of Botany 107: (2011), 1313-1320) oder auch mittels gentechnischem Ansatz erfolgen. Zahlreiche neuartige gentechnologische Verfahren zum Einbringen von DNA aber auch zum Inaktivieren von genomischen Sequenzen sind dem Fachmann bekannt (z.B. Verfahren des Genome Editings basierend auf Zinkfinger-Nukleasen, TALENs oder einem CRISPR/Cas System).

Alternativ oder zusätzlich betrifft die vorliegende Erfindung auch Wirtszellen, die das Nukleinsäuremolekül als Transgen, die Expressionskassette, rekombinante DNA als Transgen oder den Vektor wie oben dargestellt umfassen. Eine Wirtszelle im Sinne der Erfindung kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium wie *Agrobacterium tumefaciens* oder *Agrobacterium rhizogenes* oder eine Pflanzenzelle, welche das Nukleinsäuremolekül, die Expressionskassette, die rekombinante DNA oder den Vektor der vorliegenden Erfindung umfassen. Dem Fachmann sind sowohl zahlreiche Methoden wie Konjugation oder Elektroporation bekannt, mit denen er das Nukleinsäuremolekül, die Expressionskassette, die rekombinante DNA oder den Vektor der vorliegenden Erfindung in ein Agrobakterium einbringen kann, als auch Methoden wie diverse Transformationsverfahren (biolistische Transformation, Agrobakterium-vermittelte Transformation), mit denen er das Nukleinsäuremolekül, die Expressionskassette, die rekombinante DNA oder den Vektor der vorliegenden Erfindung in eine Pflanzenzelle einbringen kann (Sambrook et al. 2001).

Durch die Identifizierung der die Restauration-vermittelnden Gene, wird deren Nutzung auch in transgenen Pflanzen möglich, wobei das Mitführen von Linkage Drag auf ein Minimum reduziert werden kann. Somit schließt die Erfindung auch die Bereitstellung einer transgenen Pflanze oder Samen davon, welche eine die zuvor definierte Pflanzenzelle umfassen, ein. Eine solche transgene Pflanzenzelle oder Pflanze ist beispielsweise eine Pflanzenzelle oder Pflanze, welche mit dem erfindungsgemäßen Nukleinsäuremolekül, mit der Expressionskassette, mit der rekombinanten DNA oder mit dem Vektor der vorliegenden Erfindung, vorzugsweise stabil, transformiert ist. Die transgene Pflanze weist eine neu-vermittelte Restaurationseigenschaft für die Pollenfertilität für die Pampa cytoplasmatische männliche Sterilität (CMS) oder eine verbesserte Restaurationseigenschaft für die Pollenfertilität für die Pampa cytoplasmatische männliche Sterilität (CMS) im Vergleich zu einer Wildtyp-Pflanze, welche isogen ist, jedoch nicht mit dem erfindungsgemäßen Nukleinsäuremolekül, mit der Expressionskassette, mit der rekombinanten DNA oder mit dem Vektor der vorliegenden Erfindung, vorzugsweise stabil, transformiert ist, auf. Bevorzugt zeigen diese transgenen Pflanzen zusätzlich eine neu-vermittelte Resistenz gegen ein Pathogen, vorzugsweise gegen einen Pilz, insbesondere gegen den Pilz Claviceps purpurea (Fr.), oder eine erhöhte Resistenz gegen ein Pathogen aufweist, vorzugsweise gegen einen Pilz, insbesondere gegen den Pilz Claviceps purpurea (Fr.) im Vergleich zu einer Wildtyp-Pflanze, welche isogen ist, jedoch nicht mit dem erfindungsgemäßen Nukleinsäuremolekül, mit der Expressionskassette, mit der rekombinanten DNA oder mit dem Vektor der vorliegenden Erfindung, vorzugsweise stabil, transformiert ist.

Die vorliegende Erfindung umfasst ferner neben dem Nukleinsäuremolekül, das die Restaurationseigenschaft mit vermindertem oder vollständig aufgehobenen Linkage Drag kodiert, auch ein durch das Nukleinsäuremolekül kodierbares mTERF Protein oder Homolog, Analog, Ortholog oder ein funktionelles Fragment davon sowie einen Antikörper, der spezifisch an das mTERF Protein oder Homolog, Analog, Ortholog oder ein funktionelles Fragment davon bindet. Das mTERF Protein umfasst vorzugsweise eine Aminosäuresequenz gemäß einer der SEQ ID NO: 2 oder SEQ ID NO: 29 oder eine Aminosäuresequenz, die eine Identität von mindestens 65%, 70%, 75%, 80%, 85% oder 90%, bevorzugt von mindestens 91%, 92%, 93% 94% oder 95%, oder besonders bevorzugt von mindestens 96%, 97%, 98%, 99% oder 99,5% zu der SEQ ID NO: 2 oder SEQ ID NO: 29 aufweist. Weiterhin erfasst die vorliegende Erfindung auch einen Antikörper, der spezifisch an das mTERF Protein bindet. Die rekombinante Herstellung von Proteinen und funktionellen Fragmenten davon ist dem Fachmann geläufig und beispielsweise in Sambrook et al. (Molecular Cloning: A Laboratory Manual 3rd Ed. Cold Spring Harbor Laboratory Press. Cold Spring Harbor, NY, 2001 Wingfield, P. T. 2008. Production of Recombinant Proteins. Current Protocols in Protein Science. 52:5.0:5.0.1-5.0.4) beschrieben. Polyklonale oder monoklonale Antikörper zu dem Protein der vorliegenden Erfindung können von dem Fachmann nach bekannten Verfahren hergestellt werden, wie sie beschrieben sind in E. Harlow et al. (Herausgeber Antibodies: A Laboratory Manual (1988)). Die Herstellung von monoklonalen Antikörpern sowie von Fab- und F(ab')₂-Fragmenten, die auch bei Proteindetektionsmethoden nützlich sind, kann durchgeführt werden durch verschiedene gebräuchliche Methoden wie sie beschrieben sind in Goding (Mononoclonal Antibodies: Principles and Practice, S. 98-118, New York: Academic Press (1983)).

Die Verwendung von Antikörper zur Herstellung und Selektion von Hybridpflanzen bzw. transgenen Pflanzen mit erhöhtem Ertrag sind beispielhaft beschrieben in der internationalen Patentanmeldung WO 2011/061656 in Paragraphen [00678] und [00847] und dort zitierten Referenzen. Diese Techniken können äquivalent in der Herstellung der vorliegenden erfindungsgemäßen Pflanzen verwendet werden.

Ein weiterer Aspekt der vorliegenden Erfindung ist ein Verfahren zur Herstellung einer Pflanze, insbesondere aus der Ordnung der Grasartigen (*Poales*), die geeignet ist, als männlicher Pollenelter, die Pollenfertilität für die Pampa cytoplasmatische männliche Sterilität (CMS) zu restaurieren. Ein solches Verfahren umfasst die folgenden Schritte: A) Mutagenisieren von Pflanzenzellen oder von Teilen einer Pflanze und anschließendem Regenerieren von Pflanzen aus den mutagenisierten Pflanzenzellen oder mutagenisierten Teilen oder Mutagenisieren von Pflanzen, und B) Identifizieren einer Pflanze aus A), welche in einer endogenen DNA-Sequenz, welche zu einer Nukleinsäuresequenz, ausgewählt aus der Gruppe bestehend aus der: (i) Nukleotidsequenz gemäß einer der SEQ ID NO: 1 oder SEQ ID NO: 28 oder einem funktionellen Fragment davon, (ii) Nukleotidsequenz, die eine Aminosäuresequenz gemäß einer der SEQ ID NO: 2 oder SEQ ID NO: 29 oder einem funktionellen Fragment davon kodiert, (iii) Nukleotidsequenz, die komplementär ist zu einer Nukleotidsequenz nach (i) oder (ii), (iv) Nukleotidsequenz, welche an eine Sequenz nach (iii) unter stringenten Bedingungen hybridisiert, (v) Nukleotidsequenz, die eine Identität von mindestens 70%, 75%, 80%, 85% oder 90%, bevorzugt von mindestens 91%, 92%, 93% 94% oder 95%, oder besonders bevorzugt von mindestens 96%, 97%, 98%, 99% oder 99,5% zu der Nukleotidsequenz nach (i) oder (ii) aufweist, (vi) Nukleotidsequenz, die eine Aminosäuresequenz, die eine Identität von mindestens 65%, 70%, 75%, 80%, 85% oder 90%, bevorzugt von mindestens 91%, 92%, 93% 94% oder 95%, oder besonders bevorzugt von mindestens 96%, 97%, 98%, 99% oder 99,5% zu der SEQ ID NO: 2 oder einem funktionellen Fragment davon aufweist, kodiert, (vii) Nukleotidsequenz, die eine Aminosäuresequenz kodiert, die gegenüber der in SEQ ID NO: 2 oder SEQ ID NO: 29 gezeigten Aminosäuresequenz Abweichungen in Form von Aminosäure-Deletionen, Substitutionen, Additionen und/oder Insertionen in der Aminosäuresequenz aufweist, vorzugsweise nicht mehr als 30%, 25% oder 20%, bevorzugt nicht mehr als 18%, 16%, 14%, 12% oder 10% oder besonders bevorzugt nicht mehr als 9%, 8%, 7%, 6%, 5%, 4%, 3%, 2%, 1% oder 0,5% über die gesamte Aminosäuresequenz, identisch ist, oder in einer regulatorischen Sequenz der endogenen DNA-Sequenz mindestens eine Mutation aufweist, die bewirkt, dass die identifizierte Pflanze eine neu-vermittelte Restaurationseigenschaft für die Pollenfertilität für die Pampa cytoplasmatische männliche Sterilität (CMS) oder eine verbesserte Restaurationseigenschaft für die Pollenfertilität für die Pampa cytoplasmatische männliche Sterilität (CMS) im Vergleich zu einer nicht-mutierten Wildtyp-Pflanze, welche ansonsten isogen ist und/oder eine neu-vermittelte Resistenz gegen ein Pathogen, vorzugsweise gegen einen Pilz, insbesondere gegen den Pilz Claviceps purpurea (Fr.), oder eine erhöhte Resistenz gegen ein Pathogen aufweist, vorzugsweise gegen einen Pilz, insbesondere gegen den Pilz Claviceps purpurea (Fr.) im Vergleich zu einer nicht-mutierten Wildtyp-Pflanze, welche ansonsten isogen ist, aufweist.

Bevorzugt kodiert die endogene DNA-Sequenz aus Schritt B) für ein mTERF-Protein, besonders bevorzugt für das Protein mTERF gemäß einer der SEQ ID NOs: 2 oder der SEQ ID NO: 29 oder ein Homolog, Analog oder Ortholog davon. Bevorzugt ist die regulatorische Sequenz der endogenen DNA-Sequenz aus Schritt B) ein Promotor oder ein Teil davon. Ein Beispiel für eine regulatorische Sequenz der endogenen DNA-Sequenz ist der Promotor gemäß SEQ ID NO: 3.

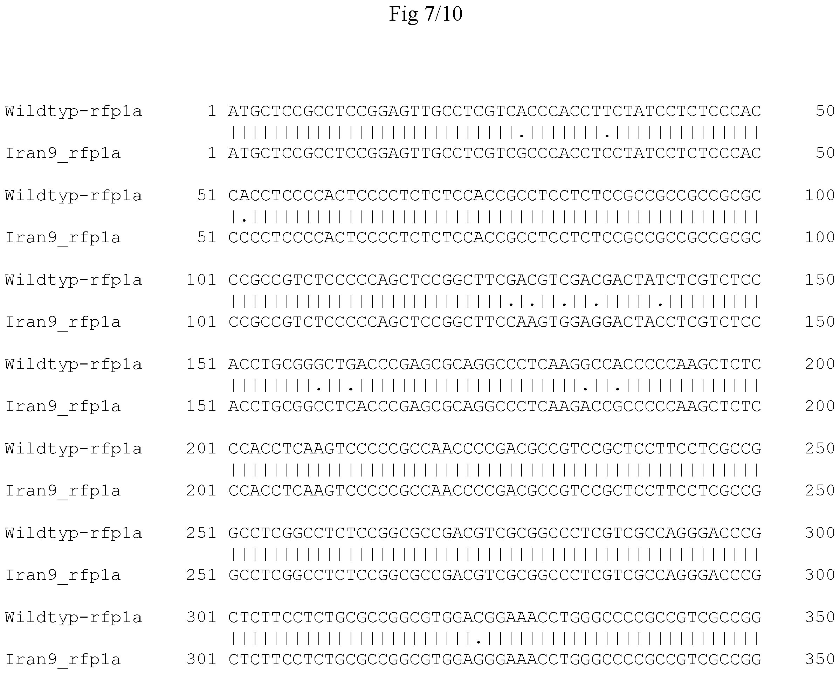

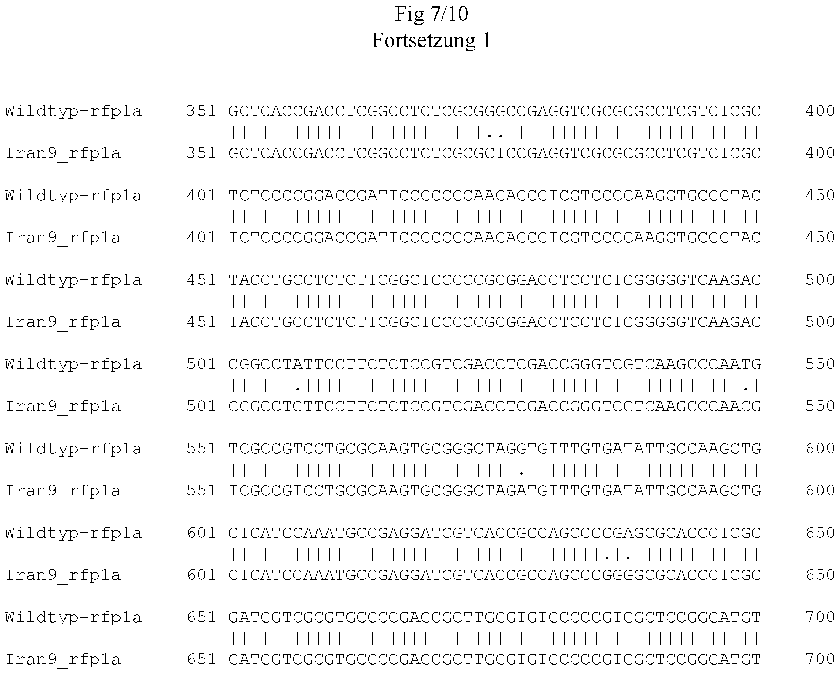

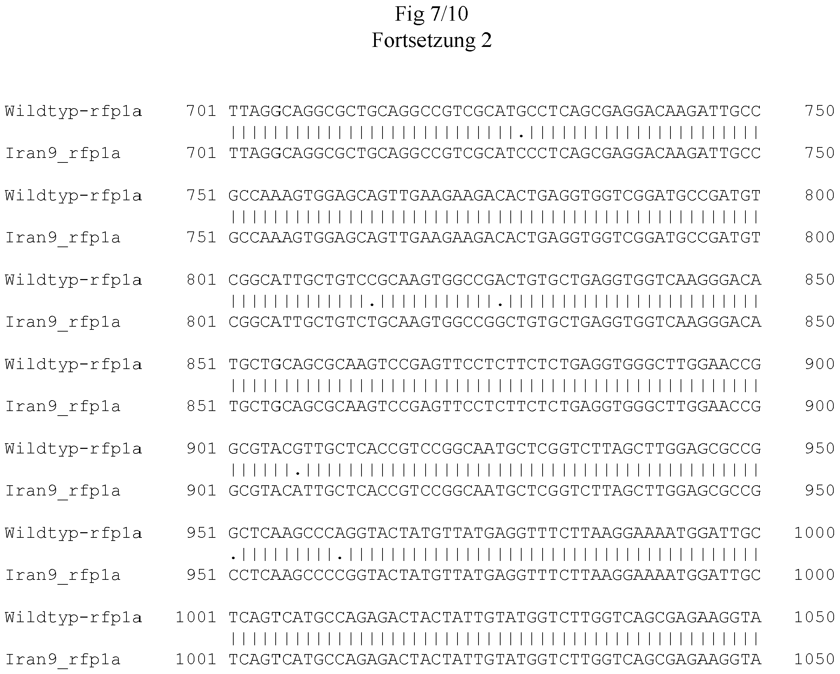

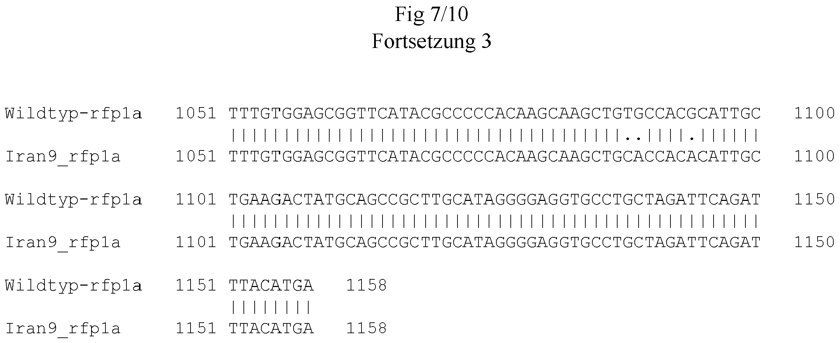

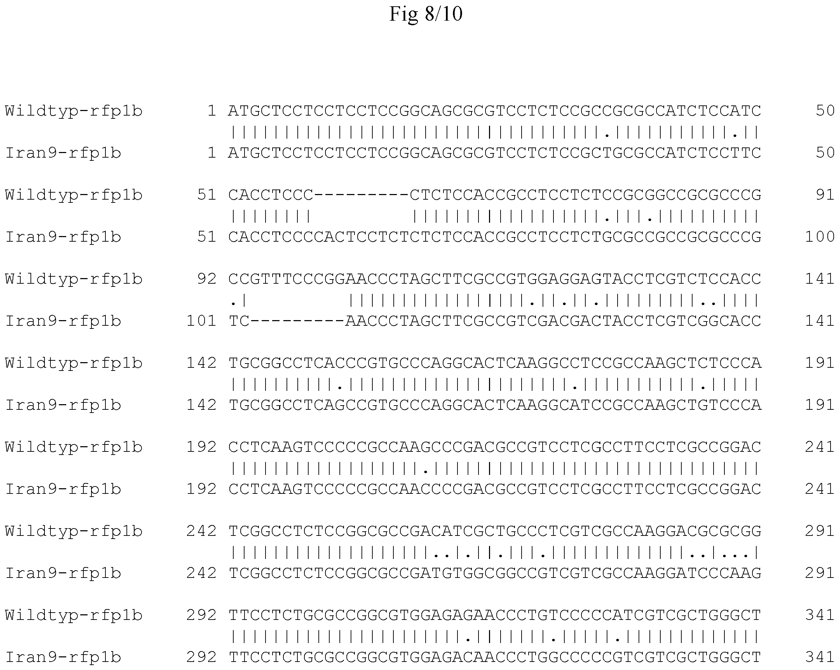

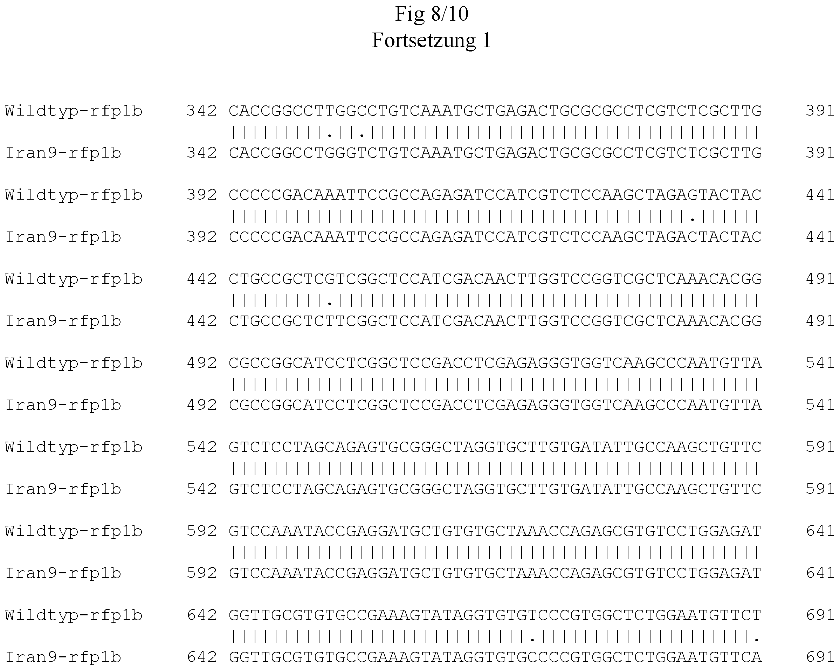

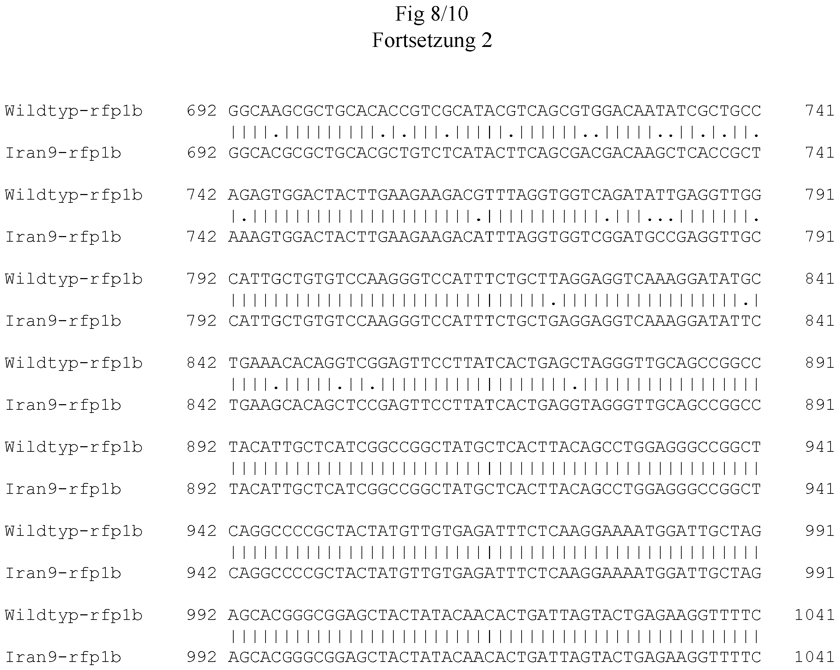

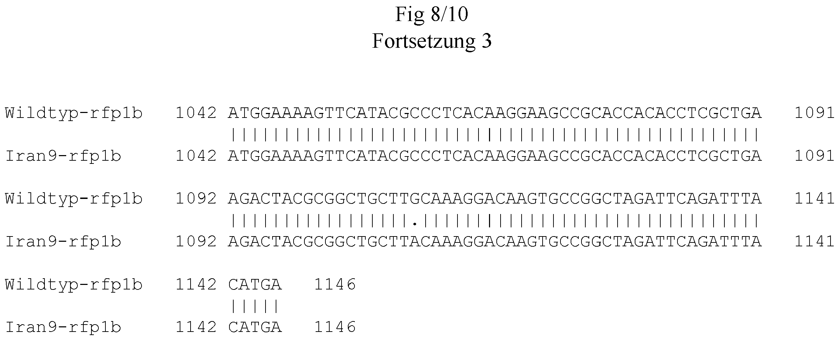

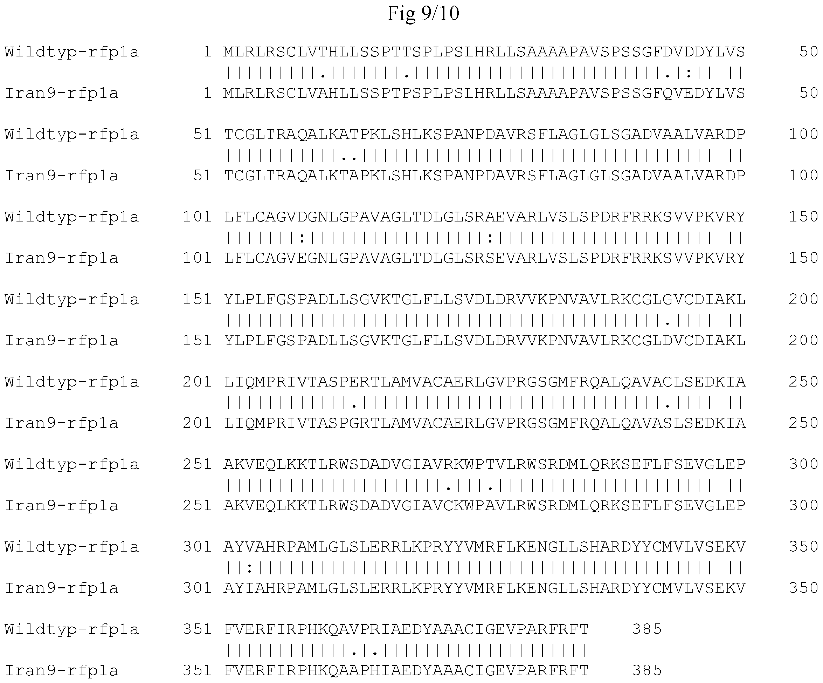

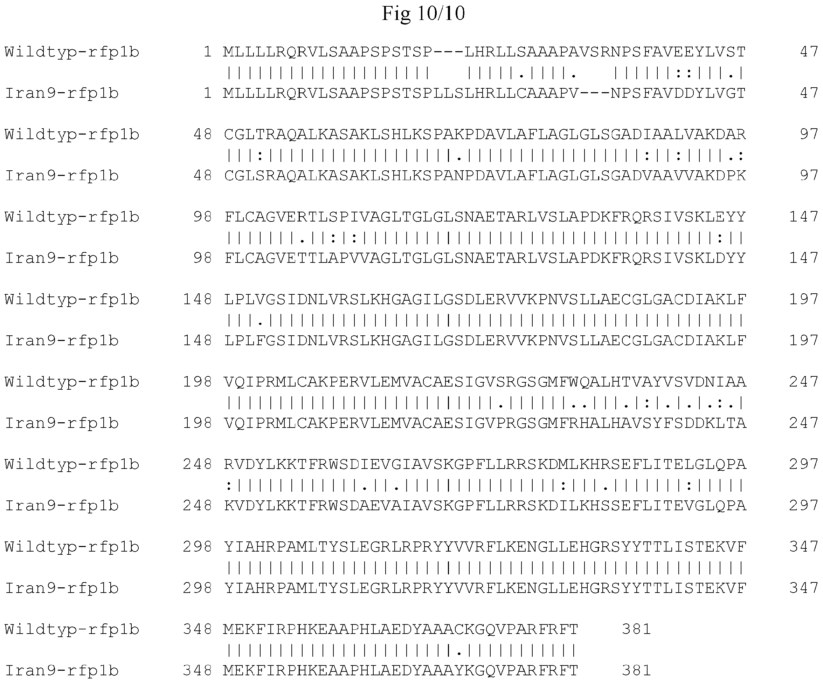

Eine Mutation meint eine Modifikation auf DNA-Ebene, also eine Veränderung der Genetik und/oder der Epigenetik. Beispielsweise kann eine Veränderung der Genetik der Austausch von mindestens einer Nukleobase in der endogenen DNA-Sequenz oder in einer regulatorischen Sequenz der endogenen DNA-Sequenz sein. Findet ein solcher Nukleobasen-Austausch z.B. in einem Promotor statt, kann dies zu einer veränderten Aktivität des Promotors führen, da beispielsweise cis-regulatorische Elemente derart modifiziert werden, dass die Affinität eines Transkriptionsfaktors zu dem mutierten cis-regulatorische Elemente im Vergleich zum Wildtyp-Promotor verändert ist, sodass die Aktivität des Promotors mit dem mutierten cis-regulatorische Elemente erhöht oder erniedrigt ist, je nachdem ob es sich bei dem Transkriptionsfaktor um einen Repressor oder Induktor handelt oder ob die Affinität des Transkriptionsfaktors zu dem mutierten cis-regulatorische Elemente verstärkt oder abgeschwächt wird. Findet ein solcher Nukleobase-Austausch z.B. in einer kodierenden Region der endogenen DNA-Sequenz statt, kann dies zu einem Aminosäureaustausch im kodierten Protein führen, was eine Veränderung der Aktivität oder Stabilität des Proteins im Vergleich zum Wildtyp-Protein bewirken kann. Mögliche Aminosäureaustausche können aus Vergleichen der Aminosäuresequenzen abgeleitet werden. Figur 9 zeigt einen Vergleich der Wildtypsequenz des rfp1a (SEQ ID NO: 33) mit der die Restaurationseigenschaft vermittelnden Aminosäuresequenz aus IRAN9 (SEQ ID NO: 29). Beispielhaft sind hier folgende potentielle Aminosäureaustausche ableitbar: An Position 10 der SEQ ID NO: 29, an welcher das die Restaurationseigenschaft vermittelnde mTERF Protein ein Alanin (A) und ein entsprechendes (nicht-restaurierendes Protein) aus dem Wildtyp (SEQ ID NO: 33) ein Threonin (T) aufweist, an Position 18 der SEQ ID NO: 29, an welcher das die Restaurationseigenschaft vermittelnde mTERF Protein ein Prolin (P) und ein entsprechendes (nicht-restaurierendes Protein) aus dem Wildtyp (SEQ ID NO: 33) ein Threonin (T) aufweist, an Position 43 der SEQ ID NO: 29, an welcher das die Restaurationseigenschaft vermittelnde mTERF Protein ein Glutamin (Q) und ein entsprechendes (nicht-restaurierendes Protein) aus dem Wildtyp (SEQ ID NO: 33) ein Asparaginsäure (D) aufweist, an Position 45 der SEQ ID NO: 29, an welcher das die Restaurationseigenschaft vermittelnde mTERF Protein ein Glutaminsäure (E) und ein entsprechendes (nicht-restaurierendes Protein) aus dem Wildtyp (SEQ ID NO: 33) ein Asparaginsäure (D) aufweist, an Position 62 der SEQ ID NO: 29, an welcher das die Restaurationseigenschaft vermittelnde mTERF Protein ein Threonin (T) und ein entsprechendes (nicht-restaurierendes Protein) aus dem Wildtyp (SEQ ID NO: 33) ein Alanin (A) aufweist, an Position 63 der SEQ ID NO: 29, an welcher das die Restaurationseigenschaft vermittelnde mTERF Protein ein Alanin (A) und ein entsprechendes (nicht-restaurierendes Protein) aus dem Wildtyp (SEQ ID NO: 33) ein Threonin (T) aufweist, an Position 108 der SEQ ID NO: 29, an welcher das die Restaurationseigenschaft vermittelnde mTERF Protein ein Glutaminsäure (E) und ein entsprechendes (nicht-restaurierendes Protein) aus dem Wildtyp (SEQ ID NO: 33) ein Asparaginsäure (D) aufweist, an Position 126 der SEQ ID NO: 29, an welcher das die Restaurationseigenschaft vermittelnde mTERF Protein ein Serin (S) und ein entsprechendes (nicht-restaurierendes Protein) aus dem Wildtyp (SEQ ID NO: 33) ein Alanin (A) aufweist, an Position 193 der SEQ ID NO: 29, an welcher das die Restaurationseigenschaft vermittelnde mTERF Protein ein Asparaginsäure (D) und ein entsprechendes (nicht-restaurierendes Protein) aus dem Wildtyp (SEQ ID NO: 33) ein Glycin (G) aufweist, an Position 213 der SEQ ID NO: 29, an welcher das die Restaurationseigenschaft vermittelnde mTERF Protein ein Glycin (G) und ein entsprechendes (nicht-restaurierendes Protein) aus dem Wildtyp (SEQ ID NO: 33) ein Glutaminsäure (E) aufweist, an Position 243 der SEQ ID NO: 29, an welcher das die Restaurationseigenschaft vermittelnde mTERF Protein ein Serin (S) und ein entsprechendes (nicht-restaurierendes Protein) aus dem Wildtyp (SEQ ID NO: 33) ein Cystein (C) aufweist, an Position 272 der SEQ ID NO: 29, an welcher das die Restaurationseigenschaft vermittelnde mTERF Protein ein Cystein (C) und ein entsprechendes (nicht-restaurierendes Protein) aus dem Wildtyp (SEQ ID NO: 33) ein Arginin (R) aufweist, an Position 276 der SEQ ID NO: 29, an welcher das die Restaurationseigenschaft vermittelnde mTERF Protein ein Alanin (A) und ein entsprechendes (nicht-restaurierendes Protein) aus dem Wildtyp (SEQ ID NO: 33) ein Threonin (T) aufweist, an Position 303 der SEQ ID NO: 29, an welcher das die Restaurationseigenschaft vermittelnde mTERF Protein ein Isoleucin (I) und ein entsprechendes (nicht-restaurierendes Protein) aus dem Wildtyp (SEQ ID NO: 33) ein Valin (V) aufweist, an Position 363 der SEQ ID NO: 29, an welcher das die Restaurationseigenschaft vermittelnde mTERF Protein ein Alanin (A) und ein entsprechendes (nicht-restaurierendes Protein) aus dem Wildtyp (SEQ ID NO: 33) ein Valin (V) aufweist, oder an Position 365 der SEQ ID NO: 29, an welcher das die Restaurationseigenschaft vermittelnde mTERF Protein ein Histidin (H) und ein entsprechendes (nicht-restaurierendes Protein) aus dem Wildtyp (SEQ ID NO: 33) ein Arginin (R) aufweist. In analoger Weise können auch potentielle Aminosäureaustausche aus Figur 10, die einen Vergleich der Wildtyp-Aminosäuresequenz des rfp1b (SEQ ID NO: 31) mit der die Restaurationseigenschaft vermittelnden Aminosäuresequenz aus IRAN9 (SEQ ID NO: 2) zeigt, abgeleitet werden. Weitere potentielle Mutationen als Modifikation auf DNA-Ebene (z.B. in Form von Nukleotidaustasuchen oder Insertionen/Deletionen können aus den Vergleichen der kodierenden Nukleotidsequenzen von rfp1a und rfp1b in den Figuren 7 und 8 in analoger Weise abgeleitet werden.

Ein weiteres Beispiel für eine Veränderung der Genetik ist die Deletion von Nukleotiden in der regulatorische Sequenz und/oder der endogenen DNA-Sequenz sowie die Addition von Nukleotiden in der regulatorischen Sequenz und/oder der endogenen DNA-Sequenz. Ein Beispiel zur Regulation von Genen durch Insertion von Nukleotiden durch Transposonmutagenese in Mais zeigt Das & Martienssen (Das, Lekha, and Robert Martienssen. "Site-selected transposon mutagenesis at the hcf106 locus in maize." The Plant Cell 7.3 (1995): 287-294.). Eine Veränderung der Epigenetik kann beispielsweise durch ein verändertes Methylierungsmuster der DNA erfolgen.

Dem Fachmann ist bekannt, wie eine Mutation im Sinne der Erfindung durch den Prozess einer Mutagenisierung im Schritt A) des Verfahrens zur Herstellung einer Pflanzenzelle/Pflanze erreicht werden kann. Die Mutagenisierung schließt hierbei sowohl die konventionelle Mutagenese als auch die ortspezifische Mutagenese beziehungsweise das ,Genome Editing' ein. Bei der konventionellen Mutagenese wird die Modifikation auf DNA-Ebene nicht gezielt herbeigeführt. Die Pflanzenzelle oder die Pflanze wird mutagenen Bedingungen wie z.B. TILLING durch UV-Licht-Bestrahlung oder den Einsatz chemischer Stoffe ausgesetzt (Till, Bradley J., et al. "Discovery of induced point mutations in maize genes by TILLING." BMC Plant Biology 4.1 (2004): 12.). Eine weitere Methode der Zufallsmutagenese ist die Mutagenese mit Hilfe eines Transposons.

Die ortspezifische Mutagenese ermöglicht die Einbringung von Modifikation auf DNA-Ebene zielgerichtet an vordefinierten Stellen der DNA. Hierfür können beispielsweise, TALENS (WO 2010/079430, WO 2011/072246), Meganukleasen (Silva, George, et al. "Meganucleases and other tools for targeted genome engineering: perspectives and challenges for gene therapy." Current gene therapy 11.1 (2011): 11.), Homing-Endonukleasen (Stoddard, Barry L. "Homing endonucleases: from microbial genetic invaders to reagents for targeted DNA modification." Structure 19.1 (2011): 7-15.), Zinkfingernukleasen (Lloyd, Alan, et al. "Targeted mutagenesis using zinc-finger nucleases in Arabidopsis." Proceedings of the National Academy of Sciences of the United States of America 102.6 (2005): 2232-2237.) oder ein CRISPR/Cas-System (Gaj, Thomas, Charles A. Gersbach, and Carlos F. Barbas. "ZFN, TALEN, and CRISPR/Cas-based methods for genome engineering." Trends in biotechnology 31.7 (2013): 397-405.) verwendet werden. Beispielsweise wird die Mutation an allen Kopien bzw. Allelen oder wo sinnvoll an allen Homologen der entsprechenden endogenen DNA-Sequenzen durchgeführt. Dies kann in Bezug auf einen diploiden Organismus wie beispielsweise *Secale cereale* oder *Hordeum vulgare* typischerweise mindestens zwei Veränderungen bedeuten.

Das Identifizieren einer Pflanze im Schritt B) kann beispielsweise mit Hilfe von molekularen Markern oder Sonden erfolgen. DNA-Sonden sind beispielsweise Primer oder Primerpaare, welche in einer PCR-Reaktion eingesetzt werden können. Beispielsweise können Tillingmutanten durch Sequenzierung des Zielgens in einer Tilling-Population oder weitere Methoden, die Fehlpaarungen in der DNA nachweisen, wie z.B. Schmelzpunktanalysen oder Einsatz fehlpaarungsspezifischer Nukleasen nachgewiesen beziehungsweise identifiziert werden. Vorliegende Erfindung schließt hierfür einsetzbare Primer/Primerpaare ebenfalls mit ein, wie z.B. Primer zum Nachweis von mTERF oder einer mutierten Form davon. Ferner können Mutanten, erzeugt mittels Transposons, durch Einsatz von Transposon-spezfischen Primern und Zielgen-spezifischen Primern in der PCR über die gesamte Population und anschließende Sequenzierung von PCR-Produkten nachgewiesen werden. Auch solche Primer sind von der vorliegenden Erfindung mit erfasst. Mutationsbedingte Änderung der Expressionsrate oder Expressionshöhe können beispielsweise mit RT-PCR in pflanzlichen Geweben bestimmt werden, eine mutationsbedingte Änderung der Stabilität beispielsweise durch Untersuchung von Ubiquitin-Bindestellen und Vorhersagen über Änderungen der Teritärstruktur. Weiterhin sind auch rekombinante Expression der Wildtyp-Proteine und der enstprechenden mutierten Proteine und biochemische Aktivitätstests geeignet. Dem Fachmann sind aus dem Stand der Technik weitere Mittel und Verfahren bekannt, welche er zum Identifizieren einer Pflanze oder Pflanzenzelle im Schritt B) verwenden kann.

Die vorliegende Erfindung betrifft auch molekulare Marker, welche die Anwesenheit oder Abwesenheit einer Mutation in der endogenen DNA-Sequenz oder in einer regulatorischen Sequenz der endogenen DNA-Sequenz nachweisen. Solche Marker basieren beispielsweise auf einem SNP und sind spezifisch für die Mutation (Beispiele: KASP oder TaqMan Marker). Geeignete SNP zur Markerentwicklung sind beispielsweise für *Secale cereale* dem Sequenzvergleich aus Figuren 7 und 8 zu entnehmen.

Die vorliegende Erfindung betrifft weiterhin auch eine Pflanze, welche mit dem vorstehenden Verfahren herstellbar ist oder hergestellt ist, oder ein Teil dieser Pflanze. Ebenso schließt die vorliegende Erfindung auch einen Nachkomme der Pflanze ein, welcher die mindestens eine Mutation und dadurch eine neu-vermittelte Restaurationseigenschaft für die Pollenfertilität für die Pampa cytoplasmatische männliche Sterilität (CMS) oder eine verbesserte Restaurationseigenschaft für die Pollenfertilität für die Pampa cytoplasmatische männliche Sterilität (CMS) im Vergleich zu einer nicht-mutierten Wildtyp-Pflanze, welche ansonsten isogen ist, und/oder eine neu-vermittelte Resistenz gegen ein Pathogen, vorzugsweise gegen einen Pilz, insbesondere gegen den Pilz Claviceps purpurea (Fr.), oder eine erhöhte Resistenz gegen ein Pathogen aufweist, vorzugsweise gegen einen Pilz, insbesondere gegen den Pilz Claviceps purpurea (Fr.) im Vergleich zu einer nicht-mutierten Wildtyp-Pflanze, welche ansonsten isogen ist, aufweist.

Ein weiterer Aspekt der vorliegenden Erfindung ist ein Verfahren zur Herstellung einer transgenen Pflanze, welche eine neu-vermittelte Restaurationseigenschaft für die Pollenfertilität für die Pampa cytoplasmatische männliche Sterilität (CMS) oder eine verbesserte Restaurationseigenschaft für die Pollenfertilität für die Pampa cytoplasmatische männliche Sterilität (CMS) im Vergleich zu einer nicht-mutierten Wildtyp-Pflanze, welche ansonsten isogen ist, und/oder eine neu-vermittelte Resistenz gegen ein Pathogen, vorzugsweise gegen einen Pilz, insbesondere gegen den Pilz Claviceps purpurea (Fr.), oder eine erhöhte Resistenz gegen ein Pathogen aufweist, vorzugsweise gegen einen Pilz, insbesondere gegen den Pilz Claviceps purpurea (Fr.) im Vergleich zu einer nicht-mutierten Wildtyp-Pflanze, welche ansonsten isogen ist, aufweist. Das Verfahren kann die folgenden Schritte umfassen: A) Bereitstellen des oben beschriebenen Nukleinsäuremoleküls, der Expressionskassette oder der rekombinanten DNA, oder Bereitstellen des oben beschriebenen Vektors, B) Transformieren, vorzugsweise stabil Transformieren, von mindestens einer Pflanzenzellen durch Einbringen des Nukleinsäuremoleküls, der Expressionskassette, der rekombinanten DNA oder des Vektors aus A), C) Regenerieren transgener Pflanzen aus der mindestens einen transformierten Pflanzenzellen aus B), und optional D) Identifizieren einer Pflanze, welche eine neu-vermittelte Restaurationseigenschaft für die Pollenfertilität für die Pampa cytoplasmatische männliche Sterilität (CMS) oder eine verbesserte Restaurationseigenschaft für die Pollenfertilität für die Pampa cytoplasmatische männliche Sterilität (CMS) im Vergleich zu einer nicht-mutierten Wildtyp-Pflanze, welche ansonsten isogen ist, und/oder eine neu-vermittelte Resistenz gegen ein Pathogen, vorzugsweise gegen einen Pilz, insbesondere gegen den Pilz Claviceps purpurea (Fr.), oder eine erhöhte Resistenz gegen ein Pathogen aufweist, vorzugsweise gegen einen Pilz, insbesondere gegen den Pilz Claviceps purpurea (Fr.) im Vergleich zu einer nicht-mutierten Wildtyp-Pflanze, welche ansonsten isogen ist, aufweist, aus C). Das Verfahren zur Herstellung der transgenen Pflanze schließt auch die Bereitstellung von zwei oder mehr der oben beschriebenen Nukleinsäuremoleküle, wahlweise auch unterschiedliche Ausgestaltungen der erfindungsgemäßen Nukleinsäuremoleküle und optional in einem oder mehreren Expressionskassetten oder Vektoren, und das Transformieren von Pflanzenzellen durch Einbringen der zwei oder mehr Nukleinsäuremoleküle ein.

Die vorliegende Erfindung betrifft weiterhin auch eine transgene Pflanze, welche mit genanntem Verfahren herstellbar ist oder hergestellt ist, oder einen Teil dieser Pflanze. Ebenso schließt die vorliegende Erfindung auch einen Nachkommen der transgenen Pflanze ein, welcher eine neu-vermittelte Restaurationseigenschaft für die Pollenfertilität für die Pampa cytoplasmatische männliche Sterilität (CMS) oder eine verbesserte Restaurationseigenschaft für die Pollenfertilität für die Pampa cytoplasmatische männliche Sterilität (CMS) im Vergleich zu einer nicht-mutierten Wildtyp-Pflanze, welche ansonsten isogen ist, und/oder eine neu-vermittelte Resistenz gegen ein Pathogen, vorzugsweise gegen einen Pilz, insbesondere gegen den Pilz Claviceps purpurea (Fr.), oder eine erhöhte Resistenz gegen ein Pathogen aufweist, vorzugsweise gegen einen Pilz, insbesondere gegen den Pilz Claviceps purpurea (Fr.) im Vergleich zu einer nicht-mutierten Wildtyp-Pflanze, welche ansonsten isogen ist, aufweist, ein.

In einem weiteren Aspekt betrifft die vorliegende Erfindung ein Verfahren zur Vermittlung oder Steigerung der Restaurationseigenschaft für die Pollenfertilität für die Pampa cytoplasmatische männliche Sterilität (CMS) in einer Pflanzenzelle oder einer Pflanze und/oder zur Vermittlung oder Steigerung der Resistenz gegen ein Pathogen, vorzugsweise gegen einen Pilz, insbesondere gegen den Pilz Claviceps purpurea (Fr.). Ein solches Verfahren kann die folgenden Schritte umfassen: A) Transformieren, vorzugsweise stabil Transformieren, von mindestens einer Pflanzenzellen durch Einbringen des oben beschriebenen erfindungsgemäßen Nukleinsäuremoleküls, der rekombinanten DNA oder der Expressionskassette der vorliegenden Erfindung, oder des oben beschriebenen Vektors der vorliegenden Erfindung, optional B) Regenerieren transgener Pflanzen aus der mindestens einen transformierten Pflanzenzellen aus A). Das Verfahren zur Herstellung der transgenen Pflanzenzelle/Pflanze schließt auch das Transformieren von zwei oder mehr der oben beschriebenen erfindungsgemäßen Nukleinsäuremoleküle, wahlweise auch unterschiedliche Ausgestaltungen der erfindungsgemäßen Nukleinsäuremoleküle und optional von einem oder mehreren Expressionskassetten oder Vektoren der vorliegenden Erfindung ein.

Darüberhinaus betrifft die vorliegende Erfindung die Verwendung der oben beschriebenen Pflanze, des oben beschriebenen Nachkommens oder der genannten transgenen Pflanze zur Herstellung einer erfindungsgemäßen Hybridpflanze oder einer erfindungsgemäßen transgenen Pflanze, vorzugsweise der Gattung *Secale* oder *Triticale,* vorzugsweise einer Pflanze der Art *Secale cereale,* deren Pollenfertilität für die Pampa CMS restauriert ist und/oder die eine erhöhte Resistenz gegen ein pilzlichen Pathogen aufweist, insbesondere gegen den Pilz *Claviceps purpurea* (Fr.).

Weiterhin können oben beschriebene Gegenstände wie Oligonukleotide, Nukleinsäuren, Expressionskassette, rekombinante DNA, Vektoren und Antikörper auch in der Herstellung der Pflanze oder der transgenen Pflanze dienlich sein. Somit umfasst die vorliegende Erfindung auch die Verwendung des oben beschriebenen Oligonukleotids, des Nukleinsäuremoleküls, der rekombinanten DNA, des Vektors oder des Antikörpers in der Herstellung einer erfindungsgemäßen Hybridpflanze oder einer erfindungsgemäßen transgenen Pflanze. In einer bevorzugten Ausführungsform ist die Hybridpflanze ausgewählt aus der Gattung *Secale* oder *Triticale* vorzugsweise einer Pflanze der Art *Secale cereale,* deren Pollenfertilität für die Pampa CMS restauriert ist und/oder die eine erhöhte Resistenz gegen ein pilzlichen Pathogen aufweist, insbesondere gegen den Pilz *Claviceps purpurea* (Fr.). Insbesondere die Oligonukleotide und Nukleinsäuren sowie rekombinante DNA, Vektoren und Antikörper können auch in der Herstellung einer transgenen Pflanze dienlich sein.

In einem weiteren Aspekt betrifft die vorliegende Erfindung die Verwendung eines Nukleinsäuremoleküls, das für ein mTERF Protein kodiert, oder des kodierten mTERF Proteins in einer Pflanze, insbesondere aus der Ordnung der Grasartigen (Poales), vorzugsweise der Familie der Süßgräser (Poaceae), zur Restauration einer cytoplasmatischen männlichen Sterilität (CMS), insbeosndere der Pampa CMS. Vorzugsweise erfolgt die Restauration durch Kreuzung der Pflanze enthaltend das Nukleinsäuremolekül als paternaler Elter mit einer zweiten Pflanze, bevorzugt von derselben Spezies, enthaltend das CMS Zytoplasma. Vorzugsweise handelt es sich bei dem Nukleinsäuremolekül um das oben beschriebene erfindungsgemäße Nukleinsäuremolekül, das in der Lage ist die Restaurationseigenschaft zu vermitteln, bzw handelt es sich beim dem mTERF Protein um das oben beschriebene erfindungsgemäße mTERF Protein.

Weitere Ausführungsformen und Vorteile der vorliegenden Erfindung gehen aus der folgenden ausführlichen Beschreibung, den Figuren und den Beispielen hervor.

Zunächst werden einige der in dieser Anmeldung verwendeten Begriffe nachfolgend näher erlautert:
Unter dem Begriff "Allel" wird eine von zwei oder mehreren verschiedenen Nukleotidsequenzen verstanden, die sich an einem bestimmten Genlocus auf einem Chromosom befinden. Ein erstes Allel findet sich auf einem Chromosom, ein zweites auf einem zweiten Chromosom an derselben Position.
Unterscheiden sich die beiden Allele liegen diese heterozygot vor, sind es dieselben Allele liegen sie homozygot vor. Verschiedene Allele eines Gens (Genallele) unterscheiden sich in mindestens einem SNP (single nucleotide polymorphisms). Je nach Kontext der Beschreibung meint ein Allel auch nur ein einzelnes SNP, das beispielsweise eine Unterscheidung zwischen Donor von RFP1 und
rekurrentem Elter erlaubt. Verschiedene Genallele können auch durch Verwendung von Markern detektiert werden. Solche Genallele an einem bestimmten Locus werden auch als Markerallele bezeichnet. Je nach Kontext der Beschreibung ist ein Markerlocus auch als ein Markerallel an einem bestimmten Locus zu verstehen.

Die Ausdrücke "Chromosomfragment", "Chromosomensegment" sowie Variationen der Begriffe wie "chromosomales Segment" oder "chromosomales Fragment" werden, wenn nicht anders angegeben, äquivalent verwendet und bezeichnen einen spezifischen chromosomalen DNA-Abschnitt eines bestimmten Chromosoms, der mindestens ein Gen umfasst. Ein integriertes Chromosomfragment stammt dabei aus einer Donor-Quelle. Im Sinne der Erfindung kann die sequenzielle Abfolge der Gene innerhalb eines integrierten Chromosomfragments derjenigen Abfolge entsprechen, wie sie im ursprünglichen Chromosomfragment der Donor-Quelle vorliegt. Ein Chromosomfragment oder ein Teil davon kann einen spezifischen "Haplotyp" darstellen, wobei das Chromosomfragment dann bestimmte SNPs aufweist, durch die der Haplotyp auch eindeutig spezifiziert ist und identifiziert werden kann.

Die Begriffe "distal" und "proximal" bezeichnen die Position eines chromosomalen Intervalls bzw. eines genetischen Abschnitts in Relation zu einem bestimmten Bezugsort (beispielsweise einem bestimmten Polynukleotid, einem anderen chromosomalen Intervall oder einem Gen) auf einem Gesamtchromosom, wobei distal bedeutet, dass das Intervall oder der Abschnitt auf der dem Chromosom-Centromer abgewandten Seite des Bezugsortes lokalisiert ist, und proximal bedeutet, dass das Intervall oder der Abschnitt auf der dem Chromosom-Centromer zugewandten Seite des Bezugsortes lokalisiert ist.

Unter den Begriffen "gekoppelt", "eng-gekoppelt" oder "eng-flankierend" wird verstanden, dass zwei Loci (beispielsweise zwei genetische Abschnitte oder zwei Marker (Markerloci bzw. Markerallele)) auf einer Genkarte weniger als als 2 cM, weniger als 1 cM, weniger als 0,5 cM, weniger als 0,2 cM, weniger als 0,1 cM, weniger als 0,05 cM oder weniger als 0,01 cM entfernt voneinander sind.

Der Begriff "Ertrag" im Sinne der vorliegenden Erfindung betrifft die Produktivität pro Flächeneinheit eines bestimmten Pflanzenproduktes mit kommerziellem Wert. Beispielsweise wird der Ertrag von Roggen gewöhnlich gemessen in metrischen Tonnen Samen oder Korn pro Hektar (ha) und Saison oder in metrischen Tonnen Trockenbiomasse pro Hektar (ha) und Saison. Sofern nicht ausdrücklich anders angegeben oder spezifiziert, kann der Ertrag die absolute Frisch- oder Trockenmasse, die relative Frisch- oder Trockenmasse, den Silageertrag (auch Total Dry Matter Yield genannt) oder den Körnerertrag betreffen. Der Ertrag wird durch genetische und umweltbedingte Faktoren beeinflusst und setzt sich prinzipiell aus zahlreichen agronomischen Eigenschaften zusammen, die auf genetischen Elementen beruhende Merkmale einer Pflanze darstellen und im Verlauf der Saison zum letztendlichen Ertrag beitragen. Zu diesen individuellen agronomischen Eigenschaften gehören beispielsweise vegetative Vitalität, Stresstoleranz, Krankheitsresistenz oder -toleranz, Herbizidresistenz, Bestockungsneigung, Blühzeitpunkt, Samenansatz, Kornzahl/Ähre, Tausendkornmasse, Standfestigkeit und Lagerneigung, Druschfähigkeit , etc.

Ein "funktionelles Fragment" eines Nukleinsäuremoleküls meint einen Abschnitt eines Nukleinsäuremoleküls, welcher die identische oder eine vergleichbare Funktionalität wie das Gesamtnukleinsäuremolekül, aus welchem das funktionelle Fragment stammt, aufweist. Als solches kann das funktionelle Fragment eine Nukleotidsequenz besitzen, welche über eine Länge von mindestens 50%, 55%, 60%, 65%, 70%, 75%, 80%, 85%, 90%, 92%, 94% 96%, 97%, 98% oder 99% identisch oder homolog mit dem Gesamtnukleinsäuremolekül ist. Ein "funktionelles Fragment" eines Proteins meint einen Abschnitt der Aminosäuresequenz eines Proteins, welcher die identische oder eine vergleichbare Funktionalität wie die Gesamtaminosäuresequenz des Proteins, aus welcher das funktionelle Fragment stammt, aufweist. Als solches kann das funktionelle Fragment eine Aminosäuresequenz besitzen, welche über eine Länge von mindestens 50%, 55%, 60%, 65%, 70%, 75%, 80%, 85%, 90%, 92%, 94% 96%, 97%, 98% oder 99% identisch oder homolog mit der Gesamtaminosäuresequenz des Proteins ist

Im Sinne der Erfindung wird unter einem "Homolog" ein Protein gleichen phylogenetischen Ursprungs verstanden, unter einem "Analog" ein Protein verstanden, welches die gleiche Funktion ausübt, jedoch einen anderen phylogenetischen Ursprung hat, und unter einem "Ortholog" ein Protein aus einer anderen Spezies verstanden, das die gleiche Funktion ausübt.

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d. h. mit diesem Basenpaarungen eingeht. Standardverfahren zur Hybridisierung sind beispielsweise in Sambrook et al., Molecular Cloning: A Laboratory Manual 3rd Ed. Cold Spring Harbor Laboratory Press. Cold Spring Harbor, NY, 2001 beschrieben. Vorzugsweise wird darunter verstanden, dass mindestens 60%, weiter bevorzugt mindestens 65%, 70%, 75%, 80% oder 85%, besonders bevorzugt 90%, 91%, 92%, 93%, 94%, 95%, 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. Ionenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85%, mindestens 90% oder mindestens 95% Sequenzidentität aufweisen. Stringente Hybridisierungsbedingungen sind beispielsweise: Hybridisieren in 4 x SSC bei 65°C und anschließendes mehrfaches Waschen in 0,1 x SSC bei 65°C für insgesamt etwa 1 Stunde. Der hier verwendete Begriff "stringente Hybridisierungsbedingungen" kann auch bedeuten: Hybridisieren bei 68°C in 0,25 M Natriumphosphat, pH 7,2, 7% SDS, 1 mM EDTA und 1% BSA für 16 Stunden und anschließendes zweimaliges Waschen mit 2 x SSC und 0,1% SDS bei 68°C. Bevorzugt findet eine Hybridisierung unter stringenten Bedingungen statt.

Der Begriff "Intervall" oder "chromosomales Intervall" meint einen durchgängigen linearen Abschnitt auf einer genomischen DNA, welcher in einem einzelnen Chromosom in planta oder auf einem Chromosomfragment vorliegt und welcher gewöhnlicherweise durch die Angabe von zwei Markern, welche die Endpunkte des Intervalls zur distalen und proximalen Seite hin darstellen, definiert ist. Dabei können die Marker, die das Intervall endständig definieren, selbst auch Teil des Intervalls sein. Weiterhin können zwei unterschiedliche Intervalle auch überlappen. In der Beschreibung wird ein Intervall durch die Angabe "zwischen Marker A und Marker B" spezifiziert. Ein endständiger Marker eines Intervalls kann auch in einer definierten Markerregion zu einer Seite des Intervalls lokalisiert sein. Eine Markerregion wird dann durch die Angabe von zwei flankierenden Markern definiert und stellt einen chromosomalen Abschnitt dar, auf dem neben den flankierenden Markern noch weitere Marker liegen können. Flankierende Marker bestimmen die Endpunkte einer Markerregion und sind selbst noch Teil der Markerregion. Sind beide endständigen Marker eines Intervalls Marker in unterschiedlichen Markerregionen zu beiden Seiten eines Intervalls, wird in der Beschreibung ein Intervall durch die Angabe "zwischen einem Marker in einer Markerregion X, welche durch die Marker C und D flankiert ist, und einem Marker in einer Markerregion Y, welche durch die Marker E und F flankiert ist" spezifiziert.

Unter dem Begriff "Introgression" wird im Zusammenhang mit der vorliegenden Erfindung die Übertragung von mindestens einem gewünschten Genallel auf einem genetischen Locus von einem genetischen Hintergrund in einen anderen. Beispielsweise kann eine Introgression eines gewünschten Genallels an einen spezifischen Locus auf einen Nachkommen durch sexuelle Kreuzung zwischen zwei Eltern derselben Spezies übertragen werden. Alternativ kann zum Beispiel die Übertragung eines Genallels auch durch Rekombination zwischen zwei Donorgenomen in einem fusionierten Protoplasten auftreten, wobei mindestens ein Donor-Protoplast das gewünschte Genallel in seinem Genom trägt. In jedem Fall können die Nachkommen, die dann das gewünschte Genallel umfassen, anschließend wiederholt mit einer Linie, die einen vorzüglichen genetischen Hintergrund aufweist, rückgekreuzt und auf das gewünschte Genallel selektiert werden. Das Ergebnis ist eine Fixierung des gewünschten Genallels in einem ausgewählten genetischen Hintergrund.

Als "Linkage Drag" bezeichnet man im Allgemeinen die phänotypische Expression von unerwünschten Donorgenen, die in derselben genomischen Region wie der Ziel-QTL (Quantitative Trait Locus) residieren und deshalb eng mit diesem gekoppelt sind. Hierunter fällt beispielsweise die Beobachtung, dass durch Introgression des Chromosomenfragmentes, das das/die Restorgen(e) trägt, negativ wirkende Donorgene in die Introgressionslinie übernommen werden, so dass diese dann für bestimmte agronomische Merkmale weniger leistet als die ursprüngliche Rezipientenlinie.

Für denFall der Restauration der männlichen Fertilität manifestieren *Rfp1*-tragende Introgressionssegmente üblicherweise Linkage Drag in Form von nachteiligen Effekten auf den Ertrag, d.h. Kornertrag und andere Eigenschaften wie Wuchshöhe, Einkörnung (Körner/Ähre), und Tausendkornmasse; siehe bspw. Hackauf et al., J. Kulturpfl. 61 (2009), 15-20; Hackauf et al., Molecular Breeding 30 (2012), 1507-1518.

Das Merkmal, dass in einer erfindungsgemäßenen Hybridpflanze ein ansonsten mit der Restaurationseigenschaft gekoppelter Linkage Drag reduziert ist oder (vollständig) unterbleibt, bezieht sich auf den ansonsten bei einer Hybridpflanze (Kontrollpflanze) auftretenden Linkage Drag. Die Kontrollpflanze weist in ihrem Genom ein chromosomales Segment auf dem Chromosom 4R mit mindestens einem Intervallvon Markerlocus tc256739 bis Markerlocus tc176835 von einem Donor ausgewählt aus der Gruppe bestehend aus IRAN IX, Pico Gentario und Altevogt 14160 auf. Das Analoge gilt für das Intervall Xp15/55-Xscxx04 Segment aus IRAN IX; siehe Hackauf et al., Molecular Breeding 30 (2012), 1507-1518. Wenn nicht anders angegeben, wird unter dem Merkmal, dass ein ansonsten mit der Restaurationseigenschaft gekoppelter Linkage Drag reduziert ist oder vollständig aufgehoben, zusätzlich oder alternativ auch die Verbesserung einer Eigenschaft der erfindungsgemäßen Hybridpflanze gegenüber einer Kontrollpflanze verstanden. So zum Beispiel eine erhöhte Pollenschüttung, welche zur Minimierung des Mutterkornbefalls führt.

Ein "Locus" ist eine Position auf einem Chromosom, wo sich ein oder mehrere Gene bzw. ein oder mehrere Genallele befinden, welche ein agronomisches Merkmal verursachen oder beeinflussen. Insbesondere meint Locus hier den *Rfp1*-Locus, welcher die Pollenfertilität für die Pampa cytoplasmatische männliche Sterilität (CMS) restauriert.

Der Begriff "Marker" bezeichnet eine Nukleotidsequenz, die als Bezugs- oder Orientierungspunkt verwendet wird. Ein Marker zum Erkennen eines Rekombinationsereignisses sollte geeignet sein, Unterschiede oder Polymorphismen innerhalb einer pflanzlichen Population zu überwachen. Für Marker finden sich diese Unterschiede auf DNA-Ebene und sind beispielsweise Polynukleotidsequenzunterschiede wie zum Beispiel SSRs (simple sequence repeats), RFLPs (restriction fragment length polymorphisms), FLPs (fragment length polymorphisms) oder SNPs (single nucleotide polymorphisms). Marker können von genomischen oder exprimierten Nukleinsäuren wie beispielsweise gespleißter RNA, cDNA oder ESTs abgeleitet sein und können sich auch auf Nukleinsäuren beziehen, die als Sonden oder Primer-Paare eingesetzt werden und als solche geeignet sind, ein Sequenzfragment unter Verwendung PCR-basierter Verfahren zu amplifizieren. Marker, welche genetische Polymorphismen zwischen Angehörigen einer Population erkennen, können mittels etablierter Verfahren aus dem Stand der Technik nachgewiesen werden (An Introduction to Genetic Analysis. 7th Edition, Griffiths, Miller, Suzuki et al., 2000). Dazu gehoren z. B.: DNA-Sequenzierung, PCR-basierte, sequenzspezifische Amplifikation, Nachweis von RFLPs, Nachweis von Polynukleotidpolymorphismen mittels Allel-spezifischer Hybridisierung (ASH), Detektion von SSRs, SNPs oder RFLPs. Darüber hinaus sind auch Verfahren zur Detektion von ESTs (expressed sequence tags) und RAPD (randomly amplified polymorphic DNA) bekannt. Je nach Kontext kann der Begriff Marker in der Beschreibung auch eine spezifische Chromosomposition in dem Genom einer Spezies, wo ein spezifischer Marker (z.B. SNP) gefunden werden kann, meinen. Eine solche Marker-Position kann genutzt werden, um die An- oder Abwesenheit eines gekoppelten Locus zu verfolgen, z.B. ein gekoppelter Locus, der zur Expression eines bestimmten phänotypischen Merkmals beitragt (z.B. *Rfp1* oder Linkage Drag). Beispielsweise kann der Marker-Locus auch genutzt werden, um die Segregation von Allelen an einem Locus (QTL oder Einzelgen), die mit der Marker-Position genetisch oder physikalisch eng-gekoppelt sind, zu beobachten.

"Operativ verknüpft" meint verbunden in einem gemeinsamen Nukleinsäuremolekül, dergestalt, dass die verbundenen Elemente so zueinander positioniert und/oder orientiert sind, dass eine Transkription des Nukleinsäuremoleküls stattfinden kann. Eine DNA, welche operativ mit einem Promotor verknüpft ist, steht unter der transkriptionellen Kontrolle dieses Promotors.

Pflanzliche "Organe" meinen beispielsweise Blätter, Sprossachse, Stamm, Wurzeln, vegetative Knospen, Meristeme, Embryos, Antheren, Ovula, Samen oder Früchte, insbesondere Samenkörner. Der Begriff "Pflanzenteil" bzw. "Pflanzenteile" beinhaltet, ist jedoch nicht beschränkt auf, die Sprossachse bzw. den Halm, Blätter, Blüten, Blütenstände, Wurzeln, Früchte und Samen sowie die Pollen. Pflanzliche "Teile" meinen ferner einen Zusammenschluss mehrerer Organe, z.B. eine Blüte oder ein Samen, oder einen Teil eines Organs, z.B. einen Querschnitt aus der Sprossachse. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe sowie das Bildungsgewebe, Grundgewebe (das sogenannte Parenchym), Leitgewebe, Festigungsgewebe und das Deckgewebe (die sogenannte Epidermis). Das Gewebe wird durch diese Auflistung jedoch nicht beschränkt. Unter pflanzlichen "Zellen" sind beispielsweise isolierte Zellen mit einer Zellwand oder Aggregate davon oder Protoplasten zu verstehen.

Eine "Pflanze" im Sinne der Erfindung kann, sofern nicht anders angegeben, jeder dikotyledonen, monokotyledonen und gymnospermen Spezies entstammen. Vorzugsweise sind Pflanzen monokotyl und sind in Landwirtschaft und Gartenbau oder zur Erzeugung von Bioenergie (Bioethanol, Biogas etc.) interessant. Hierzu zählen beispielhaft Gossypium sp., Zea mays, Brachypodium distachyon, Triticum sp., Hordeum vulgare, Oryza sativa, Sorghum sp., Musa sp., Saccharum officinarum, Secale cereale, Avena sp., Rasen- und Futtergras. Eine erfindungsgemäße Pflanze ist vorzugsweise eine Pflanze der Gattung Secale, insbesondere der Spezies Roggen (Secale cereale).

Der Ausdruck "Resistenz" oder "resistent" gegenüber einem Pathogen ist als die Widerstandsfähigkeit oder Abwehrkraft einer Pflanze oder pflanzlichen Zelle gegenüber den schädlichen Einflüsse des Pathogens zu verstehen und erstreckt sich von einer Verzögerung in der Krankheitsentwicklung bis hin zu einer kompletten Unterdrückung der Krankheitsentwicklung. Zum Beispiel handelt es sich bei der Abwehrkraft von Rfpl-tragenden Hybriden gegenüber Mutterkorn um eine Resistenz basierend auf einem "escape"-Mechanismus: Sporen des Pilzes wird der Zugang zum Gynäceum durch die sich schnell schliessenden Spelzen nach Befruchtung durch den Pollen mechanisch verwehrt. Eine vermittelte Resistenz kann eine neu-erworbene Resistenz oder die Erhöhung einer bereits existierenden partiellen Resistenz sein. Im Zusammenhang mit der vorliegenden Erfindung ist eine Pflanze/Pflanzenzelle resistent oder besitzt eine Resistenz gegenüber dem Pathogen Mutterkorn, d.h. eine Hybridpflanze, die eine erhöhte Resistenz gegen ein Pathogen aufweist, vorzugsweise gegen einen Pilz, insbesondere gegen den Pilz Claviceps purpurea (Fr.).

Unter "Getreidepflanzen" werden insbesondere monokotyle Pflanzen verstanden, die zur Ordnung Grasartige (Poales), bevorzugt zur Familie der Süßgräser (Poaceae) gehören. Beispiele hierfür sind die Pflanzen, die zu den Gattungen Avena (Hafer), Triticum (Weizen), Secale (Roggen), Oryza (Reis), Panicum, Pennisetum, Setaria, Sorghum (Hirse), Zea (Mais) etc. gehören, bevorzugt ist Hordeum (Gerste). Besonders bevorzugt ist Secale (Roggen), d.h. eine Pflanze Secale cereale, S. africanum, S. ancestrale, S. dalmaticum, S. kuprijanovii, S. montanum, S. silvestre, S.vavilovii.

Eine "Transgene Pflanze" bezieht sich auf eine Pflanze, in deren Genom mindestens ein Polynukleotid, vorzugsweise ein heterologes Polynukleotid, integriert ist. Bevorzugt ist das Polynukleotid stabil integriert, was bedeutet, dass das integrierte Polynukleotid in der Pflanze stabil erhalten bleibt, exprimiert wird und auch stabil an die Nachkommen vererbt werden kann. Das stabile Einbringen eines Polynukleotids in das Genom einer Pflanze schließt auch die Integration in das Genom einer Pflanze der vorhergehenden Parentalgeneration mit ein, wobei das Polynukleotid stabil weitervererbt werden kann. Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

Unter den Begriffen "ertragsmindernder Effekt" wird die phänotypische Expression einer DNA-Sequenz verstanden, die mit dem Zielgen, hier dem Restorergen, gekoppelt bzw. eng-gekoppelt ist und daher co-segregiert. Es handelt sich dabei um ein bei Rückkreuzungsprogrammen mit exotischen Donoren häufig auftretendes Problem der gemeinsamen Vererbung von erwünschten und züchterisch unerwünschten Genen, das beispielsweise von Brinkmann et al., Crop Sci. 17 (1977), 165-168 und Tanksley et al., Bio/Technology 7, (1989) 257-264 beschrieben wurde. Dieser Komplex aus Restorergen und weiteren unerwünschten Genen, die teilweise ertragsmindernd sind, wird bislang immer vollständig oder zum Teil mit in das Zuchtmaterial übertragen, wodurch die Introgressionslinien beispielsweise neben der vorteilhaften Restaurationseigenschaft zusätzlich negative Merkmale enthalten, welche beispielsweise je nach Standort eine deutliche Ertragsminderung bewirken. Entsprechend ist der Linkage Drag hier vorzugsweise ein negativer Ertrags-Effekt, der mit effizienter Restaurationsleistung verbunden ist.

Eine Reduzierung oder Verminderung des Linkage Drag liegt vor, wenn sich seine negativen phänotypischen Eigenschaften gegenüber derKontrollpflanze nur zu 0 bis 75% ausprägen , was einer Reduzierung von 25-100% entspricht. In einer bevorzugten Ausführungsform beträgt die Reduzierung 50-100 % oder 75-100%. In einer besonders bevorzugten Ausführungsform sind die negativen Eigenschaften, die mit dem Linkage Drag verbunden sind, fast vollständig oder vollständig aufgehoben und die Verminderung des Linkage Drag beträgt zwischen 90 bis 100%. Eine Reduzierung oder Verminderung des Linkage Drag insbesondere für Hybridpflanzen kann auch der Linkage Drag-Effekt auf Ertrag weniger als 7 dt/ha (Doppelzentner pro Hektar), weniger als 6,5 dt/ha oder weniger als 6 dt/ha, bevorzugt weniger als 5,5 dt/ha, weniger als 5 dt/ha, weniger als 4,5 dt/ha oder weniger als 4 dt/ha, oder ganz besonders weniger als 3,5 dt/ha, weniger als 3 dt/ha, weniger als 2,5 dt/ha oder weniger als 2 dt/ha im Vergleich zu einer entsprechenden nahisogenen Pflanzen bzw. Hybridpflanze, welche das erfindungsgemäße chromosomale Segment oder das erfindungsgemäße Nukleinsäuremolekül nicht aufweist, bedeuten. Zur Quantifizierung des Linkage Drags kann eine Standardisierung des Linkage Drag Effektes als Prozent der Leistung des NIB-E Partners wie weiter unten in Beispiele 1 und 2 beschrieben.

Der Begriff "Vektor" oder "Vektorsystem", wie hier in Zusammenhang mit Genome Editing verwendet, bezieht sich auf ein Transportmittel, um ein rekombinantes Konstrukt, umfassend Nukleinsäuren oder auch Polypeptide sowie gegebenenfalls weitere Sequenzen wie regulatorische Sequenzen oder Lokalisationssequenzen, direkt oder indirekt in eine gewünschte Zielzelle bzw. pflanzliche Zielstruktur in das erwünschte zelluläre Kompartiment einbringen zu können. Die direkte Einbringung erfolgt direkt in eine pflanzliche Zielzelle bzw. pflanzliche Zielstruktur, welche Nukleinsäuren enthält, die gemäß der vorliegenden Offenbarung gezielt verändert werden sollen. Die indirekte Einbringung umfasst die Einbringung in eine Struktur, z.B. Zellen von Blätter oder weitere pflanzliche Organe und Gewebe, welche zwar nicht direkt die pflanzliche Zielzelle von Interesse umfassen, wodurch jedoch die systemische Ausbreitung und Weiterleitung des Vektors, umfassend ein rekombinantes Konstrukt gemäß der vorliegenden Offenbarung, in die pflanzliche Zielstruktur, z.B. meristematische Gewebe oder Zellen oder Stammzellen, gewährleistet wird. Der Begriff Vektor umfasst im Kontext der Transfektion von Aminosäuresequenzen geeignete Agenzien zur Peptid- oder Proteintransfektion wie z.B. ionische Lipidmischungen oder Agenzien, die zur Transfektion einer Nukleinsäure geeignet sind, wie z.B. Trägermaterialen, durch welche Nukleinsäure- und Aminosäuresequenzen mittels Partikel-Beschuss in eine Zelle eingeführt werden können, wie z.B. Gold- und Wolframpartikel. Ferner umfasst dieser Begriff auch virale Vektoren, d.h. modifizierte Viren, wie zum Beispiel solche, welche aus einem der folgenden Viren stammen: Barley Stripe Mosaic Virus (BSMV), Brome Mosaic virus (BMV), Maize yellow dwarf virus (MYDV) und bakterielle Vektoren, wie zum Beispiel *Agrobacterium* spp., wie zum Beispiel *Agrobacterium tumefaciens.* Schließlich umfasst der Begriff auch geeignete Transportmittel zur Einführung von linearen Nukleinsäuren (einzel- oder doppelsträngig) in eine Zielzelle. Dem Fachmann auf dem Gebiet sind weitere Sequenzen bekannt, über welche ein Vektor verfügen muss, um in einer erwünschten Zielzelle funktional zu sein. Auch die gängige Herstellung, Aufarbeitung und Anwendung derartiger Vektoren ist dem Fachmann auf dem Gebiet bekannt.

Der Begriff "rekombinantes Konstrukt" wie hierin in Zusammenhang mit Genome Editing verwendet, bezieht sich auf ein Konstrukt, umfassend unter anderen Plasmide oder Plasmidvektoren, Cosmide, künstliche Hefe- oder bakterielle Chromosomen (YACs und BACs), Phagemide, Bakteriophagenvektoren, eine Expressionskassette, einzelsträngige oder lineare Nukleinsäuresequenzen oder Aminosäuresequenzen, und virale Vektoren, d.h. modifizierte Viren, welche in eine Zielzelle gemäß der vorliegenden Offenbarung eingebracht werden können. Ein erfindungsgemäßes rekombinantes Konstrukt kann Genome Editing Werkzeuge oder Teile davon umfassen. Beispielsweise umfassen CRISPR/Cas Werkzeuge oder Teile davon mindestens eine gRNA oder mindestens eine Cas-Nuklease Variante und/oder mindestens eine weitere Effektor-Domäne entweder in der Form einer Nukleinsäure oder einer Aminosäuresequenz. TALENs Werkzeuge oder Teile davon umfassen beispielsweise mindestens eine TAL-Effektor-Domäne und/oder mindestens Nuklease-Variante, vorzugsweise eine Endonuklease des Typ II wie z. B. FokI. Ferner kann das rekombinante Konstrukt regulatorische Sequenzen und/oder Lokalisationssequenzen umfassen. Das rekombinante Konstrukt kann etwa in einen Plasmidvektor integriert vorliegen und/oder isoliert von einem Plasmidvektor vorliegen, z.B. in der Form einer Polypeptidsequenz oder einer nicht-Plasmidvektor verknüpften einzel- oder doppelsträngigen Nukleinsäure vorliegen. Nach Einbringung liegt das Konstrukt introchromosomal oder vorzugsweise extrachromosomal und nicht in das Genom integriert und gewöhnlich in der Form einer doppelsträngigen oder einzelsträngigen DNA, einer doppelsträngigen oder einzelsträngigen RNA oder eines Polypeptids vor.

Ausgestaltungen und Ausführungsformen der vorliegenden Erfindung werden in exemplarischer Weise mit Bezug auf die angehängten Figuren und Sequenzen beschrieben:
- Fig. 1:: Genetische und physikalische Karte des *Rfp1* Locus. A) Hochauflösende genetische Karte des *Rfp1* Locus auf dem langen Arm von Roggenchromosom 4R. Die Zahlen unterhalb der obersten horizontalen Linie beschreiben die Anzahl an beobachteten Rekombinationsereignissen zwischen den betreffenden Markern unter 4563 untersuchten Einzelpflanzen. Informationen zu den Markerkodierungen sind in Tabelle 2 der Anlage aufgeführt. B) *Rfp1* überspannendes Contig aus BAC-Klonen der Bibliothek Sce-B-R05104. C) Vorhergesagte Gene am *Rfp1* Locus. Die hervorgehobenen Boxen stellen Exons funktioneller Gene dar oder Genfragmente, Pseudogene oder mutierte Gene dar. Die Orientierung der Gene wird durch horizontale Pfeile angezeigt. Die vertikale Linie im mTERF-Gen 175019_c7 beschreibt ein vorzeitiges Stop-Codon in der Gensequenz. Die Abkürzungen F und C kennzeichnen, dass für den betreffenden Marker ein dominanter, für den Fertilität (F) verleihenden Restorergenotypen spezifischer bzw. ein kodominanter (C) Erbgang beobachtet worden ist.
- Fig. 2:: Kartierung funktioneller Restorergene am *Rfp1* Locus. Mit Hilfe molekularer Selektionsmarker wurden in 2 exemplarischen Versuchsserien rekombinante Einzelpflanzen mit unterschiedlich langen Donorchromosomensegmenten (D) im genetischen Hintergrund einer Pollenelterlinie (E) identifiziert. Die Expression der funktionellen Restorergene *Rfp1a* und *Rfp1b* wurde in Testkreuzungsnachkommenschaften jeder rekombinanten Pflanze mit dem hochdiagnostischen männlich-sterilen Testergenotyp Lo6-P(SR) bestimmt. Tabelle 2 gibt den Marker-Haplotyp des NIB-Partners D wieder, der das Donor-Introgressionssegment trägt. Δ_{E-D}: Differenz zwischen Testkreuzungsmittelwerten von NIB-Partnern, welche jeweils homozygot das Eliteallel (E) bzw. das Donorallel (D) tragen. Diese Differenz im Mittel über 7 Standorte bestimmt den Linkage Drag Effekt für Korn- Ertrag absolut in dt/ha und in Prozent des NIB-Partners E. LSD5%: Grenzdifferenz bei 5% Irrtumswahrscheinlichkeit.

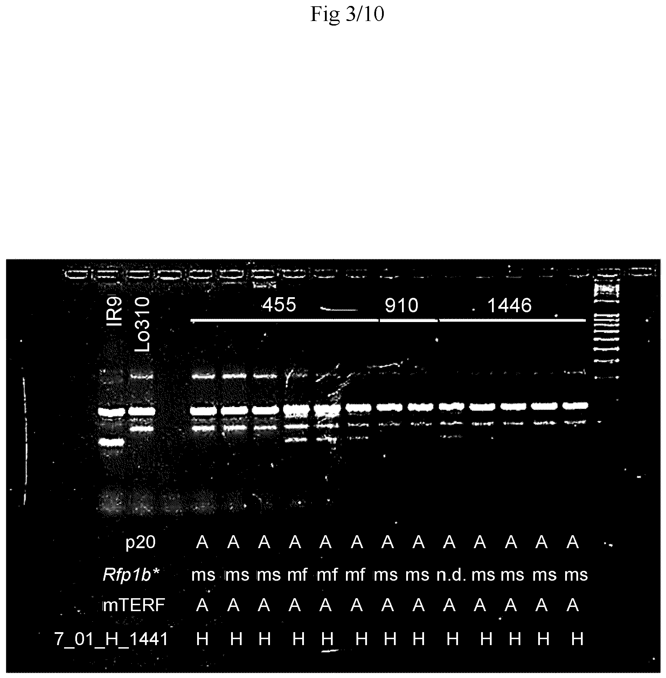

- Fig. 3:: Kartierung des Restorergens *Rfp1b.* Unter 13, zwischen den Markern P20 und 7_01_H_1441 rekombinanten Pflanzen lässt sich am Markerlocus 72F13_c2_mTERF das Allel des Donorgenotyps IR9 zweifelsfrei in 4 Pflanzen nachweisen. Der *Rfp1* b-Phänotyp wurde in Testkreuzungsnachkommenschaften der rekombinanten Genotypen mit dem CMS-Tester Lo6-P(SR) erfasst und steht in perfekter Übereinstimmung mit den Markergenotypen des durch 72F13_c2_mTERF abgebildeten mitochondrialen Transkriptions tERminations Faktors (mTERF) [A= homozygoter Träger des Elite-Allel; H= heterozygoter Träger des Elite- bzw. Donor-Allels; Rfp1b*= Rfp1- bzw. Elite-Phänotypen wurden anhand des Pollenschüttungsvermögens von jeweils 15 einzelnen Testkreuzungsnachkommen des rekombinanten Genotyps und des hochdiagnostischen Testers von Lo6-P(SR) erfasst.]

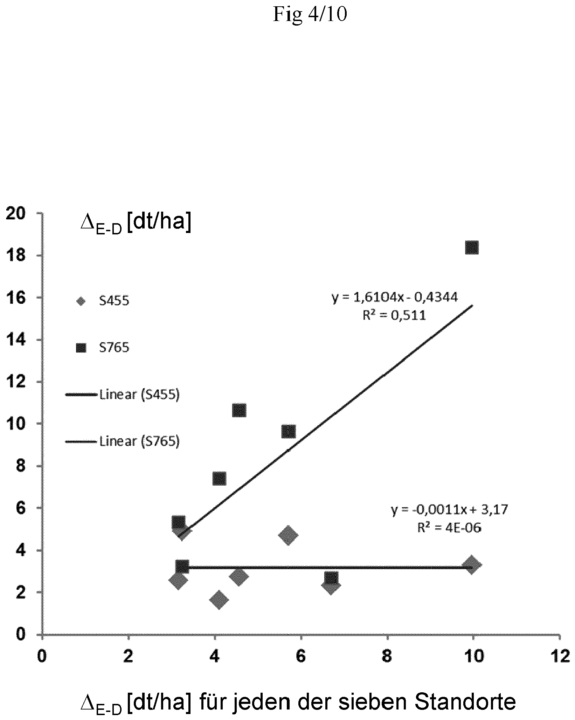

- Fig. 4:: zeigt den Linkage Drag-Effekt für Kornertrag (Δ_{E-D}) des Introgressionssegments 455 und 765 (y-Achse), aufgetragen gegen den mittleren Linkage Drag-Effekt für jeden der sieben getesteten Standorte (x-Achse). Die Rekombinante mit dem kurzen Introgressionssegment 455 zeigt einen geringen, die Rekombinante 765 mit einem langen Introgressionssegment zeigt einen großen Linkage Drag-Effekt. Aus den experimentellen Daten wird weiterhin deutlich, dass die Linkage Drag-Effekte sich für die sieben Umwelten stark unterscheiden. Witterungsdaten legen nahe, dass hierfür insbesondere Stressbedingungen während der Schossphase verantwortlich sind.

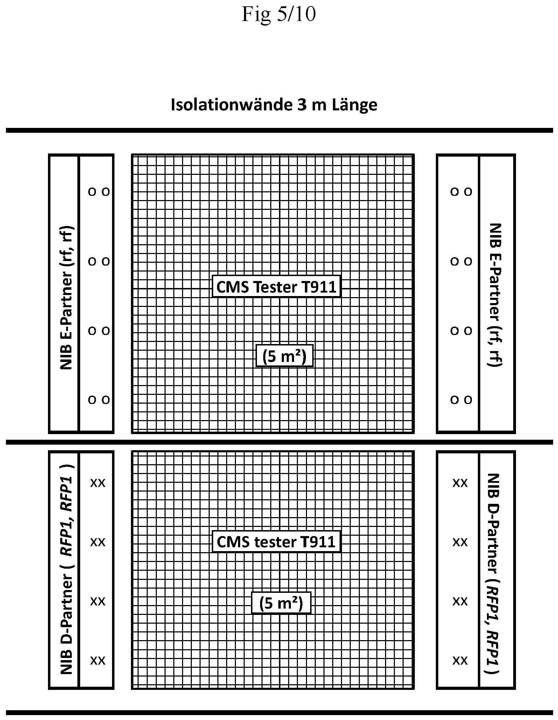

- Fig. 5:: Produktion von Testkreuzungssaatgut mit Near-Isogenic Bulk Partnern als Pollenelter and CMS ,single cross'-Tester T911 als weiblicher Elter. Gezeigt ist die Verwendung von NIB-Partnern (NIB-Paaren) in Isolationsparzellen, die zur Saatgutproduktion von Testkreuzungssaatgut dienen. Hierbei bestäuben NIB-Partner einen CMS ,single cross'-Tester, welcher den entgegengesetzten heterotischen Pool repräsentiert. Saatgut, das auf den CMS Testern geerntet wird, wird dann in Feldexperimenten mit multiplen Umwelten ausgesät, um den Linkage Drag-Effekt phänotypisch zu bestimmen.

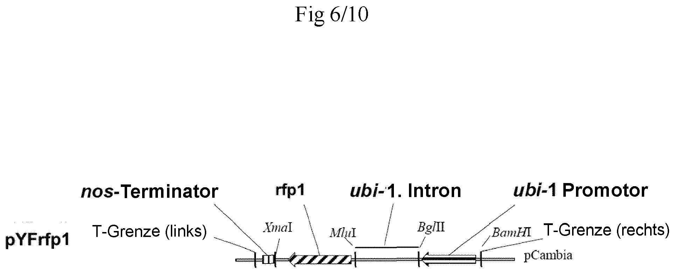

- Fig. 6:: Expressionskassette im Vektor pYFrfp1 beinhaltend das Restaurationsgen *rfp1b* (SEQ ID NO: 1) unter Kontrolle des Ubiquitin-Promotors aus Mais mit dem ersten Intron und des nos-Terminators.
- Fig. 7:: Vergleich der Nukleotidsequenz des Wildtyp-rfpla Gens (SEQ ID NO: 32) mit Nukleotidsequenz des rfp1a Gens aus IRAN9 (SEQ ID NO: 28).
- Fig. 8:: Vergleich der Nukleotidsequenz des Wildtyp-rfplb Gens (SEQ ID NO: 30) mit Nukleotidsequenz des rfp1b Gens aus IRAN9 (SEQ ID NO: 1).
- Fig. 9:: Vergleich der Amonsäuresequenz des Wildtyp-rfpla Proteins (SEQ ID NO: 33) mit Aminosäuresequenz des rfp1a Proteins aus IRAN9 (SEQ ID NO: 29).
- Fig. 10:: Vergleich der Amonsäuresequenz des Wildtyp-rfpla Proteins (SEQ ID NO: 31) mit Aminosäuresequenz des rfp1a Proteins aus IRAN9 (SEQ ID NO: 2).

Die folgenden Beispiele erläutern die Erfindung, ohne jedoch den Gegenstand der Erfindung einzuschränken. Sofern nicht anders angegeben, wurden Standardmethoden verwendet.

### BEISPIELE

### Beispiel 1: Beispielhafte 'Near Isogenic Bulks'-Entwicklung der Roggenlinie 455 im Lo310-Hintergrund

Wie in Figur 5 dargestellt, wurden für alle rekombinanten Genotypen NIB D- und E-Partner hergestellt, indem Bulks von jeweils mehr als 100 BC₆S₁ Pflanzen, welche homozygote Träger oder Nichtträger des Rfp1 sind, auf den single cross CMS-Tester T911 ausgekreuzt. Aufgrund angrenzender Isolationswände wurde sichergestellt, dass keine Fremdbestäubung stattfand. Das so erzeugte Testkreuzungssaatgut wurde dann für Feldversuche in multiplen Umwelten verwendet. Testkreuzungspflanzen wurden verifiziert auf korektes Pedigree durch (i) nachfolgende Markeranalyse und (ii) Bewertung der Pollenschüttung in den Feldversuchen. Alle bewerteten Testkreuzungspflanzen, welche von den NIB D-Partner generiert wurden, zeigten volle Pollenschüttung, während solche, die von den E-Partnern abstammen, eine sehr deutlich reduzierte und nur teilweise restaurierte männliche Fertilität zeigten.

### Beispiel 2: Feldversuche

Die Versuche zur Ertragsevaluierung wurden an Standorten mit unterschiedlichen Umweltbedingungen durchgeführt. So beispielsweise im Jahr 2012 an sieben Standorten in Deutschland (D) und Polen (PL). Wie der Tabelle 1 zu entnehmen ist, wurden die Standorte so gewählt, dass sie den landwirtschaftlichen Praxisbedingungen in Zentraleuropa mit zudem unterschiedlichen Stressbedingungen (Trockenstress und Stickstoffmangel) gerecht zu werden. Im Niedrig-Stickstoff Regime wurde Stickstoff in Mengen weit unterhalb der üblichen Gaben angewendet. In einem nicht-bewässerten Versuch stellte der natürliche Niederschlag die einzige Wasserquelle dar, während in bewässerten Versuchen eine zusätzliche Wassermenge von etwa 25 mm pro Woche appliziert wurde. Somit war es möglich, Effekte der Rfp1-Introgressionssegmente unter sehr unterschiedlichen Umwelten zu messen. Die Ergebnisse wurden dann (1) zur Bestimmung des Introgressionssegment-spezifischen Linkage Drag-Effekts, (2) zur Identifikation von Introgressionssegnmenten mit hoher Umweltstabilität und (3) zur Identifikation von diagnostischen Umwelten, welche den Linkage Drag in einem höheren Ausmaß sichtbar machen, genutzt.

Tabelle 1: Beschreibung der Versuchsstandorte und der angewandten Behandlungen im Jahr 2012 (BEK=Bekedorf (Niedersachsen); KON=Kondratowice (Niederschlesien); BBG=Bernburg (Sachsen-Anhalt); KO2 und KO3= Bergen (Niedersachsen); PET_I und PET_N=Petkus mit Bewässerung (I) und Stickstoffvarianten (N) (Brandenburg); Bodenpunkte: Index, der die Qualität einer Ackerfläche bemisst. Die Skala möglicher Werte reicht von 1 (sehr schlecht) bis 100 (sehr gut).)

| Standort | Land | Bodenpunkte | Langfristmittelwert Niederschlag [mm] | Agronomisches Regime |
|---|---|---|---|---|
| BEK | D | 51 | 769 | |
| KON | PL | 55 | 581 | lokalen agrikulturelle Praxis |
| BBG | D | 93 | 469 | |
| KO2 | D | 43 | 769 | niedrig Stickstoff |
| KO3 | D | 43 | 769 | nicht bewässert |
| PET_I | D | 28 | 636 | bewässert |
| PET_N | D | 28 | 636 | nicht bewässert |

Für alle Umwelten wurde ein 'Split Plot'-Versuchsdesign angewendet. Als Mainplots wurden die Testkreuzungen der rekombinanten BC₆S₁-Linien verwendet. Als Subplots dienten deren jeweilige nahe-isogene D- und E- bulks NIB Paare. Der "NIB D-Partner" ist homozygoter Träger des Donor-Introgressionssegments, während der "NIB E-Partner" homozygoter Träger des korrespondierenden Elitelinien-Segments ist. Die entsprechenden D- und E-Partner wurden unmittelbar benachbart zueinander ausgesät, um Umgebungsunterschiede zu minimieren und um damit die auf das Introgressionssegment zurückzuführende Unterschiede mit einer höheren Genauigkeit messen zu können. Versuchseinheiten der Ertragsexperimente waren die Testkreuzungen von 7 BC₆S₁-Linien, die wiederum vier verschiedene Haplotypen repräsentierten. Exemplarisch sind die Ergebnisse der Rekombinante mit dem kürzesten Introgressionssegment (455) im Vergleich zu der mit dem längsten Introgressionssegment (765) im Detail berücksichtigt. Letzeres ist bereits deutlich kürzer als die Segmente, die gegenwärtig für bereits zugelassene Hybridsorten verfügbar sind.

Die Vorbereitung und Durchführung der Ertragsversuche entsprechen den allgemeinen Regeln und sind dem Fachmann gut bekannt. Die statistische Analyse der Daten wurde in zwei Schritten durchgeführt: Zunächst wurde an jedem Einzelstandort eine Varianzanalyse über alle Wiederholungen gerechnet, mit dem Ziel die Versuchsgenauigkeit zu bestimmen und jeweils ortsspezifische Ertragsmittelwerte für die rekombinanten Linien und deren Introgressionssegmente zu bestimmen. In einem zweiten Schritte wurden die genannten Mittelwerte dann für eine Analyse über die Umwelten hinweg verwendet.

Drastisch und statistisch signifikante Unterschiede (t-Test) für den Linkage drag-Effekt wurden beispielsweise zwischen den rekombinanten Genotypen 455 und 765 detektiert. Wie in Fig. 2 dargestellt, betrug der über die Orte gemittelte Linkage Drag-Effekt (Δ_{E-D}) 3,7 dt/ha für Haplotyp 455, während er nahezu das Doppelte (7,0 dt/ha) für den Haplotyp 765 erreichte. Die Unterschiede zwischen den beiden Rekombinanten manifestieren sich besonders deutlich am Standort PET_N unter hohem Stress durch Frühjahrstrockenheit. Hier stieg der Linkage Drag-Effekt (Δ_{E-D}) von Rekombinante 765 auf 18 dt/ha, während er bei nur 3 dt/ha für Haplotyp 455 verblieb. An einem anderen Standort (BBG) mit moderaten Stressbedingungen sinkt der Linkage Drag-Effekt (Δ_{E-D}) auf 11 dt/ha für denHaplotyp 765 ab, was jedoch ein Mehrfaches dessen ist, was Haplotyp 455 mit nur etwa 3 dt/ha zu verzeichnen hat. Grundsätzlich analoge Verhältnisse finden sich in dem im Jahr 2014 durchgeführten Experiment. Auch hier korrespondiert die Verkürzung des Introgressionssegmentes mit einer Verminderung des Linkage Drags für Ertrag. Um die beiden Experimente aus 2012 und 2014 miteinander vergleichen zu können, empfiehlt sich die Standardisierung des Linkage Drag Effektes als Prozent des Leistung des NIB-E Partners. Fig. 2 verdeutlicht, dass der Linkage Drag der Rekombinanten mit den kürzesten Introgressionssegmenten (1120 und 455) nur zwischen 3,9 und 4,7% beträgt, während die Rekombinanten mit den längsten Introgressionssegmenten (1110 und 765) mit 6,2 und 7,1% erhebliche Leistungseinbußen zu verzeichnen haben. Dennoch sind die letztgenannten Ertragsminderungen noch relativ gering, wenn man dazu ins Verhältnis setzt, dass aktuell bekannte Introgressionssegmente, die die beiden Marker tc256739 und tc300731 beinhalten, Linkage Drag Effekte von über 10% verursachen.

Die Standorte unterscheiden sich hinsichtlich ihres diagnostischen Wertes zur Detektion des Linkage Drags (siehe Figur 4). Mittelwerte für Δ_{E-D} über alle getesteten Introgressionssegmente reichten in 2012 (Serie 018/2012) von 3,2 (PET_I), 3,3 (KON), 4,1 (KO2), 4,6 (BBG), 5,7 (Ko3), 6,7 (BEK) bis zu 10,0 (PET_N) dt/ha. Der niedrigste mittlere Linkage Drag-Effekt konnte in den bewässerten Versuchen in Petkus (PET_I), in denen die Verfügbarkeit von Wasser nicht limitiert war, beobachtet werden. Demgegenüber waren die nicht-bewässerten Versuche in derselben Makroumwelt (PET_N) sehr stark in der Vorblütephase von der Trockenheit beieinträchtigt. Es zeigte sich (Figur 4), dass das Segment aus 765 deutlich auf Umweltstress reagiert (Regressionskoeffizient auf den mittleren Linkage Drag-Effekt: 1,6 dt/ha). Im Gegensatz hierzu weist das Segment aus 455 eine sehr hohe Umweltstabilität auf, was namentlich in der Stress-Umgebung PET-N bestätigt werden konnte.

### Beispiel 3: Identifizierung von rekombinanten Genotypen

Zur Identifizierung von rekombinanten Genotypen und zur Beschreibung des jeweils verbleibenden Introgressionssegmentes wurden die folgenden Marker verwendet: ctg24, ctg32, ctg16b, P20, c40745, wobei der Marker P20 die bedeutendste Rolle bei allen anschließenden Arbeiten gespielt hat. Mit Hilfe des Markers P20 konnten aus einer öffentlich zugänglichen Roggen BAC-Bibliothek entwickelt aus der cv. Blanco (Shi BJ, et al. (2009) Physical analysis of the complex rye (Secale cereale L.) Alt4 aluminium (aluminum) tolerance locus using a whole-genome BAC library of rye cv. Blanco. Theor Appl Genet. 119(4):695-704), welche kein Träger des *Rfp1*-Gens ist, BACs als Quelle für weitere Markersequenzen identifiziert werden. Es gelang einen vielversprechenden BAC zu isolieren und zu sequenzieren. Damit eröffnete sich die Möglichkeit eine BAC Bibliothek des Restorergen-tragenden Genotyps (hier bezeichnet als "IR9" bzw. ROS104) zur Verfügung zu stellen, welche mit spezifischen DNA Sonden mittels PCR gesichtet werden konnte. Mit Hilfe dieser Bibliothek konnten zwar keine, den *Rfp1*-Locus überspannende BAC Contig erstellt, aber immerhin den Locus flankierende BAC Klone identifiziert werden. Anhand der Sequenzen wurden zahlreiche Marker-Kombinationen entworfen; siehe Tabelle 2. Diese wurden für die Selektion von neuen Rekombinanten benutzt und teilweise in ein neues Markersystem (SNP-basiert) konvertiert.

Weiterhin konnte anhand der Untersuchungen mit mTerf ein neues *Rf*-Gen identifiziert werden, welches bis jetzt bei noch keiner Pflanzenart als für die Fertilitätsrestoration relevant beschrieben wurde. Es konnte erstmals gezeigt werden, dass an dem 4R Introgressionssegment zwei eigenständige und im Hinblick auf das Restorationsvermögen auch gleichwertige *Rf*-Gene wirksam sind.

Anhand eng-flankierender Marker und eines phänotypischen Tests konnte für beide beteiligten *Rf-*Gene gezeigt werden, dass die jeweiligen Donor-Introgressionssegmente noch weiter verkleinert und die Restorationsfähigkeit in vollem Umfang erhalten werden konnte.

### Beispiel 4: Entwicklung eng-gekoppelter Marker

Zur Entwicklung eng-gekoppelter Marker für den *Rfp1*-Locus in Roggen, sowie der Isolierung des funktionellen Restorergens wurde ein *Rfp1*-Allel der exotischen Landrasse IRAN IX als effizienteste Quelle der Fertilitätsrestauration genutzt. Verbunden mit dieser sehr effizienten Restaurationsleistung ist jedoch ein Linkage Drag, der je nach Standort eine deutliche Ertragsminderung bewirken kann.

Neben dem eng-gekoppelten Marker P20 wurden für die Feinkartierung der *Rfp1*-Region weitere proximal eng-gekoppelten Marker zur Verfügung gestellt. Im Wesentlichen erfolgte dies mittels zweier Strategien, die es erlaubten, ein rekombinatorisch verkleinertes genomisches Intervall per molekularer Marker zu selektieren und somit letztendlich den unerwünschten Linkage Drag zu identifizieren und reduzieren.
1) Die erste Strategie basierte auf der Nutzung konservierter Syntänie zwischen Roggen und *Brachypodium* sowie Roggen und Gerste. Auf diese Weise wurden mittels Geninformationen aus den beiden genannten Modell-Gräsern/Getreidearten neue eng-gekoppelte Marker abgeleitet.
2) Die zweite Strategie basierte, ausgehend von der Annahme, dass die enge Kopplung des Markers P20 auch eine enge physikalische Kopplung anzeigt, auf der Methode des Chromosome Walkings. Das heißt, mittels eng-gekoppelter Marker wurde eine frei verfügbare Roggen BAC Bibliothek (Populationssorte 'Blanco' (Shi et al., Theor Appl Genet 119 (2009), 695-704) durchsucht, um einen initialen BAC Contig als Startpunkt für eine Contig Analyse des *Rfp1* Locus zu erstellen. Hierfür konnte eine neu erstellte BAC Bibliothek des Restorergen-tragenden Genotyps (hier bezeichnet als 'IR9' bzw. ROS104) mit spezifischen DNA Sonden mittels PCR gesichtet werden.

Mit Hilfe dieser Bibliotheken konnten BAC Klone identifiziert werden, aus denen sich neue Marker ableiten ließen, die schließlich eine Selektion eines verkleinerten Intervals um *Rfp1* gestatteten.

### Beispiel 5: Kartierung neuer Marker in der Population ROS13024-BC1 und Identifizierung zweier unabhängiger aber äquivalent wirkender Loci der Restorationseigenschaft (Rfp1a und Rfp1b)

Ergänzend zu dem Marker P20 wurden im Rahmen der vorliegenden Erfindung einzelne zur Selektion geeignete neue Marker auf Grundlage der isolierten BAC-Klone aus der BAC-Bibliothek ROS104 entwickelt. Die anhand der isolierten BAC Klone gewonnenen Marker wurden zur hochauflösenden Kartierung in fortgeschrittenem Zuchtmaterial eingesetzt, wodurch sich letztendlich das Zielintervall weiter auflösen ließ. Die Kartierung dieser Marker im Zielintervall sowie relativ zum Zielgen erfolgte in zahlreichen Experimenten an eigenentwickelten, spaltenden Populationen. Die Marker und dazugehörigen Primersequenzen, mit deren Hilfe sich die Loci der Restorationseigenschaft in Pflanzen identifizieren lassen, sind in der nachfolgenden Tabelle 2 zuammengefasst.

Mit Hilfe der neu etablierten Selektionsmarker konnte in den vorgenommenen Kartierungsarbeiten überraschenderweise erstmals gezeigt werden, dass die Restorationseigenschaft zwei unabhängigen aber eng gekoppelte und nahezu äquivalent wirkenden Restorergene (*Rfp1a* und *Rfp1b*) am Locus *Rfp1* zuordnenbar ist (Fig. 1). Zudem erwies sich eines der beteiligten *Rf*-Gene, nämlich das *Rfp1b-*Gen, als ein Gen, das für ein mTERF-Protein kodiert. Auch das *Rfp1a* zeigt sequentiell sehr hohe Übereinstimmungen und wird ebenfalls mit hoher Wahrscheinlichkeit als ein mTERF-Gen annotiert. Da bis heute nicht bekannt war, dass ein solches Gen bei Getreide für die Fertilitätsrestauration und/oder die Pollenschüttung relevant ist, war dieses Ergebnis völlig unerwartet.

Folglich konnte anhand der vorliegenden Erfindung und der damit verbundenen Experimente erstmals gezeigt werden, dass an dem 4R Introgressionssegment zwei eigenständige und im Hinblick auf das Restaurationsvermögen auch nahezu gleichwertige *Rf*-Gene wirksam sind. Diese beiden Gene können daher nun beispielsweise mit Hilfe der in dieser Erfindung beschriebenen Marker auch getrennt züchterisch evaluiert und getrennt oder in Kombination miteinander genutzt werden. Daher ist ein Aspekt der vorliegenden Erfindung die Verwendung *Rf*-Gens *Rfp1a* allein oder in Kombination mit *Rfp1b.* In einer weiteren Ausführungsform kann auch das *Rf*-Gen *Rfp1b* unabhängig von *Rfp1a* genutzt werden. Vorzugsweise führen beide zuvor genannten äquivalent wirkenden Loci zu einer Restauration der Fertilität.

**Tabelle 2: Marker-Übersicht (Tm=Schmelztemperatur; * beschrieben in Hackauf et al, 2012)**

| Marker ID | Abgeleitet von BAC | Forward Primer (5'-3') [SEQ ID NO] | Reverse Primer (5'-3') [SEQ ID NO] | Tm [°C] | Produkt - Größe [bp] | Performance | Kategorie |
|---|---|---|---|---|---|---|---|
| tc256739* | Barley EST | 21 | 22 | 60 | 200/300 | codominant | COS |
| #1: ctg32 | 541014 contig32 | 16 | 17 | 60 | 371 | fertile pool specific | gene based STS |
| #2: ctg24met2a5 | 541014 contig24 | 14 | 15 | 60 | 1148 | codominant | gene based STS |
| #3: ctg2 | 541014 contig2 | 4 | 5 | 60 | 221 | codominant | ISBP |
| #4: ctg16b | 541014 contig16 | 10 | 11 | 60 | 516 | codominant | gene based STS |
| #5:c40745_1 | SceAssembly02 | 18 | 19 | 60 | 675 | codominant | gene based STS |
| #6: P20 | 72F13 contig2 | 6 | 7 | 65 | 424 | fertile pool specific | gene based STS |
| #7:72F13_c2_ mTERF | 72F13 contig2 | 8 | 9 | 68 | 475 | fertile pool specific | gene based STS |
| #8: 7_01_H_1441 | 72F13 contigl | 12 | 13 | 60 | 480 | fertile pool specific | STS |
| tc300731* | Wheat EST | 23 | 24 | 55 | 340/300 | codominant | COS |

In einem der durchgeführten Experimente (Ro14037) wurden fast 5000 Einzelpflanzen einer BCxS1-Population genotypisiert. Hierbei konnte für verfügbare Marker ein genetischer Polymorphismus zwischen dem *Rfp1*-Donorchromosomensegment und der Pollenelterlinie Lo727 nachgewiesen werden. Der auf Basis dieser Marker erstellte genetische Fingerabdruck ermöglichte die zuverlässige Identifizierung von nur etwa 20 Pflanzen, welche durch eine Rekombination im Bereich der wertvollen *Rfp1*-Genvariante charakterisiert werden konnten. Somit wird das genetische Intervall um *Rfp1* im genetischen Hintergrund der Linie Lo727 durch die flankierenden Marker ctg2 und 7_01_H_1441 definiert, für die ein genetischer Abstand von etwa 0,2 cM oder etwa 120 kb berechnet werden konnte (Fig. 1). Die erstellte genetische Karte dokumentiert, dass sich das Zielintervall um *Rfp1* mit Hilfe der neu entwickelten Marker in der gewünschten Weise auflösen lässt. Zunächst wurden die ersten Gen-basierten Marker sowie der Marker c40745_1 zur Selektion auf den genetischen Hintergrund eines Elitepolleneltergenotyps genutzt. Für den Nachweis des Segmentes mit dem Restorergen *Rfp1* wurde der Marker P20 eingesetzt. In einer Versuchsserie (018/2012) gelang es dann, die Expression von *Rfp1* und damit verbunden die vollständige Restauration der männlichen Fertilität für unterschiedlich lange *Rfp1*-Introgressionssegmente (Fig. 2 unten) anhand von Testkreuzungen mit dem männlich sterilen CMS-Tester Lo6-P(SR) zu beobachten.

Dieser Befund belegt (1) die Kopplung zwischen *Rfp1* und P20 sowie (2) den Wert der entwickelten Selektionsmarker zur rekombinatorischen Verkleinerung des Donorchromosomensegmentes.

Aufbauend auf diesem Ergebnis wurden in weiteren Experimenten (z.B. Ro12011) weitere spaltende BCx-Familien zunächst mit dem Marker P20 genotypisiert. In einem als Versuchsserie 12-1-23 bezeichneten Experiment wurden etwa 3200 Einzelpflanzen identifiziert, die an diesem Markerlocus reinerbig für das Allel der Elitelinie Lo310 waren. Mit den zuvor genannten Gen-basierten Markern wurden in dieser Materialgruppe 4 rekombinante Pflanzen mit unterschiedlich langen *Rfp1-*Introgressionssegmenten identifiziert (Fig. 2 oben). In Testkreuzungen dieser 4 Linien sowie des Kontrollgenotyps #1058 ohne *Rfp1*-Donorsegment mit dem männlich sterilen CMS-Tester Lo6-P(SR) konnte die Expression von *Rfp1* in 3 vollständig männlich fertilen Nachkommenschaften der Linien 1110, 1039 und 1120 beobachtet werden. Die genetische Konstitution der Rekombinanten lässt den Schluss zu, dass ein weiteres, unabhängiges und äquivalent wirkendes Restorergen im Bereich des Zielintervalls lokalisiert ist. Dieses mit dem Marker ctg2-gekoppelte Restorergen wird als *Rfp1a* bezeichnet, während das mit P20-gekoppelte Restorergen die Bezeichnung *Rfp1b* erhält (siehe auch Fig. 1).

Für die exakte Lokalisation des Restorergens *Rfp1b* wurden zusätzliche Kartierungsexperimente (z.B. Ro13030) durchgeführt. Analog zu obigen Experimenten wurden BCx-Einzelpflanzen, in denen zuvor das Donorchromosomensegment mit Hilfe der Gen-basierten Marker aus dem BAC-Klon 541014 rekombinatorisch verkleinert wurde, zunächst mit dem Marker P20 genotypisiert. Auf diese Weise wurden fast 4300 Genotypen identifiziert, die an diesem Markergenort reinerbig für das Eliteallel der Pollenelterlinie Lo310 waren. In dieser Materialgruppe konnten mit Hilfe beispielsweise des Markers 7_01_H_1441 insgesamt 13 Rekombinanten zum Marker P20 nachgewiesen werden (Fig. 3). Am Markerlocus 72F13_c2_mTERF konnte in 4 dieser 13 Rekombinanten das Donorallel aus der genetischen Ressource beobachtet werden (Fig. 3). Für 3 dieser 4 Rekombinanten wurden Testkreuzungsnachkommenschaften etabliert, in denen die männliche Fertilität vollständig restauriert worden ist. Demgegenüber zeigten die Testkreuzungsnachkommenschaften der 9 Träger des Nichtrestorer-Markerallels von mTERF einen vollkommen männlich sterilen Phänotyp.

Durch die Übereinstimmung der beobachteten Phänotypen mit den Markergenotypen eines mitochondrialen Transkriptions tERminations Faktors (mTERF) war es möglich, einen genetischen Abstand zwischen P20 und *Rfp1b* von r=0,094 cM zu berechnen. Dieser Rekombinationsschätzwert steht in sehr guter Übereinstimmung mit dem in früheren Experimenten zwischen P20 und dem mTERF-Gen berechneten Rekombinationsschätzwert r=0,011 cM.

### Beispiel 6: Rfp1 Contig Erstellung mit Hilfe der BAC Bibliothek ROS104

BAC Klone selektiert aus der BAC Bibliothek ROS104 dienten als Grundlage zur Entwicklung von Sonden und Primer zur Fortsetzung des Chromosome Walking. Hierdurch gelang es ein ca. 350 kbp Contig abzuleiten. Durch die Marker und deren Kartierung in dem fortgeschrittenen Zuchtmaterial ergibt sich, dass dieser Contig Marker trägt, die beide Restorer Loci flankieren (Fig. 1 und Tabelle 2). Untersuchungen haben ergeben, dass in diesem Intervall kein PPR-Protein kodierendes Gen liegt, jedoch dort 3 sogenannte mTERF (mitochondrialer Transkriptions-Terminations-Faktor)-Gene oder Genfragmente liegen, welche somit eindeutig als Kandidatengen für *Rfp1* anzusehen sind.

Auf Basis der bisherigen Arbeiten konnte ein BAC contig des *Rfp1*-Locus im Hintergrund eines Restorergenotypen (Eliteinzuchtlinie Lo310 aus dem Polleneltergenpool) aufgebaut werden und das Vorhandensein zweier *Rf*-Gene durch Analysen von rekombinanten Nachkommenschaften gezeigt werden.

### Beispiel 7: Validierung der Ergebnisse

Neben den in Beispiel 5 durchgeführten Nachweis der identifizierten des *Rfp1b*-Gens durch genetische Rekombination wird die Funktionalität des Gens auch im transgenen Ansatz überprüft. Hierfür wird das Protokoll zur Agrobacterium tumefaciens-vermittelten Roggentransformation von Herzfeld (2002. Development of a genetic transformation protocol for rye (Secale cereale L.) and characterisation of transgene expression after biolistic or Agrobaeterium-mediated gene transfer. Dissertation, IPK, Deuschland) angewendet. Hierfür werden Donorpflanzen der Inzuchtlinie L22 im Gewächshaus bei etwa 20°C und 16h Licht zum Blütezeitpunkt aufgezogen, anschließend unreifen Karyopsen oberflächensterilisiert und unreife Embryonen präpariert. Diese werden mit der Skutellum-Seite nach oben auf Kallus-induzierendes Medium (enthaltend MS-Salze (Murashige und Skoog, 1962. "A revised medium for rapid growth and bio assays with tobacco tissue cultures." Physiologia plantarum 15.3: 473-497.), 100 mg/l Casein-Hydrolysat, 500 mg/l Glutamin, 30 g/l Saccharose, 2,5 mg/l 2,4-D, pH 5,8, 3,0 g/l Phytagel) gegeben und in Dunkelheit bei 25°C über einen Zeitraum von 5 Tagen vor der Transformation vorkultiviert. Zum Zwecke der Transformation werden die unreife Embryonen nach vorhergehender Vorkultivierung auf 6x Makroplaten platziert (Greiner Cellstar) und in 10 ml flüssigem Kallus-induzierendem Medium suspendiert. Zur osmotischen Behandlung wird das flüssige Medium gegen 10 ml osmotisches Medium (enthaltend MS-Salze (Murashige und Skoog, 1962), 100 mg/l Casein-Hydrolysat, 500 mg/l Glutamin, 30 g/l Saccharose, 6,0 mg/l 2,4-D, 72,9 g/l Mannitol, pH 5,8) ausgetauscht und die Explantate werden über einen Zeitraum von 4-6 h plasmolysiert. Anschließend wird das osmotische Medium wieder entfernt und die Kalli mit etwa 300 µl Agrobakteriensuspension inokuliert. Anschließend erfolgt eine Vakuum-Behandlung bei 500 mbar über eine Minute und danach eine Inkubation von 10 min. Die Explantate werden zweimal in 10 ml Infektionsmedium (enthaltend MS-Salze (Murashige und Skoog, 1962), 100 mg/l Casein-Hydrolysat, 500 mg/l Glutamin, 15 g/l Saccharose, 15 g/l Glucose, 6,0mg/l 2,4D, pH 5,2, 200 µM Acetosyringon) gewaschen und über Nacht bei 22°C co-kultiviert. Nach 14-16 h werden die Explantate erneut mehrfach im Infektionsmedium gewaschen und schließlich auf festes Co-Kultivierungsmedium (Infektionsmedium supplementiert mit 3,0 g/l Phytagel) übertragen, wobei die Skutellum-Seite nach oben ausgerichtet bleibt. Die Explantate werden über zwei weitere Tage kultiviert und anschließend auf festes Kallus induzierenden Medium transferiert, welches mit 150 mg/l Timentin zur Inhibierung des Wachstums von Agrobakterien angereichert ist.

Nach 14 Tagen werden die Kalli auf selektives Regenerationsmedium (enthaltend MS-Salze (Murashige und Skoog, 1962), 100 mg/l Casein-Hydrolysat, 500 mg/l Glutamin, 30 g/l Saccharose, pH 5,8, 5,0 g/l Agarose Typ I, 150 mg/l Timentin, 30 mg/l Paromomycin) transferriert. Nach weiteren drei Wochen wurden die Kalli in geeignete Kulturgefäße transferriert, welche zur Sprossverlängerung selektives Regenerationsmedium mit 50 mg/l Paromomycin-Sulfat enthalten.

Der Vektor pYFrfp1 (Figur 6) beinhaltend das Restaurationsgen *rfp1b* (SEQ ID NO: 1) unter Kontrolle des Ubiquitin-Promotors aus Mais mit dem ersten Intron und der 35-S-Terminator inseriert im Vektor pPZP111 werden durch Elektroporation (Mersereau et al., 1990. "Efficient transformation of Agrobacterium tumefaciens by electroporation." Gene 90.1: 149-151) in den Agrobakterienstamm AGLO (Lazo et al., 1991. "A DNA transformation-competent Arabidopsis genomic library in Agrobacterium." Nature Biotechnology 9.10 (1991): 963-967) eingebracht. Eine AGLO (pYFrfp1) Kultur wird über Nacht in LB-Medium mit 50mg/l herangezogen, bis eine Sättigung (OD660 2-2,5) eingetreten war. 2 ml wurden bei 5000 RCF für 5 min zentrifugiert und das Pellet in 1 ml LB-Medium sowie 1 ml Infektionsmedium aufgelöst. Vor der Infektion der Implantate werden die Bakterien abermals für etwa zwei Stunden inkubiert (OD660 1,5-2,0).

Zur Analyse der tDNA werden die Verbindungsregion der tDNA-Grenze und des Roggengenom unter Einsatz von inverser PCR (Ochman et al., 1990. "Amplification of flanking sequences by inverse PCR." PCR protocols: A guide to methods and applications: 219-227) amplifiziert. Hierzu wird die DNA der transgenen Roggenpflanzen mit BamHI oder BglII verdaut, durch T4 DNA-Ligase zirkularisiert und anschließend als Template für die PCR verwendet. Die Amplifikation wird im Rahmen einer Nested-PCR mit dem GeneAmp-PCR System 9700 (Perkin Elmer) durchgeführt. Die Reaktionsbedingungen entsprachen den durch den Hersteller empfohlenen, wobei 200 ng Template-DNA in der ersten Reaktion und 0,5 µl aus der ersten Reaktion als Template für die zweite Reaktion eingesetzt werden, so dass das finale Volumen 25 µl entspricht.

Für die rechte Border (RB) werden für die erste Reaktion (28 Zyklen bei 94 °C für 30 s, 48 °C für 60 s und 72 °C für 2 min) die folgenden Primer verwendet RB1R 5'- CTG AAT GGC GAA TGC TAG AGC AG -3' (LacZ-Region) and UBIF 5'- CTG CAG TGC AGC GTG ACC CG -3' (3'-Region des Mais Ubiquitin Promotors). Für die zweite Reaktion (32 Zyklen bei 94 °C für 30 s, 52 °C für 60 s und 72 °C für 2 min) werden die folgenden Primer verwendet: RB2R 5' - CGT TTC CCG CCT TCA GTT TAA AC -3' und UBIF-Primer. PCR-Amplifikationsprodukte mit stumpfen Enden werden erhalten, indem Pwo DNA-Polymerase zum zweiten Reaktionsansatz hinzugegeben werden. Diese Amplifikationsprodukte werden in den PCR-Vektor (Invitrogen, San Diego, CA) kloniert, woraufhin eine Sequenzanalyse durchgeführt wird.

Erfolgreich transformierte Roggenpflanzen wurden vermehrt und mit Pampa-männlich sterilen Inzuchtlinien verkreuzt. Nachkommen, welche das Restaurationsgen *rfp1b* als Transgen tragen und exprimieren, zeigen eine Restauration der männlichen Sterilität.

Alternativ zu vorstehend beschriebenen transgenen Ansatz kann die Genfunktion auch durch den Knockout des Restaurationsgens in einer Restorerlinie erfolgen. Hierfür kann der Fachmann beispielsweise auch das TILLING oder Genome Editing (z.B. TALENs oder CRISPR/Cas) zurückgreifen, um beispielsweise ein vorzeitiges Stoppcodon in die kodierende Sequenz einzubringen oder durch eine Insertion/Deletion eine Verschiebung des Leserasters zu bewirken. Das Ergebnis wäre ein nicht funktionales mTERF-Protein und ein Verlust der Restaurationsfähigkeit.

### Beispiel 8: Charakterisierung des pflanzlichen Materials im Hinblick auf Pollenschüttung:

Die vorliegenden Ergebnisse erlauben es, einem Pflanzenzüchter nun die gewünschte Restauration für die Pampa CMS zusammen mit einer exzellenten Pollenschüttung in der Entwicklung neuer Getreidepflanzen, insbesondere Roggen und Gerste, zu verwenden. Im Zuge dessen konnten negative agronomische Merkmale auf den Ertrag signifikant reduziert und das Risiko für Mutterkornbefall gleichzeitig minimiert werden. Der Grad der Pollenschüttung, welcher durch einen erfindungsgemäßen männlicher Pollenelter erreicht wird, kann durch eine Skala von 1 bis 9 (Geiger HH, Morgenstern K (1975) Angewandt-genetische Studien zur cytoplasmatischen Pollensterilitat bei Winterroggen. Theor Appl Genet 46:269-276) bestimmt werden. Hierbei bedeuten Werte von 1 bis 3 nicht-aufspringende, leere Antheren mit geringen Maß an Degeneration, Werte von 4 bis 6 verweisen auf eine teilweise aufgehobene männliche Sterilität mit <10% bis >50% fertilen Antheren, Werte von 7 bis 8 bedeuten Pollen-schüttende Antheren mit gesteigerter Antherengröße und ein Wert von 9 entspricht einer vollständig männlich fertilen Pflanze wie sie auch in normalem Zytoplasma zu erwarten ist. Testkreuzungen ergaben erfindungsgemäße Pflanzen, die einen Wert von 7 oder höher, bevorzugt sogar einen Wert von 8 oder höher oder nahezu regelmäßig einen Wert von 9 zeigten.

In Deutschland wird die Mutterkomanfälligkeit neuer Roggensorten in Feldversuchen mit künstlicher Inokulation über mehrere Jahre und verschiedene Standorte geprüft. Die Bewertung der MutterkornAnfälligkeit basiert hierbei auf einem Notensystem von 1 (sehr gering anfällig) bis 9 (sehr stark anfällig). Wie in Tabelle 3 dargestellt, zeigen Hybridsorten, welche ein Restaurationsgen der Donoren IRAN IX, Pico Gentario oder Altevogt 14160 tragen (#1 - #4), aufgrund der hervorragenden Pollenschüttung einen deutlich reduzierten Befall mit Mutterkorn-Erregern (*Claviceps purpurea*).

**Tabelle 3. Ausprägungsstufen für Mutterkornanfälligkeit für vier Hybridsorten, welche Restaurationsgene der Donoren IRAN IX, Pico Gentario oder Altevogt 14160 tragen (linke Hälfte; #1 bis #4) und für vier Hybridsorten mit anderen Restaurationssystemen (rechte Hälfte).**

| Hybridsorten, welche Restaurationsgene der Donoren IRAN IX, Pico Gentario oder Altevogt 14160 tragen | Wert | Hybridsorten mit anderen Restaurationsgenen oder Restaurationssystemen | Wert |
|---|---|---|---|
| Visello | 3 | SU Drive | 6 |
| Minello | 4 | SU Forsetti | 5 |
| Palazzo | 4 | SU Performer | 6 |
| KWS Bono | 4 | SU Mephisto | 6 |

Im Rahmen der Besonderen Ernteermitlungen erfasst das MRI (Max Rubner-Institut, Bundesforschungsinstitut für Ernährung und Lebensmittel) regelmäßig Mutterkornbefallsdaten aus der Roggenernte der deutschen Landwirtschaft. Eigene Auswertungen dieser Daten zeigen, dass das Mutterkornaufkommen mehr als halbiert werden kann, wenn statt Hybridsorten mit einer Ausprägungsstufe von 5 bis 6 Sorten verwendet werden, die eine mit einer Ausprägungsstufe von 3-4 deutlich weniger anfällig gegenüber dem Mutterkorn sind.

### Beispiel 9: Strukturvergleich von rfp1a und rfp1b auf DNA- and Aminosäureebene:

Strukturvergleiche von *rfp1a* und *rfp1b* auf DNA- (Tabelle 4) and Aminosäureebene (Tabelle 5) zeigen eine vergleichweise hohe Übereintimmung zwischen nicht-restaurirendem Wildtyp und restaurierendem IRAN9. Überraschenderweise zeigen jedoch *rfp1a* und *rfp1b* aus IRAN9 mit nur 76% auf DNA-Ebene und nur 66% bzw. 68% auf Proteinebene eine sehr niedrige Übereinstimmung, obwohl beide eine Resaturationsvermittelnde Wirkung haben. Das zeigt, dass die Eignung von mTERF-Proteinen zur Wiederherstellung der männlichen Fertilität über eine breite strukturelle Variablilität möglich ist.

**Tabelle 4. Vergleich der Identitäten der cDNAs von rfp1a und rfp1b**

| | | *rfp1a* | | *rfp1b* | |
|---|---|---|---|---|---|
| | | Wildtyp | Iran9 | Wildtyp | Iran9 |
| *rfp1a* | Wildtyp | - | 97% | 76% | 76% |
| | Iran9 | | - | 76% | 76% |
| *rfp1b* | Wildtyp | | | - | 95% |
| | Iran9 | | | | - |

**Tabelle 5. Vergleich der Identitäten der cDNAs von rfp1a und rfp1b**

| | | *rfp1a* | | *rfp1b* | |
|---|---|---|---|---|---|
| | | Wildtyp | Iran9 | Wildtyp | Iran9 |
| *rfp1a* | Wildtyp | - | 96% | 67% | 68% |
| | Iran9 | | - | 66% | 67% |
| *rfp1b* | Wildtyp | | | - | 90% |
| | Iran9 | | | | - |

### Beispiel 10: Nachweis der Restaurationsleistung der rfp1a und rfp1b Gene einzeln und in Kombination sowie aus unterschiedlichen Quellen:

Tabelle 6 zeigt deutlich, dass Testkreuzungspflanzen, die mit nur einer Kopie, *rfp1a* oder *rfp1b* ausgestattet sind, eine leicht geringere aber insgesamt voll ausreichende Pollenschüttung und Antherengröße aufweisen, wenn man sie mit Pflanzen vergleicht, die beide Kopien besitzen.

**Tabelle 6. Antheren-Bonitur nach Geiger & Morgenstern (1975) von Testkreuzungspflanzen (Tx...) mit unterscheidlicher rfp1-Kopien-Ausstattung:**

| Testkreuzungen | rfp1-Kopien-Ausstattung | Mittelwert der restaurierten Testkreuzungspflanzen | |
|---|---|---|---|
| | | Antheren-Bonitur | Antherenlänge (mm) |
| TxBC7(Lo310) 1120 | *rfp1a* | 8 | 7 |
| TxBC7S1(Lo310) 3308 | *rfp1a* | 8 | 7 |
| TxBC6S1(Lo310) 455 | *rfp1b* | 8 | 7 |
| TxBC6S1(Lo310) 217 | *rfp1a* und *rfp1b* | 9 | 8 |
| TxBC6S1(Lo310) 765 | *rfp1a* und *rfp1b* | 9 | 8 |
| TxBC4(Lo316xIRAN IX) | *rfp1a* und *rfp1b* | 9 | 8 |
| TxBC2(Lo316xAltevogt) | *rfp1a* und *rfp1b* | 9 | 8 |
| TxLo310 (Original line) | - | 3 | |

## Patentansprüche

1. Pflanze, insbesondere aus der Ordnung der Grasartigen (*Poales*), vorzugsweise der Familie der Süßgräser (Poaceae), die geeignet ist, als männlicher Pollenelter die Pollenfertilität für die Pampa cytoplasmatische männliche Sterilität (CMS) zu restaurieren, wobei
a) in der Pflanze oder in einer Hybridpflanze aus einer Kreuzung mit einem weiblichen CMS Elter ein ansonsten mit der Restaurationseigenschaft gekoppelter Linkage Drag, vorzugsweise ertragsmindernder Effekt reduziert ist oder vollständig unterbleibt, und
b) die Pflanze ein chromosomales Segment umfasst, das mindestens ein Nukleinsäuremolekül, das in der Lage ist die Restaurationseigenschaft zu vermitteln, aufweist, und das mindestens eine Nukleinsäuremolekül eine Nukleotidsequenz aufweist, welche ausgewählt ist aus der Gruppe bestehend aus einer:
(i) Nukleotidsequenz gemäß einer der SEQ ID NO: 1 oder SEQ ID NO: 28 oder einem funktionellen Fragment davon,
(ii) Nukleotidsequenz, die eine Aminosäuresequenz gemäß einer der SEQ ID NO: 2 oder SEQ ID NO: 29 oder einem funktionellen Fragment davon kodiert,
(iii) Nukleotidsequenz, die komplementär ist zu einer Nukleotidsequenz nach (i) oder (ii),
(iv) Nukleotidsequenz, welche an eine Sequenz nach (iii) unter stringenten Bedingungen hybridisiert,
(v) Nukleotidsequenz, die eine Identität von mindestens 70% zu der Nukleotidsequenz nach (i) oder (ii) aufweist,
(vi) Nukleotidsequenz, die eine Aminosäuresequenz, die eine Identität von mindestens 65% zu der SEQ ID NO: 2 oder SEQ ID NO: 29 aufweist, kodiert,
(vii) Nukleotidsequenz, die eine Aminosäuresequenz kodiert, die gegenüber der in SEQ ID NO: 2 oder SEQ ID NO: 29 gezeigten Aminosäuresequenz Abweichungen in Form von Aminosäure-Deletionen, Substitutionen, Additionen und/oder Insertionen in der Aminosäuresequenz aufweist.

2. Pflanze nach Anspruch 1, wobei das chromosomale Segment ein Intervall zwischen den Markerloci tc256739 (Amplifikationsprodukt der Primer mit SEQ ID NOs: 21 und 22), ctg32 (Amplifikationsprodukt der Primer mit SEQ ID Nr.: 16 und 17) oder ctg24met2a5 (Amplifikationsprodukt der Primer mit SEQ ID Nr.: 14 und 15) und tc300731 (Amplifikationsprodukt der Primer mit SEQ ID NOs: 23 und 24) oder 7_01_H_1441 (Amplifikationsprodukt der Primer mit SEQ ID NOs: 12 und 13) auf dem Chromosom 4R von einem Donor ausgewählt aus der Gruppe bestehend aus IRAN IX, Pico Gentario und Altevogt 14160 ist.

3. Pflanze nach Anspruch 1 oder 2, wobei das chromosomale Segment einen oder mehrere der folgenden Markerloci des Donors aufweist: ctg2 (Amplifikationsprodukt der Primer mit SEQ ID NOs: 4 und 5), P20 (Amplifikationsprodukt der Primer mit SEQ ID NOs: 6 und 7), 72F13_c2_mTERF (Amplifikationsprodukt der Primer mit SEQ ID NOs: 8 und 9) oder ctg16b (Amplifikationsprodukt der Primer mit SEQ ID NOs: 10 und 11).

4. Pflanze nach einem der Ansprüche 1 bis 3, wobei das chromosomale Segment durch Abwesenheit einer oder mehrerer der folgenden Markerloci des Donors gekennzeichnet ist:
7_01_H_1441 (Amplifikationsprodukt der Primer mit SEQ ID NOs: 12 und 13), ctg24met2a5 (Amplifikationsprodukt der Primer mit SEQ ID NOs: 14 und 15) oder ctg32 (Amplifikationsprodukt der Primer mit SEQ ID NOs: 16 und 17).

5. Pflanze nach einem der Ansprüche 1 bis 4, wobei das chromosomale Segment nicht größer als 190 kb ist.

6. Pflanze nach einem der Ansprüche 1 bis 5, wobei die Pflanze eine Inzuchtpflanze, eine Pflanze, einer doppelhaploide Pflanze oder eine Hybridpflanze ist.

7. Pflanze nach einem der Ansprüche 1 bis 6, die eine erhöhte Resistenz gegen ein Pathogen aufweist, vorzugsweise gegen einen Pilz, insbesondere gegen den Pilz *Claviceps purpurea* (Fr.).

8. Pflanze nach einem der Ansprüche 1 bis 7, die eine Pflanze der Gattung *Secale, Hordeum* oder *Triticale* ist, vorzugsweise eine Pflanze der Art *Secale cereale* oder *Hordeum vulgare.*

9. Samen oder Nachkomme der Pflanze nach einem der Ansprüche 1 bis 8, wobei der Samen oder der Nachkomme das in den vorhergehenden Ansprüchen definierte chromosomale Segment umfasst.

10. Organ, Pflanzenteil, Gewebe oder Zelle der Pflanze nach einem der Ansprüche 1 bis 9.

11. Verwendung der Pflanze nach einem der Ansprüche 1 bis 8 oder des Nachkommens nach Anspruch 9 zur Herstellung einer Hybridpflanze, vorzugsweise der Gattung *Secale* oder *Triticale,* vorzugsweise einer Pflanze der Art *Secale cereale,* deren Pollenfertilität für die Pampa CMS restauriert ist und/oder die eine erhöhte Resistenz gegen ein pilzlichen Pathogen aufweist, insbesondere gegen den Pilz *Claviceps purpurea* (Fr.).

12. Verfahren zum Nachweis einer Pflanze, insbesondere aus der Ordnung der Grasartigen (*Poales*), vorzugsweise der Familie der Süßgräser (Poaceae), die geeignet ist, als männlicher Pollenelter die Pollenfertilität für die P-CMS zu restaurieren, wobei in einer Hybridpflanze aus einer Kreuzung mit einem weiblichen CMS Elter ein ansonsten mit der Restaurationseigenschaft gekoppelter Linkage Drag, vorzugsweise ertragsmindernder Effekt reduziert ist oder vollständig unterbleibt, umfassend
(i) Nachweis in der Pflanze der Anwesenheit oder Abwesenheit von mindestens einem Markerallel, stammend aus einem Donor ausgewählt aus der Gruppe bestehend aus IRAN IX, Pico Gentario und Altevogt 14160, auf bzw. in dem Rfp1-Locus, und
(ii) Selektieren von Pflanzen, in welchen das mindestens eine Markerallel vorhanden ist.

13. Verfahren nach Anspruch 12, wobei der Rfp1-Locus einen chromosomalen Abschnitt auf dem Chromosom 4R von einem Donor ausgewählt aus der Gruppe bestehend aus IRAN IX, Pico Gentario und Altevogt 14160 darstellt, ausgewählt aus:
a) zwischen den Markerloci ctg32 und tc300731,
b) zwischen den Markerloci ctg24met2a5 und tc300731,
c) zwischen den Markerloci ctg2 und tc300731,
d) zwischen den Markerloci ctg16b und tc300731,
e) zwischen den Markerloci c40745_1 und tc300731,
f) zwischen den Markerloci P20 und tc300731,
g) zwischen den Markerloci tc256739 und 7_01_H_1441,
h) zwischen den Markerloci ctg32 und 7_01_H_1441,
i) zwischen den Markerloci ctg24met2a5 und 7_01_H_1441,
j) zwischen den Markerloci ctg2 und 7_01_H_1441,
k) zwischen den Markerloci ctg16b und 7_01_H_1441,
l) zwischen den Markerloci c40745_1 und 7_01_H_1441,
m) zwischen den Markerloci P20 und 7_01_H_1441,
n) zwischen den Markerloci tc256739 und 72F13_c2_mTERF,
o) zwischen den Markerloci tc256739 und P20,
p) zwischen den Markerloci tc256739 und c40745_1,
q) zwischen den Markerloci tc256739 und ctg16b,
r) zwischen den Markerloci ctg32 und 72F13_c2_mTERF,
s) zwischen den Markerloci ctg32 und P20,
t) zwischen den Markerloci ctg32 und c40745_1,
u) zwischen den Markerloci ctg32 und ctg16b,
v) zwischen den Markerloci ctg24met2a5 und 72F13_c2_mTERF,
w) zwischen den Markerloci ctg24met2a5 und P20,
x) zwischen den Markerloci ctg24met2a5 und c40745_1,
y) zwischen den Markerloci ctg24met2a5 und ctg16b,
z) zwischen den Markerloci ctg2 und 72F13_c2_mTERF,
aa) zwischen den Markerloci ctg2 und P20,
bb) zwischen den Markerloci ctg2 und c40745_1, oder
cc) zwischen den Markerloci ctg2 und ctg16b.
